(19) Europäisches Patentamt
European Patent Office
Office européen des brevets

(11) EP 3 067 368 A1

(12) **EUROPEAN PATENT APPLICATION**

(43) Date of publication:
**14.09.2016 Bulletin 2016/37**

(51) Int Cl.:
***C07K 16/00*** *(2006.01)* ***C07K 16/28*** *(2006.01)*
***A61K 39/00*** *(2006.01)*

(21) Application number: **16164079.2**

(22) Date of filing: **28.04.2008**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **27.04.2007 US 926516 P**

(62) Document number(s) of the earlier application(s) in accordance with Art. 76 EPC:
**12007260.8 / 2 599 791**
**08767432.1 / 2 150 562**

(71) Applicant: **Genentech, Inc.**
**South San Francisco, CA 94080-4990 (US)**

(72) Inventors:
- **FUNG, Sek Chung**
**Gaithersburg, MD 20878 (US)**
- **HUANG, Dan**
**Short Hills, NJ 07078-2310 (US)**
- **SINGH, Sanjaya**
**Sandy Hook, CT 06482 (US)**

(74) Representative: **Jones Day**
**Rechtsanwälte, Attorneys-at-Law, Patentanwälte**
**Prinzregentenstrasse 11**
**80538 München (DE)**

Remarks:
This application was filed on 06-04-2016 as a divisional application to the application mentioned under INID code 62.

# (54) POTENT, STABLE AND NON-IMMUNOSUPPRESSIVE ANTI-CD4 ANTIBODIES

(57) The invention relates to novel antibodies and their fragments are disclosed that specifically bind to CD4. Anti-CD4 antibodies are disclosed having amino acid substitutions in the hinge region that prevent intrachain disulfide bond formation resulting in antibody molecules with surprisingly improved bivalent stability. Increased stability facilitates manufacturing and provides increased in vivo stability. Antibodies are also disclosed that have amino acid substitutions that reduce binding to Fc receptor and ADCC activity that also result in a surprising effect on CD4 internalization. In addition, genes for such antibodies are disclosed that have been modified such that they have increased expression over their unmodified counterparts.

**FIGURE 1A**

**TNX-355 Amino Acid Sequence**

**Kappa Light Chain Variable Region (SEQ ID NO:1)**

```
DIVMTQSPDS LAVSLGERVT MNCKSSQSLL YSTNQKNYLA WYQQKPGQSP KLLIYWASTR
                            CDRL1                         CDRL2
ESGVPDRFSG SGSGTDFTLT ISSVQAEDVA VYYCQQYYSY RTFGGGTKLE IK
                                      CDRL3
```

**Light Chain Constant Region (SEQ ID NO:2)**

```
RTVAAPSVFI FPPSDEQLKS GTASVVCLLN NFYPREAKVQ WKVDNALQSG NSQESVTEQD
SKDSTYSLSS TLTLSKADYE KHKVYACEVT HQGLSSPVTK SFNRGEC
```

**Heavy Chain Variable Region (SEQ ID NO:3)**

```
QVQLQQSGPE VVKPGASVKM SCKASGYTFT SYVIHWVRQK PGQGLDWIGY INPYNDGTDY
                            CDRH1                         CDRH2
DEKFKGKATL TSDTSTSTAY MELSSLRSED TAVYYCAREK DNYATGAWFA YWGQGTLVTV
                                           CDRH3
SS
```

**IgG4 Constant Region (SEQ ID NO:4)**

```
ASTKGPSVFP LAPCSRSTSE STAALGCLVK DYFPEPVTVS WNSGALTSGV
HTFPAVLQSS GLYSLSSVVT VPSSSLGTKT YTCNVDHKPS NTKVDKRVES
KYGPPCPSCP APEFLGGPSV FLFPPKPKDT LMISRTPEVT CVVVDVSQED
PEVQFNWYVD GVEVHNAKTK PREEQFNSTY RVVSVLTVLH QDWLNGKEYK
CKVSNKGLPS SIEKTISKAK GQPREPQVYT LPPSQEEMTK NQVSLTCLVK
GFYPSDIAVE WESNGQPENN YKTTPPVLDS DGSFFLYSRL TVDKSRWQEG
NVFSCSVMHE ALHNHYTQKS LSLSLGK
```



Printed by Jouve, 75001 PARIS (FR)

(Cont. next page)

## FIGURE 1 B

### G4H Amino Acid Sequence

**Kappa Light Chain Variable Region (SEQ ID NO:1)**

DIVMTQSPDS LAVSLGERVT MNCKSSQSLL YSTNQKNYLA WYQQKPGQSP KLLIYWASTR
CDRL1 CDRL2
ESGVPDRFSG SGSGTDFTLT ISSVQAEDVA VYYCQQYYSY RTFGGGTKLE IK
CDRL3

**Light Chain Constant Region (SEQ ID NO:2)**

RTVAAPSVFI FPPSDEQLKS GTASVVCLLN NFYPREAKVQ WKVDNALQSG NSQESVTEQD

SKDSTYSLSS TLTLSKADYE KHKVYACEVT HQGLSSPVTK SFNRGEC

**Heavy Chain Variable Region (SEQ ID NO:3)**

QVQLQQSGPE VVKPGASVKM SCKASGYTFT SYVIHWVRQK PGQGLDWIGY INPYNDGTDY
CDRH1 CDRH2
DEKFKGKATL TSDTSTSTAY MELSSLRSED TAVYYCAREK DNYATGAWFA YWGQGTLVTV
CDRH3
SS

**G4H Heavy Chain Constant Region (SEQ ID NO:5)**

ASTKGPSVFP LAPCSRSTSE STAALGCLVK DYFPEPVTVS WNSGALTSGV
HTFPAVLQSS GLYSLSSVVT VPSSSLGTKT YTCNVDHKPS NTKVDKRVES
KYGPPCPPCP APEFLGGPSV FLFPPKPKDT LMISRTPEVT CVVVDVSQED
PEVQFNWYVD GVEVHNAKTK PREEQFNSTY RVVSVLTVLH QDWLNGKEYK
CKVSNKGLPS SIEKTISKAK GQPREPQVYT LPPSQEEMTK NQVSLTCLVK
GFYPSDIAVE WESNGQPENN YKTTPPVLDS DGSFFLYSRL TVDKSRWQEG
NVFSCSVMHE ALHNHYTQKS LSLSLGK

## FIGURE 1 C

### G4D Amino Acid Sequence

**Kappa Light Chain Variable Region (SEQ ID NO:1)**

DIVMTQSPDS LAVSLGERVT MNCKSSQSLL YSTNQKNYLA WYQQKPGQSP KLLIYWASTR
CDRL1 CDRL2
ESGVPDRFSG SGSGTDFTLT ISSVQAEDVA VYYCQQYYSY RTFGGGTKLE IK
CDRL3

**Light Chain Constant Region (SEQ ID NO:2)**

RTVAAPSVFI FPPSDEQLKS GTASVVCLLN NFYPREAKVQ WKVDNALQSG NSQESVTEQD

SKDSTYSLSS TLTLSKADYE KHKVYACEVT HQGLSSPVTK SFNRGEC

**Heavy Chain Variable Region (SEQ ID NO:3)**

QVQLQQSGPE VVKPGASVKM SCKASGYTFT SYVIHWVRQK PGQGLDWIGY INPYNDGTDY
CDRH1 CDRH2
DEKFKGKATL TSDTSTSTAY MELSSLRSED TAVYYCAREK DNYATGAWFA YWGQGTLVTV
CDRH3
SS

**G4D Heavy Chain Constant Region (SEQ ID NO:6)**

ASTKGPSVFP LAPCSRSTSE STAALGCLVK DYFPEPVTVS WNSGALTSGV
HTFPAVLQSS GLYSLSSVVT VPSSSLGTKT YTCNVDHKPS NTKVDKRV ES
KYGPPCPPCP APEFEGGPSV FLFPPKPKDT LMISRTPEVT CVVVDVSQED
PEVQFNWYVD GVEVHNAKTK PREEQFNSTY RVVSVLTVLH QDWLNGKEYK
CKVSNKGLPS SIEKTISKAK GQPREPQVYT LPPSQEEMTK NQVSLTCLVK
GFYPSDIAVE WESNGQPENN YKTTPPVLDS DGSFFLYSRL TVDKSRWQEG
NVFSCSVMHE ALHNHYTQKS LSLSLGK

## FIGURE 1 D

### MV1 Amino Acid Sequence

**Kappa Light Chain Variable Region (SEQ ID NO:1)**

DIVMTQSPDS LAVSLGERVT MNCKSSQSLL YSTNQKNYLA WYQQKPGQSP KLLIYWASTR
CDRL1 CDRL2
ESGVPDRFSG SGSGTDFTLT ISSVQAEDVA VYYCQQYYSY RTFGGGTKLE IK
CDRL3

**Light Chain Constant Region (SEQ ID NO:2)**

RTVAAPSVFI FPPSDEQLKS GTASVVCLLN NFYPREAKVQ WKVDNALQSG NSQESVTEQD

SKDSTYSLSS TLTLSKADYE KHKVYACEVT HQGLSSPVTK SFNRGEC

**Heavy Chain Variable Region (SEQ ID NO:3)**

QVQLQQSGPE VVKPGASVKM SCKASGYTFT SYVIHWVRQK PGQGLDWIGY INPYNDGTDY
CDRH1 CDRH2
DEKFKGKATL TSDTSTSTAY MELSSLRSED TAVYYCAREK DNYATGAWFA YWGQGTLVTV
CDRH3
SS

**MV1 Heavy Chain Constant Region (SEQ ID NO:7)**

ASTKGPSVFP LAPSSKSTSG GTAALGCLVK DYFPEPVTVS WNSGALTSGV
HTFPAVLQSS GLYSLSSVVT VPSSSLGTQT YICNVNHKPS NTKVDKKVEP
KSCDKTHTCP PCPAPEFEGG PSVFLFPPKP KDTLMISRTP EVTCVVVDVS
HEDPEVKFNW YVDGVEVHNA KTKPREEQYN STYRVVSVLT VLHQDWLNGK
EYKCKVSNKA LPASIEKTIS KAKGQPREPQ VYTLPPSRDE LTKNQVSLTC
LVKGFYPSDI AVEWESNGQP ENNYKTTPPV LDSDGSFFLY SKLTVDKSRW
QQGNVFSCSV MHEALHNHYT QKSLSLSPGK

SEQUENCE LISTING

<110> Genentech, INC.

<120> POTENT, STABLE AND NON-IMMUNOSUPPRESSIVE ANTI-CD4 ANTIBODIES

<130> 102346PCEPT1T1

<150> 60/926,516
<151> 2007-04-27

<160> 40

<170> PatentIn version 3.4

<210> 1
<211> 112
<212> PRT
<213> Artificial Sequence

<220>
<223> VARIABLE LIGHT CHAIN SEQUENCE OF TNX-355

<400> 1

```
Asp Ile Val Met Thr Gln Ser Pro Asp Ser Leu Ala Val Ser Leu Gly
1               5                   10                  15

Glu Arg Val Thr Met Asn Cys Lys Ser Ser Gln Ser Leu Leu Tyr Ser
            20                  25                  30

Thr Asn Gln Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Val Gln Ala Glu Asp Val Ala Val Tyr Tyr Cys Gln Gln
                85                  90                  95

Tyr Tyr Ser Tyr Arg Thr Phe Gly Gly Gly Thr Lys Leu Glu Ile Lys
            100                 105                 110
```

<210> 2
<211> 107
<212> PRT
<213> Artificial Sequence

<220>
<223> LIGHT CHAIN CONSTANT REGION OF TNX-355

<400> 2

Arg Thr Val Ala Ala Pro Ser Val Phe Ile Phe Pro Pro Ser Asp Glu
1               5                   10                  15

Gln Leu Lys Ser Gly Thr Ala Ser Val Val Cys Leu Leu Asn Asn Phe
            20                  25                  30

Tyr Pro Arg Glu Ala Lys Val Gln Trp Lys Val Asp Asn Ala Leu Gln
        35                  40                  45

Ser Gly Asn Ser Gln Glu Ser Val Thr Glu Gln Asp Ser Lys Asp Ser
    50                  55                  60

Thr Tyr Ser Leu Ser Ser Thr Leu Thr Leu Ser Lys Ala Asp Tyr Glu
65                  70                  75                  80

Lys His Lys Val Tyr Ala Cys Glu Val Thr His Gln Gly Leu Ser Ser
                85                  90                  95

Pro Val Thr Lys Ser Phe Asn Arg Gly Glu Cys
            100                 105

<210> 3
<211> 122
<212> PRT
<213> Artificial Sequence

<220>
<223> HEAVY CHAIN VARIABLE REGION OF TNX-355

<400> 3

Gln Val Gln Leu Gln Gln Ser Gly Pro Glu Val Val Lys Pro Gly Ala
1               5                   10                  15

Ser Val Lys Met Ser Cys Lys Ala Ser Gly Tyr Thr Phe Thr Ser Tyr
            20                  25                  30

Val Ile His Trp Val Arg Gln Lys Pro Gly Gln Gly Leu Asp Trp Ile
        35                  40                  45

Gly Tyr Ile Asn Pro Tyr Asn Asp Gly Thr Asp Tyr Asp Glu Lys Phe
    50                  55                  60

Lys Gly Lys Ala Thr Leu Thr Ser Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Glu Lys Asp Asn Tyr Ala Thr Gly Ala Trp Phe Ala Tyr Trp
100 105 110

Gly Gln Gly Thr Leu Val Thr Val Ser Ser
115 120

<210> 4
<211> 326
<212> PRT
<213> Artificial Sequence

<220>
<223> HEAVY CHAIN constant REGION OF TNX-355

<400> 4

Ala Ser Thr Lys Gly Pro Ser Val Phe Pro Leu Ala Pro Cys Ser Arg
1 5 10 15

Ser Thr Ser Glu Ser Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Lys Thr
65 70 75 80

Tyr Thr Cys Asn Val Asp His Lys Pro Ser Asn Thr Lys Val Asp Lys
85 90 95

Arg Val Glu Ser Lys Tyr Gly Pro Pro Cys Pro Ser Cys Pro Ala Pro
100 105 110

Glu Phe Leu Gly Gly Pro Ser Val Phe Leu Phe Pro Pro Lys Pro Lys
115 120 125

Asp Thr Leu Met Ile Ser Arg Thr Pro Glu Val Thr Cys Val Val Val
130 135 140

Asp Val Ser Gln Glu Asp Pro Glu Val Gln Phe Asn Trp Tyr Val Asp
145 150 155 160

Gly Val Glu Val His Asn Ala Lys Thr Lys Pro Arg Glu Glu Gln Phe
165 170 175

Asn Ser Thr Tyr Arg Val Val Ser Val Leu Thr Val Leu His Gln Asp
180 185 190

Trp Leu Asn Gly Lys Glu Tyr Lys Cys Lys Val Ser Asn Lys Gly Leu
195 200 205

Pro Ser Ser Ile Glu Lys Thr Ile Ser Lys Ala Lys Gly Gln Pro Arg
210 215 220

Glu Pro Gln Val Tyr Thr Leu Pro Pro Ser Gln Glu Glu Met Thr Lys
225 230 235 240

Asn Gln Val Ser Leu Thr Cys Leu Val Lys Gly Phe Tyr Pro Ser Asp
245 250 255

Ile Ala Val Glu Trp Glu Ser Asn Gly Gln Pro Glu Asn Asn Tyr Lys
260 265 270

Thr Thr Pro Pro Val Leu Asp Ser Asp Gly Ser Phe Phe Leu Tyr Ser
275 280 285

Arg Leu Thr Val Asp Lys Ser Arg Trp Gln Glu Gly Asn Val Phe Ser
290 295 300

Cys Ser Val Met His Glu Ala Leu His Asn His Tyr Thr Gln Lys Ser
305 310 315 320

Leu Ser Leu Ser Leu Gly
325

<210> 5
<211> 326
<212> PRT
<213> Artificial Sequence

<220>
<223> HEAVY CHAIN CONSTANT REGION OF G4H

<400> 5

Ala Ser Thr Lys Gly Pro Ser Val Phe Pro Leu Ala Pro Cys Ser Arg
1 5 10 15

Ser Thr Ser Glu Ser Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

```
Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Pro Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300
```

```
Cys Ser Val Met His Glu Ala Leu His Asn His Tyr Thr Gln Lys Ser
305                 310                 315                 320

Leu Ser Leu Ser Leu Gly
                325

<210>  6
<211>  326
<212>  PRT
<213>  Artificial Sequence

<220>
<223>  HEAVY CHAIN CONSTANT REGION OF G4D

<400>  6

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Pro Cys Pro Ala Pro
            100                 105                 110

Glu Phe Glu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175
```

```
Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly
                325


<210>  7
<211>  329
<212>  PRT
<213>  Artificial Sequence

<220>
<223>  HEAVY CHAIN CONSTANT REGION OF MV1

<400>  7

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
```

```
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Phe Glu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Ser Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300
```

Val Phe Ser Cys Ser Val Met His Glu Ala Leu His Asn His Tyr Thr
305                 310                 315                 320

Gln Lys Ser Leu Ser Leu Ser Pro Gly
                325

<210> 8
<211> 448
<212> PRT
<213> Artificial Sequence

<220>
<223> G4H HEAVY CHAIN SEQUENCE

<400> 8

Gln Val Gln Leu Gln Gln Ser Gly Pro Glu Val Val Lys Pro Gly Ala
1               5                   10                  15

Ser Val Lys Met Ser Cys Lys Ala Ser Gly Tyr Thr Phe Thr Ser Tyr
            20                  25                  30

Val Ile His Trp Val Arg Gln Lys Pro Gly Gln Gly Leu Asp Trp Ile
        35                  40                  45

Gly Tyr Ile Asn Pro Tyr Asn Asp Gly Thr Asp Tyr Asp Glu Lys Phe
    50                  55                  60

Lys Gly Lys Ala Thr Leu Thr Ser Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Glu Lys Asp Asn Tyr Ala Thr Gly Ala Trp Phe Ala Tyr Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Cys Ser Arg Ser Thr Ser Glu Ser Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Lys Thr Tyr Thr Cys Asn Val Asp
        195                 200                 205

His Lys Pro Ser Asn Thr Lys Val Asp Lys Arg Val Glu Ser Lys Tyr
    210                 215                 220

Gly Pro Pro Cys Pro Pro Cys Pro Ala Pro Glu Phe Leu Gly Gly Pro
225                 230                 235                 240

Ser Val Phe Leu Phe Pro Pro Lys Pro Lys Asp Thr Leu Met Ile Ser
245 250 255

Arg Thr Pro Glu Val Thr Cys Val Val Val Asp Val Ser Gln Glu Asp
260 265 270

Pro Glu Val Gln Phe Asn Trp Tyr Val Asp Gly Val Glu Val His Asn
275 280 285

Ala Lys Thr Lys Pro Arg Glu Glu Gln Phe Asn Ser Thr Tyr Arg Val
290 295 300

Val Ser Val Leu Thr Val Leu His Gln Asp Trp Leu Asn Gly Lys Glu
305 310 315 320

Tyr Lys Cys Lys Val Ser Asn Lys Gly Leu Pro Ser Ser Ile Glu Lys
325 330 335

Thr Ile Ser Lys Ala Lys Gly Gln Pro Arg Glu Pro Gln Val Tyr Thr
340 345 350

Leu Pro Pro Ser Gln Glu Glu Met Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Arg Leu Thr Val Asp Lys
405 410 415

Ser Arg Trp Gln Glu Gly Asn Val Phe Ser Cys Ser Val Met His Glu
420 425 430

Ala Leu His Asn His Tyr Thr Gln Lys Ser Leu Ser Leu Ser Leu Gly
435 440 445

<210> 9
<211> 448
<212> PRT
<213> Artificial Sequence

<220>
<223> G4D HEAVY CHAIN SEQUENCE

<400> 9

Gln Val Gln Leu Gln Gln Ser Gly Pro Glu Val Val Lys Pro Gly Ala
1 5 10 15

Ser Val Lys Met Ser Cys Lys Ala Ser Gly Tyr Thr Phe Thr Ser Tyr
20 25 30

Val Ile His Trp Val Arg Gln Lys Pro Gly Gln Gly Leu Asp Trp Ile
35 40 45

Gly Tyr Ile Asn Pro Tyr Asn Asp Gly Thr Asp Tyr Asp Glu Lys Phe
50 55 60

Lys Gly Lys Ala Thr Leu Thr Ser Asp Thr Ser Thr Ser Thr Ala Tyr

65 70 75 80

Met Glu Leu Ser Ser Leu Arg Ser Glu Asp Thr Ala Val Tyr Tyr Cys
85 90 95

Ala Arg Glu Lys Asp Asn Tyr Ala Thr Gly Ala Trp Phe Ala Tyr Trp
100 105 110

Gly Gln Gly Thr Leu Val Thr Val Ser Ser Ala Ser Thr Lys Gly Pro
115 120 125

Ser Val Phe Pro Leu Ala Pro Cys Ser Arg Ser Thr Ser Glu Ser Thr
130 135 140

Ala Ala Leu Gly Cys Leu Val Lys Asp Tyr Phe Pro Glu Pro Val Thr
145 150 155 160

Val Ser Trp Asn Ser Gly Ala Leu Thr Ser Gly Val His Thr Phe Pro
165 170 175

Ala Val Leu Gln Ser Ser Gly Leu Tyr Ser Leu Ser Ser Val Val Thr
180 185 190

Val Pro Ser Ser Ser Leu Gly Thr Lys Thr Tyr Thr Cys Asn Val Asp
195 200 205

His Lys Pro Ser Asn Thr Lys Val Asp Lys Arg Val Glu Ser Lys Tyr
210 215 220

Gly Pro Pro Cys Pro Pro Cys Pro Ala Pro Glu Phe Glu Gly Gly Pro
225 230 235 240

Ser Val Phe Leu Phe Pro Pro Lys Pro Lys Asp Thr Leu Met Ile Ser
245 250 255

Arg Thr Pro Glu Val Thr Cys Val Val Val Asp Val Ser Gln Glu Asp
260 265 270

Pro Glu Val Gln Phe Asn Trp Tyr Val Asp Gly Val Glu Val His Asn
275 280 285

Ala Lys Thr Lys Pro Arg Glu Glu Gln Phe Asn Ser Thr Tyr Arg Val
290 295 300

Val Ser Val Leu Thr Val Leu His Gln Asp Trp Leu Asn Gly Lys Glu
305 310 315 320

Tyr Lys Cys Lys Val Ser Asn Lys Gly Leu Pro Ser Ser Ile Glu Lys
325 330 335

Thr Ile Ser Lys Ala Lys Gly Gln Pro Arg Glu Pro Gln Val Tyr Thr
340 345 350

Leu Pro Pro Ser Gln Glu Glu Met Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Arg Leu Thr Val Asp Lys

405 410 415

Ser Arg Trp Gln Glu Gly Asn Val Phe Ser Cys Ser Val Met His Glu
420 425 430

Ala Leu His Asn His Tyr Thr Gln Lys Ser Leu Ser Leu Ser Leu Gly
435 440 445

<210> 10
<211> 451
<212> PRT
<213> Artificial Sequence

<220>
<223> MV1 HEAVY CHAIN SEQUENCE

<400> 10

Gln Val Gln Leu Gln Gln Ser Gly Pro Glu Val Val Lys Pro Gly Ala
1 5 10 15

Ser Val Lys Met Ser Cys Lys Ala Ser Gly Tyr Thr Phe Thr Ser Tyr
20 25 30

Val Ile His Trp Val Arg Gln Lys Pro Gly Gln Gly Leu Asp Trp Ile
35 40 45

Gly Tyr Ile Asn Pro Tyr Asn Asp Gly Thr Asp Tyr Asp Glu Lys Phe
50 55 60

Lys Gly Lys Ala Thr Leu Thr Ser Asp Thr Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Glu Lys Asp Asn Tyr Ala Thr Gly Ala Trp Phe Ala Tyr Trp
100 105 110

Gly Gln Gly Thr Leu Val Thr Val Ser Ser Ala Ser Thr Lys Gly Pro
115 120 125

Ser Val Phe Pro Leu Ala Pro Ser Ser Lys Ser Thr Ser Gly Gly Thr
130 135 140

Ala Ala Leu Gly Cys Leu Val Lys Asp Tyr Phe Pro Glu Pro Val Thr
145 150 155 160

Val Ser Trp Asn Ser Gly Ala Leu Thr Ser Gly Val His Thr Phe Pro
165 170 175

Ala Val Leu Gln Ser Ser Gly Leu Tyr Ser Leu Ser Ser Val Val Thr
180 185 190

Val Pro Ser Ser Ser Leu Gly Thr Gln Thr Tyr Ile Cys Asn Val Asn
195 200 205

His Lys Pro Ser Asn Thr Lys Val Asp Lys Lys Val Glu Pro Lys Ser
210 215 220

Cys Asp Lys Thr His Thr Cys Pro Pro Cys Pro Ala Pro Glu Phe Glu
225 230 235 240

```
Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Ser
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly
    450


<210>  11
<211>  17
<212>  PRT
<213>  Artificial Sequence

<220>
<223>  CDRL1 OF TNX-355

<400>  11

Lys Ser Ser Gln Ser Leu Leu Tyr Ser Thr Asn Gln Lys Asn Tyr Leu
1               5                   10                  15


Ala


<210>  12
```

<211> 7
<212> PRT
<213> Artificial Sequence

<220>
<223> CDRL2 OF TNX-355

<400> 12

Trp Ala Ser Thr Arg Glu Ser
1 5

<210> 13
<211> 8
<212> PRT
<213> Artificial Sequence

<220>
<223> CDRL3 OF TNX-355

<400> 13

Gln Gln Tyr Tyr Ser Tyr Arg Thr
1 5

<210> 14
<211> 10
<212> PRT
<213> Artificial Sequence

<220>
<223> CDRH1 OF TNX-355

<400> 14

Gly Tyr Thr Phe Thr Ser Tyr Val Ile His
1 5 10

<210> 15
<211> 17
<212> PRT
<213> Artificial Sequence

<220>
<223> CDRH2 OF TNX-355

<400> 15

Tyr Ile Asn Pro Tyr Asn Asp Gly Thr Asp Tyr Asp Glu Lys Phe Lys
1 5 10 15

Gly

<210> 16
<211> 13
<212> PRT
<213> Artificial Sequence

<220>
<223> CDRH3 OF TNX-355

<400> 16

Glu Lys Asp Asn Tyr Ala Thr Gly Ala Trp Phe Ala Tyr
1               5                   10

<210> 17
<211> 393
<212> DNA
<213> Artificial Sequence

<220>
<223> OPTIMIZED VARIABLE HEAVY CHAIN SEQUENCE

<400> 17
atggaatgga gcggggtctt tatctttctc ctgtcagtaa ctgcaggtgt ccactccgac 60

atcgtgatga cccagtcccc tgactccctg gctgtgtccc tgggcgaacg ggtcaccatg 120

aactgcaaat cttcccagtc cctgctgtac tccaccaacc agaagaacta cctggcttgg 180

taccagcaga aacctggcca gtcccccaaa ctgctcatct actgggcttc caccagggaa 240

agcggcgtgc ctgacagatt ctccggaagc ggcagcggaa ccgacttcac cctgaccatc 300

tccagcgtgc aggctgaaga cgtggctgtc tactactgcc agcagtacta cagctacagg 360

accttcggcg gaggcaccaa gctggagatc aag 393

<210> 18
<211> 423
<212> DNA
<213> Artificial Sequence

<220>
<223> OPTIMIZED VARIABLE HEAVY CHAIN SEQUENCE

<400> 18
atggaatgga gcggggtctt tatctttctc ctgtcagtaa ctgcaggtgt ccactcccag 60

gtgcaactgc aacagtccgg acccgaagtc gtgaaaccag gagcttccgt gaagatgagc 120

tgcaaagcat ccggatacac cttcaccagc tacgtgatcc actgggtgag gcagaaacct 180

ggccagggcc tggactggat cggctacatc aacccttaca acgacggaac cgactacgac 240

gagaaattca aaggcaaagc taccctgacc agcgacacca gcacctccac tgcttacatg 300

gagctgtcca gcctgaggtc cgaagacacc gctgtgtact actgcgctag ggagaaggac 360

aactacgcta ccggcgcttg gttcgcctac tggggccagg gaaccctggt gaccgtgtcc 420

agc 423

<210> 19
<211> 34
<212> DNA

<213> Artificial Sequence

<220>
<223> OLIGONUCLEOTIDE PRIMER

<400> 19
ctagactagt ggatccagct ttctggggca ggcc 34

<210> 20
<211> 31
<212> DNA
<213> Artificial Sequence

<220>
<223> OLIGONUCLEOTIDE PRIMER

<400> 20
cctgggcatg gtgggcatgg gggaccatat t 31

<210> 21
<211> 32
<212> DNA
<213> Artificial Sequence

<220>
<223> OLIGONUCLEOTIDE PRIMER

<400> 21
ccccatgccc accatgccca ggtaagccaa cc 32

<210> 22
<211> 33
<212> DNA
<213> Artificial Sequence

<220>
<223> OLIGONUCLEOTIDE PRIMER

<400> 22
cgcctcgagg gatccagtgt ggggacagtg gga 33

<210> 23
<211> 32
<212> DNA
<213> Artificial Sequence

<220>
<223> OLIGONUCLEOTIDE PRIMER

<400> 23
tggtcccccc tcgaactcag gtgctgagga ag 32

<210> 24
<211> 32
<212> DNA
<213> Artificial Sequence

<220>

<223> OLIGONUCLEOTIDE PRIMER

<400> 24
acctgagttc gagggggac catcagtctt cc 32

<210> 25
<211> 18
<212> DNA
<213> Artificial Sequence

<220>
<223> OLIGONUCLEOTIDE PRIMER

<400> 25
gggatccact agttctag 18

<210> 26
<211> 18
<212> DNA
<213> Artificial Sequence

<220>
<223> OLIGONUCLEOTIDE PRIMER

<400> 26
tgtgaggcgt gcacgtgt 18

<210> 27
<211> 18
<212> DNA
<213> Artificial Sequence

<220>
<223> OLIGONUCLEOTIDE PRIMER

<400> 27
tccacgccgt ccacgtac 18

<210> 28
<211> 30
<212> DNA
<213> Artificial Sequence

<220>
<223> OLIGONUCLEOTIDE PRIMER

<400> 28
ggtccccct cgaattcagg tgctgaggaa 30

<210> 29
<211> 31
<212> DNA
<213> Artificial Sequence

<220>
<223> OLIGONUCLEOTIDE PRIMER

<400> 29

acctgaattc gagggggac cgtcagtctt c 31

<210> 30
<211> 18
<212> DNA
<213> Artificial Sequence

<220>
<223> OLIGONUCLEOTIDE PRIMER

<400> 30
gtacagcggt cactctca 18

<210> 31
<211> 18
<212> DNA
<213> Artificial Sequence

<220>
<223> OLIGONUCLEOTIDE PRIMER

<400> 31
gacgtgagcc acgaagac 18

<210> 32
<211> 30
<212> DNA
<213> Artificial Sequence

<220>
<223> OLIGONUCLEOTIDE PRIMER

<400> 32
tcccagccag catcgagaaa accatctcca 30

<210> 33
<211> 29
<212> DNA
<213> Artificial Sequence

<220>
<223> OLIGONUCLEOTIDE PRIMER

<400> 33
ttctcgatgc tggctgggag ggctttgtt 29

<210> 34
<211> 29
<212> DNA
<213> Artificial Sequence

<220>
<223> OLIGONUCLEOTIDE PRIMER

<400> 34
ggcttaggca tctcctatgg caggaagaa 29

<210> 35
<211> 27
<212> DNA
<213> Artificial Sequence

<220>
<223> OLIGONUCLEOTIDE PRIMER

<400> 35
ctgccaatca gggaagtagc cttgtgt 27

<210> 36
<211> 37
<212> DNA
<213> Artificial Sequence

<220>
<223> OLIGONUCLEOTIDE PRIMER

<400> 36
gatcgaattc acgcgtagca gaagacagtg gcaatga 37

<210> 37
<211> 37
<212> DNA
<213> Artificial Sequence

<220>
<223> OLIGONUCLEOTIDE PRIMER

<400> 37
gatcgtcgac tctagatttt gaccacttgc cacccat 37

<210> 38
<211> 219
<212> PRT
<213> Artificial Sequence

<220>
<223> G4H LIGHT CHAIN SEQUENCE

<400> 38

```
Asp Ile Val Met Thr Gln Ser Pro Asp Ser Leu Ala Val Ser Leu Gly
1               5                   10                  15

Glu Arg Val Thr Met Asn Cys Lys Ser Ser Gln Ser Leu Leu Tyr Ser
            20                  25                  30

Thr Asn Gln Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Val Gln Ala Glu Asp Val Ala Val Tyr Tyr Cys Gln Gln
                85                  90                  95
```

```
Tyr Tyr Ser Tyr Arg Thr Phe Gly Gly Gly Thr Lys Leu Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215


<210>  39
<211>  219
<212>  PRT
<213>  Artificial Sequence

<220>
<223>  G4D LIGHT CHAIN SEQUENCE

<400>  39

Asp Ile Val Met Thr Gln Ser Pro Asp Ser Leu Ala Val Ser Leu Gly
 1               5                  10                  15

Glu Arg Val Thr Met Asn Cys Lys Ser Ser Gln Ser Leu Leu Tyr Ser
            20                  25                  30

Thr Asn Gln Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Val Gln Ala Glu Asp Val Ala Val Tyr Tyr Cys Gln Gln
                85                  90                  95

Tyr Tyr Ser Tyr Arg Thr Phe Gly Gly Gly Thr Lys Leu Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140
```

```
Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215



<210>  40
<211>  219
<212>  PRT
<213>  Artificial Sequence

<220>
<223>  MV1 LIGHT CHAIN SEQUENCE

<400>  40

Asp Ile Val Met Thr Gln Ser Pro Asp Ser Leu Ala Val Ser Leu Gly
 1               5                  10                  15

Glu Arg Val Thr Met Asn Cys Lys Ser Ser Gln Ser Leu Leu Tyr Ser
            20                  25                  30

Thr Asn Gln Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Val Gln Ala Glu Asp Val Ala Val Tyr Tyr Cys Gln Gln
                85                  90                  95

Tyr Tyr Ser Tyr Arg Thr Phe Gly Gly Gly Thr Lys Leu Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190
```

```
Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215
```

**Description**

## CROSS REFERENCE TO RELATED APPLICATIONS

The present application claims benefit of U.S. provisional application serial no. 60/926,516, filed April 27, 2007, incorporated herein by reference.

## FIELD OF INVENTION

The present invention relates to anti-CD4 antibodies and their use in the amelioration, treatment, or prevention of diseases or disorders resulting from improper CD4 activity or metabolism, or the inappropriate or adventitious use thereof by a pathogen, such as human immunodeficiency virus (HIV). Prophylactic, immunotherapeutic and diagnostic compositions comprising those antibodies and their use in methods for preventing or treating diseases in mammals, including humans, caused by infective agents whose primary targets are $CD4^+$ T lymphocytes are also disclosed. Such diseases include acquired immune deficiency syndrome ("AIDS"), AIDS related complex, and human immunodeficiency virus infection.

## BACKGROUND

CD4 is a 433 amino acid, transmembrane glycoprotein expressed on the surface of T lymphocytes (helper and inducer cells), macrophages, and certain brain cells. T lymphocytes carrying the cell surface glycoprotein, CD4, are important components of the immune system involved in controlling antibody production by the B cells, the maturation of cytotoxic T lymphocytes (killer T cells), the maturation and activity of macrophages and natural killer cells. They are also involved either directly or indirectly with numerous other regulator and effector functions of the immune system.

In T lymphocytes ("CD4+ T lymphocytes") CD4 is known to be targeted by a variety of infectious agents, including viruses such as the human immunodeficiency virus ("HIV"). Infection and depletion of helper T cells, for example by HIV, generally occurs in a multistep process that requires viral attachment to the CD4 receptor on helper T cells, viral attachment to co-receptor CXCR4 or CCR5, viral fusion with the cell, viral uncoating, reverse transcription of viral RNA to form DNA, synthesis of a second strand of DNA, migration of the DNA to the helper T cell nucleus, integration of viral DNA into the helper T cell genome, transcription of the DNA to produce RNA, translation of viral RNA to produce a viral polyprotein, viral protease cleavage of the polyprotein to produce viral proteins, and assembly and budding of the viral proteins to form new virus which destroy the host cell.

A variety of drugs and treatment methods have been designed to interfere with one or more of these steps. Compounds are known that can inhibit attachment of the virus to the helper T cell or other target cells such as macrophages (attachment inhibitors), inhibit the fusion of the virus with the target cell (fusion inhibitors), prevent the integration of the viral DNA into the helper T cell DNA (integrase inhibitors), prevent the synthesis of DNA from viral RNA (reverse transcriptase inhibitors), or prevent the cleavage of the viral polyprotein into viral proteins by proteases (protease inhibitors), for example.

An early step in AIDS infection of T cells is the interaction between an HIV-1 epitope and CD4. The extracellular region of CD4 consists of 4 domains (D1, D2, D3, and D4). The HIV-1 gp120 binding site on CD4 comprises amino acids 40 to 60 (strand C', C", and D) in the CD4 domain 1 (D1), a stretch analogous to the CDR2 of an immunoglobulin (Ig) V domain. After the binding of gp120 to CD4, gp120 undergoes a conformational change that facilitates its binding to a chemokine co-receptor (CCR5 or CXCR4).

The epitope on HIV-1 that binds to CD4 epitopes on lymphocytes is located on an external envelope glycoprotein, gp120. The 120 serves to indicate that gp120 has a molecular weight of about 120,000 daltons. The envelope proteins of HIV include a combination of gp120 and gp41. The gp120 protein is a heavily glycosylated exterior membrane protein of about 481 amino acids. While gp41 is a smaller transmembrane protein of about 345 amino acids, which may be glycosylated. (See L. Ratner et. al., Nature 313, pp 277-84 (1985)).

Attempts to prevent HIV-1 infection by inhibiting viral attachment have met with success. For example, U.S. Pat. No. 6,309,880 to Chang, et al. discloses an epitope located within the CD4-binding region of gp120 and antibodies specific for that epitope that inhibit HIV-1 infection of human cells by diverse viral strains and isolates. U.S. Pat No. 5,817,767 to Allaway, et al. discloses compositions containing CD4-based immunoconjugates and antibodies specific for the envelope glycoprotein of HIV-1 that are said to act synergistically to neutralize HIV-1. U.S. Pat. No. 5,922,325 to Tilley, discloses what is said to be a synergistic combination of antibodies specific for H-1 envelope glycoprotein gp120. One of the antibodies specific for the V3 loop and the other antibody is said to be specific for the CD-4 binding site of gp120. U.S. Pat. No. 6,241,986 to Zolla-Pazner, discloses monoclonal antibodies that are said to be specific for the CD4-binding domain of HIV gp 120 and useful in the neutralization of HIV-1 or for the prevention of HIV infection or the treatment of a subject infected with HIV. U.S. Pat. No. 6,136,310 to Hanna discloses chimeric antibodies specific to human CD4 antigen. In addition, U.S. Pat. No. 5,871,732 to Burkly discloses anti-CD4 antibodies useful for preventing or treating diseases in mammals, including AIDS and in particular, a murine hybridoma 5A8 and a humanized 5A8 antibody are disclosed.

TNX-355 is a humanized IgG4 monoclonal antibody ("Mab") (formerly known as humanized 5A8 as disclosed in U.S. Pat. No. 5,871,732) that binds to the V2 region of CD4 and inhibits HIV-1 entry. TNX-355 is a unique anti-CD4 antibody that binds domain 2 of the extracellular portion of the CD4 receptor, thereby blocking HIV-induced syncytia formation without blocking gp120 binding and is not immunosuppressive. However, the TNX-355 molecule has several problems that accompany its manufacture and therapeutic use. For example, TNX-355 has a characteristically short half-life of several days as compared to the typical 2-3 week half-life for other examples of IgG therapeutics. It is thought that one source of instability is an *in vivo* exchange of IgG halves between different IgG4 antibodies that results in bispecific monoclonal antibodies. Such antibodies are effectively monovalent for CD4. In addition, it is believed that the internalization of the CD4 receptor may also be responsible for this reduced half-life. Thus, new antibodies are needed that have improved stability and/or potency *in vivo* or have altered Fc receptor binding and reduced ADCC activity. Moreover, there is always a need for improved expression of therapeutic molecules.

## SUMMARY

The present invention provides novel antibodies and fragments thereof that specifically bind to CD4. Such antibodies include humanized antibodies and can be effective in preventing HIV entry into $CD4^+$ cells. Antibodies and CD4 binding fragments thereof are disclosed having amino acid substitutions in the hinge region that prevent intrachain disulfide bond formation resulting in antibody molecules with surprisingly improved bivalent stability. Such antibodies undergo minimal chain shuffling in vivo and/or are more potent in blocking viral entry compared to an antibody that is effectively monovalent for CD4. Increased stability facilitates manufacturing and provides increased *in vivo* stability.

Antibodies are also disclosed that have amino acid substitutions that reduce binding to Fc receptor that also result in a surprising increase of *in vivo* stability and its surprising effect on CD4 internalization. In addition, the genes for such antibodies have been modified such that they have increased expression over their unmodified counterparts.

In one embodiment, an antibody or antigen binding fragment thereof of the invention that specifically binds CD4 comprises CDRL1, CDRL2, and CDRL3 comprising the sequences set forth in SEQ ID NO:11, SEQ ID NO:12, and SEQ ID NO:13, respectively, wherein the antibody has an increased half life *in vivo* as compared to TNX-355. In an aspect of this embodiment, the antibody further comprises CDRH1, CDRH2, and CDRH3 having the sequences set forth in SEQ ID NO:14, SEQ ID NO:15, and SEQ ID NO:16, respectively. In another aspect of this embodiment, the variable light chain region has an amino acid sequence having at least 70%, or 80%, 81%, 92%, 83%, 84%, 85%, 86%, 87%, 88%, 89%, or 90%, 91%, 92%, 93%, 94%, or 95% 96%, 97%, 98%, 99% sequence identity to the sequence set forth in SEQ ID NO:1 and the light chain constant region has an amino acid sequence having at least 70%, or 80%, 81%, 82%, 83%, 84%, 85%, 86%, 87%, 88%, 89%, or 90%, 91%, 92%, 93%, 94%, or 95% 96%, 97%, 98%, 99% sequence identity to set forth in SEQ ID NO:2. In a certain aspect of this embodiment, the variable light chain region of the antibody comprises the sequence set forth in SEQ ID NO: 1 and the light chain constant region comprises the sequence set forth in SEQ ID NO:2. In one aspect of this embodiment, the variable heavy chain region has an amino acid sequence having at least 70%, or 80%, 81 %, 82%, 83%, 84%, 85%, 86%, 87%, 88%, 89%, or 90%, 91%, 92%, 93%, 94%, or 95% 96%, 97%, 98%, 99% sequence identity to set forth in SEQ ID NO:3, wherein residue 228 of the antibody is a residue other than a serine residue.. In a certain aspect of this embodiment, the variable heavy chain region of the antibody comprises the sequence set forth in SEQ ID NO:3, wherein residue 228 of the antibody is a residue other than a serine residue. In a still further aspect, residue 228 of the antibody is a proline residue. According to this embodiment, the antibody is an IgG antibody, which can be any of IgG1, IgG2, IgG3, or IgG4.

In another embodiment, an antibody or antigen binding fragment thereof of the invention that specifically binds CD4 comprises CDRH1, CDRH2, and CDRH3 having the sequences set forth in SEQ ID NO:14, SEQ ID NO:15, and SEQ ID NO:16, respectively, wherein the antibody has an increased half life *in vivo* as compared to TNX-355. In an aspect of this embodiment, the variable light chain region has an amino acid sequence having at least 70%, or 80%, 81%, 82%, 83%, 84%, 85%, 86%, 87%, 88%, 89%, or 90%, 91%, 92%, 93%, 94%, or 95% 96%, 97%, 98%, 99% sequence identity to the sequence set forth in SEQ ID NO:1 and the light chain constant region has an amino acid sequence having at least 70%, or 80%, 81%, 82%, 83%, 84%, 85%, 86%, 87%, 88%, 89%, or 90%, 91%, 92%, 93%, 94%, or 95% 96%, 97%, 98%, 99% sequence identity to set forth in SEQ ID NO:2. In a certain aspect of this embodiment, the variable light chain region of the antibody comprises the sequence set forth in SEQ ID NO:1 and the light chain constant region comprises the sequence set forth in SEQ ID NO:2. In one aspect of this embodiment, the variable heavy chain region has an amino acid sequence having at least 70%, or 80%, 81%, 82%, 83%, 84%, 85%, 86%, 87%, 88%, 89%, or 90%, 91%, 92%, 93%, 94%, or 95% 96%, 97%, 98%, 99% sequence identity to set forth in SEQ ID NO:3, wherein residue 228 of the antibody is a residue other than a serine residue.. In a certain aspect of this embodiment, the variable heavy chain region of the antibody comprises the sequence set forth in SEQ ID NO:3, wherein residue 228 of the antibody is a residue other than a serine residue. In a still further aspect, residue 228 of the antibody is a proline residue. According to this embodiment, the antibody is an IgG antibody, which can be any of IgG1, IgG2, IgG3, or IgG4.

In another embodiment, an antibody or fragment thereof of the invention that specifically binds CD4 has an amino acid sequence having at least 70%, or 80%, 81%, 82%, 83%, 84%, 85%, 86%, 87%, 88%, 89%, or 90%, 91%, 92%, 93%, 94%, or 95% 96%, 97%, 98%, 99% sequence identity to any of those set forth in SEQ ID NO:5, SEQ ID NO:6, or SEQ ID NO:7, wherein the antibody has an increased half life *in vivo* as compared to TNX-355. In an aspect of this embodiment, the variable light chain region of the antibody comprises the sequence set forth in SEQ ID NO:1 and the light chain constant region comprises the sequence set forth in SEQ ID NO:2. In a further aspect, the variable heavy chain region of the antibody comprises the sequence set forth in SEQ ID NO:3, wherein residue 228 of the antibody is a residue other than a serine residue. In a still further aspect, residue 228 of the antibody is a proline residue. According to this embodiment, the antibody is an IgG antibody, which can be any of IgG1, IgG2, IgG3, or IgG4.

In one embodiment, an antibody or fragment thereof of the invention that specifically binds CD4 comprises a heavy chain with an amino acid sequence having at least 70%, or 80%, 81%, 82%, 83%, 84%, 85%, 86%, 87%, 88%, 89%, or 90%, 91%, 92%, 93%, 94%, or 95% 96%, 97%, 98%, 99% sequence identity to SEQ ID NO:8 and a light chain with an amino acid sequence having at least 70%, or 80%, 81%, 82%, 83%, 84%, 85%, 86%, 87%, 88%, 89%, or 90%, 91%, 92%, 93%, 94%, or 95% 96%, 97%, 98%, 99% sequence identity to SEQ ID NO:38. In a preferred embodiment, the antibody comprises the heavy chain amino acid sequence of SEQ ID NO:8 and the light chain amino acid sequence of SEQ ID NO:38.

In one embodiment, an antibody or fragment thereof of the invention that specifically binds CD4 comprises a heavy chain with an amino acid sequence having at least 70%, or 80%, 81%, 82%, 83%, 84%, 85%, 86%, 87%, 88%, 89%, or 90%, 91%, 92%, 93%, 94%, or 95% 96%, 97%, 98%, 99% sequence identity to SEQ ID NO:9 and a light chain with an amino acid sequence having at least 70%, or 80%, 81%, 82%, 83%, 84%, 85%, 86%, 87%, 88%, 89%, or 90%, 91%, 92%, 93%, 94%, or 95% 96%, 97%, 98%, 99% sequence identity to SEQ ID NO:39. In a preferred embodiment, the antibody comprises the heavy chain amino acid sequence of SEQ ID NO:9 and the light chain amino acid sequence of SEQ ID NO:39.

In one embodiment, an antibody or fragment thereof of the invention that specifically binds CD4 comprises a heavy chain with an amino acid sequence having at least 70%, or 80%, 81%, 82%, 83%, 84%, 85%, 86%, 87%, 88%, 89%, or 90%, 91%, 92%, 93%, 94%, or 95% 96%, 97%, 98%, 99% sequence identity to SEQ ID NO:10 and a light chain with an amino acid sequence having at least 70%, or 80%, 81%, 82%, 83%, 84%, 85%, 86%, 87%, 88%, 89%, or 90%, 91%, 92%, 93%, 94%, or 95% 96%, 97%, 98%, 99% sequence identity to SEQ ID NO:40. In a preferred embodiment, the antibody comprises the heavy chain amino acid sequence of SEQ ID NO:10 and the light chain amino acid sequence of SEQ ID NO:40.

In certain embodiments, the antibodies of the invention have a modification in the hinge region, and have increased stability and an increased half-life as compared to the unmodified antibody, in particular, TNX-355. In a preferred embodiment, the modification or mutation comprises a substitution at residue 228. The antibodies of the invention comprise an amino acid sequence having at least 70%, or 80%, 81%, 82%, 83%, 84%, 95%, 86%, 87%, 88%, 89%, or 90%, 91%, 92%, 93%, 94%, or 95% 96%, 97%, 98%, 99% identity to SEQ ID NO:8, 9, or 10; wherein residue 228 is a proline residue, and wherein said antibody has an increased half-life as compared to the unmodified antibody, *i.e.,* TNX-355. In a preferred embodiment, the antibodies are humanized.

The antibodies of the invention comprise an amino acid sequence having at least 70%, or 80%, 81%, 82%, 83%, 84%, 85%, 86%, 87%, 88%, 89%, or 90%, 91%, 92%, 93%, 94%, or 95% 96%, 97%, 98%, 99% identity to SEQ ID NO:8, 9, or 10; wherein residue 228 is a proline residue, and wherein at least 60% to 80% of the total antibody is maintained as a bivalent antibody. In an embodiment of the invention, at least 60% to 80% of the total antibody is maintained as a bivalent antibody for at least 200 hours in serum.

The antibodies of the invention include bivalent anti-CD4 antibodies comprising a mutation in the hinge region, wherein the half-life of the antibody is at least 1.5 to 5 times longer than that of the unmodified bivalent antibody, in particular, TNX-355. In a certain embodiment, the half-life of the antibody is at least 1.5 times longer than that of the unmodified bivalent antibody, in particular, TNX-355. In a preferred embodiment, the mutation in the hinge region comprises a substitution at residue 228. In a preferred embodiment, the antibodies of the invention are humanized.

In certain embodiments, an antibody of this invention comprises a variant Fc region such that the half-life of the antibody *in vivo* is increased or decreased relative to the parent antibody or the antibody comprising a native sequence Fc region by increasing or decreasing neonatal Fc receptor *(FcRn)* binding affinity to the antibody. For example, such an antibody can comprise an amino acid modification at any one or more of amino acid positions 238, 252, 253, 254, 255, 256, 265, 272, 286, 288, 303, 305, 307, 309, 311, 312, 317, 340, 356, 360, 362, 376, 378, 380, 382, 386, 388, 400, 413, 415, 424, 433, 434, 435, 436, 439 or 447 of the Fc region, wherein the numbering of the residues in the Fc region is that of the EU index as in Kabat. Such polypeptide variants with reduced binding to an *FcRn* may comprise an amino acid modification at any one or more of amino acid positions 252, 253, 254, 255, 288, 309, 386, 388, 400, 415, 433, 435, 436, 439 or 447 of the Fc region, wherein the numbering of the residues in the Fc region is that of the EU index as in Kabat. The above-mentioned polypeptide variants may, alternatively, display increased binding to *FcRn* and comprise an amino acid modification at any one or more of amino acid positions 238, 256, 265, 272, 286, 303, 305, 307, 311, 312, 317, 340, 356, 360, 362, 376, 378, 380, 382, 413, 424 or 434 of the Fc region, wherein the numbering of the residues in the Fc region is that of the EU index as in Kabat. In one preferred embodiment, the antibody comprises a variant Fc region that binds with increased half-life in vivo relative to the parent antibody or the antibody comprising a native sequence Fc region. In another preferred embodiment, the antibody comprises a variant Fc region that binds with increased affinity to FcRn relative to the parent antibody or the antibody comprising a native sequence Fc region.

Antibodies are disclosed that have amino acid substitutions that have improved half-life, stability and/or potency *in vivo.* The antibodies of the invention have a therapeutic effect at a lower dose; as a result these antibodies may be administered at either a lower dose or administered less frequently. In a preferred embodiment the antibodies may be administered once a week or every week. The antibodies of the invention may be formulated for intravenous or subcutaneous administration.

In a preferred embodiment, the antibody of the invention is administered once a week in a subcutaneous dosage form. In a preferred embodiment, the antibody of the invention is administered at a dose ranging from 1 to 10 mg/kg once weekly. In another preferred embodiment, the antibody of the invention is administered at a dose ranging from 1 to 15 mg/kg every other week.

The invention includes the amino acid sequences of the variable heavy and light chain of the antibodies and their corresponding nucleic acid sequences.

Other embodiments of the invention include antibodies with one or more modifications, substitutions or mutations in the constant region(s) of IgG4 or IgG1 in combination with the CDR sequences in the variable region(s) of these antibodies to obtain binding molecules that comprise one or more CDR regions or CDR-derived regions that retain the CD4-binding capacity of the parent molecule from which the CDR was (were) obtained.

An antibody of interest can be one that prevents HIV entry into CD4+ cells.

Other embodiments of the present invention include the cell lines and vectors harboring the antibody sequences of the present invention.

Other embodiments of the present invention are the uses of the antibodies for the preparation of a medicament or composition for the treatment of diseases and disorders associated with CD4 function and metabolism, including infections such as viral infections (e.g., HIV).

Other embodiments of the present invention are the uses of these antibodies in the treatment of disorders associated with normal or abnormal CD4 biology and function. The present invention addresses the problem of stability of IgG4 antibodies and various modifications, substitutions or mutations introduced in the hinge region and in the Fc region of the IgG4 molecule or an IgG 1 molecule are disclosed that have helped solve the problem of decreased stability of IgG4 antibodies such as TNX-355. The present invention also addresses the need for improved expression of the antibody and vectors optimized for mammalian expression are also disclosed.

Additional features and advantages are described herein, and will be apparent from, the following Detailed Description and the figures.

## BRIEF DESCRIPTION OF THE FIGURES

Figure 1 provides the amino acid sequences of the parent molecule TNX-355 (1A) and the improved molecules of the present invention, including G4H (Fig. 1B), G4D (Fig. 1C) and MV1 (Fig. 1D).

Figure 2 provides the codon optimized nucleic acid sequences encoding the heavy and light chain variable regions of G4D, G4H and MV1.

Figure 3 provides a comparison of the heavy chain (3A) and light chain (3B) variable region nucleic acid sequences depicted in Fig. 2 with the nucleic acid sequences of TNX-355. Boxes indicate those nucleotides that were modified in the nucleic acid sequences of the codon optimized sequence used in G4D, G4H and MV1.

Figure 4 illustrates that the G4H variant has an *in vivo* potency equivalent to the parent TNX-355 molecule at Day 1 and at Week 9.

Figure 5 provides a graphical representation of the ratio of bivalent antibody to total antibody for variants MV1, G4D, G4H and two samples of TNX-355 over time.

Figure 6 provides a bar graph showing the half-life of total and bivalent MAbs in monkey serum after a single injection of TNX-355 (355-1, 355-2) or variants MV1, G4D and G4H.

Figure 7A provides a chart showing relative titers of transfectomas expressing the original TNX-355 sequence compared to transfectomas expressing the optimized G4H sequence.

Figure 7B depicts the tabular data of 7A in a graphic representation.

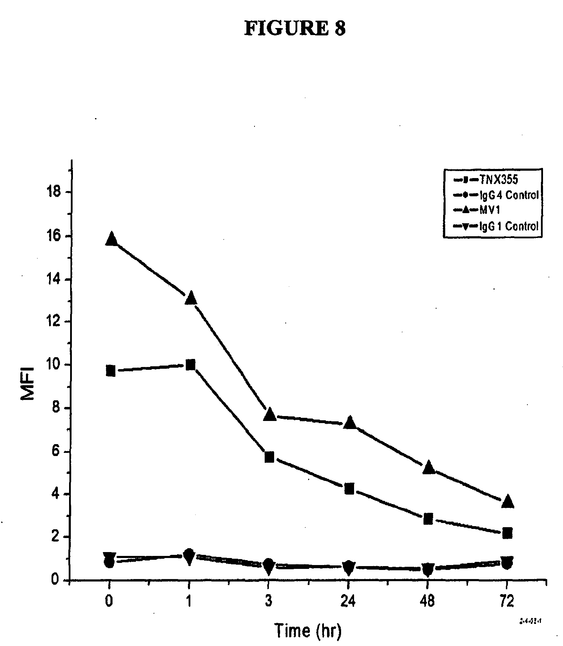

Figure 8 provides a graphical representation of CD4 mediated cell processing comparing variant MV1 (IgG1) to TNX-355 (IgG4) and two isotype controls.

Figure 9 provides a FACS analysis of CD4 levels on lymphocytes in the presence of G4H or G4D (9A) or MV1 (9B) as compared to TNX-355 or controls.

## BRIEF DESCRIPTION OF SEQUENCES

SEQ ID NO: 1 is the amino acid sequence of the variable light chain region of TNX-355.
SEQ ID NO: 2 is the amino acid sequence of the light chain constant region of TNX-355.
SEQ ID NO: 3 is the amino acid sequence of the variable heavy chain region of TNX-355.
SEQ ID NO: 4 is the amino acid sequence of the IgG4 heavy chain constant region of TNX-355.
SEQ ID NO: 5 is the amino acid sequence of the IgG4 heavy chain constant region of G4H.
SEQ ID NO: 6 is the amino acid sequence of the IgG4 heavy chain constant region of G4D.
SEQ ID NO: 7 is the amino acid sequence of the IgG1 heavy chain constant region of MV1.
SEQ ID NO: 8 is the amino acid sequence of the heavy chain of G4H.
SEQ ID NO: 9 is the amino acid sequence of the heavy chain of G4D.
SEQ ID NO: 10 is the amino acid sequence of the heavy chain of MV 1.
SEQ ID NO: 11 is the amino acid sequence of CDRL1 of TNX-355.
SEQ ID NO: 12 is the amino acid sequence of CDRL2 of TNX-355.
SEQ ID NO: 13 is the amino acid sequence of CDRL3 of TNX-355.
SEQ ID NO: 14 is the amino acid sequence of CDRH1 of TNX-355.
SEQ ID NO: 15 is the amino acid sequence of CDRH2 of TNX-355.
SEQ ID NO: 16 is the amino acid sequence of CDRH3 of TNX-355.
SEQ ID NO: 17 is the nucleic acid sequence of the codon optimized variable light chain sequence of G4H, G4D, and MV1.
SEQ ID NO: 18 is the nucleic acid sequence of the codon optimized variable heavy chain sequence of G4H, G4D, and MV1.
SEQ ID NOs 19-24 are oligonucleotide primers used in the construction of G4H and G4D.
SEQ ID NOs 25-33 are oligonucleotide primers used in the construction of MV1.
SEQ ID NOs 34-37 are oligonucleotide primers used in the generation of a population of envelope sequences from HIV viruses present in two patient samples at D1 and W9.
SEQ ID NO: 38 is the amino acid sequence of the light chain of G4H.
SEQ ID NO: 39 is the amino acid sequence of the light chain of G4D.
SEQ ID NO: 40 is the amino acid sequence of the light chain of MV1.

## DETAILED DESCRIPTION

This invention is not limited to the particular methodology, protocols, cell lines, vectors, or reagents described herein because they may vary. Further, the terminology used herein is for the purpose of describing particular embodiments only and is not intended to limit the scope of the present invention. As used herein and in the appended claims, the singular forms "a," "an," and "the" include plural reference unless the context clearly dictates otherwise, e.g., reference to "a host cell" includes a plurality of such host cells. Unless defined otherwise, all technical and scientific terms and any acronyms used herein have the same meanings as commonly understood by one of ordinary skill in the art in the field of the invention. Although any methods and materials similar or equivalent to those described herein can be used in the practice of the present invention, the exemplary methods, devices, and materials are described herein.

Throughout the present specification and claims, the numbering of the residues in an immunoglobulin heavy chain constant region is that of the EU index as in Kabat et al., Sequences of Proteins of Immunological Interest, 5th Ed. Public Health Service, National Institutes of Health, Bethesda, Md. (1991), expressly incorporated herein by reference ("EU numbering system" or "EU index as in Kabat").

All patents and publications mentioned herein are incorporated herein by reference in their entirety or to the extent allowed by law for the purpose of describing and disclosing the proteins, enzymes, vectors, host cells, and methodologies reported therein that might be used with the present invention. However, nothing herein is to be construed as an admission that the invention is not entitled to antedate such disclosure by virtue of prior invention.

## DEFINITIONS

The terms used throughout this application are to be construed with ordinary and typical meaning to those of ordinary skill in the art. However, the following definitions are provided in order to facilitate an understanding of the invention.

The phrase "substantially identical" with respect to an antibody chain polypeptide sequence may be construed as an antibody chain exhibiting at least 70%, or 80%, 81%, 82%, 83%, 84%, 85%, or 90%, or 95% sequence identity to the reference polypeptide sequence, including 90%, 91%, 92%, 93%, 94%, 95%, 96%, 97%, 98%, 99% or 100%. The term with respect to a nucleic acid sequence may be construed as a sequence of nucleotides exhibiting at least about 70%, 80%, 81%, 82%, 83%, 84%, 85%, or 90%, or 95%, or 97% sequence identity to the reference nucleic acid sequence, including 90%, 91%, 92%, 93%, 94%, 95%, 96%, 97%, 98%, 99% or 100%.

The term "identity" or "homology" shall be construed to mean the percentage of amino acid residues in the candidate sequence that are identical with the residue of a corresponding sequence to which it is compared, after aligning the sequences and introducing gaps, if necessary to achieve the maximum percent identity for the entire sequence, and not considering any conservative substitutions as part of the sequence identity. Neither N- or C-terminal extensions nor insertions shall be construed as reducing identity or homology. Methods and computer programs for the alignment are well known in the art. Sequence identity may be measured using sequence analysis software.

As used herein, the terms "about" or "approximately," unless otherwise indicated, refer to a value that is no more than 10% above or below the value being modified by the term.

The term "antibody" is used in the broadest sense, and specifically covers monoclonal antibodies (including full length monoclonal antibodies), polyclonal antibodies, and multispecific antibodies (*e.g.,* bispecific antibodies), and antibody fragments so long as they exhibit the desired biological activity. Antibodies (Abs) and immunoglobulins (Igs) are glycoproteins having the same structural characteristics. While antibodies exhibit binding specificity to a specific target, immunoglobulins include both antibodies and other antibody-like molecules which lack target specificity. The antibodies of the invention can be of any type (*e.g.,* IgG, IgE, IgM, IgD, IgA and IgY), class (*e.g.,* IgG1, IgG2, IgG3, IgG4, IgA1 and IgA2) or subclass. Native antibodies and immunoglobulins are usually heterotetrameric glycoproteins of about 150,000 Daltons, composed of two identical light (L) chains and two identical heavy (H) chains. Each heavy chain has at one end a variable domain ($V_H$) followed by a number of constant domains. Each light chain has a variable domain at one end ($V_L$) and a constant domain at its other end. Antibodies include, but are not limited to, monoclonal antibodies, polyclonal antibodies, multispecific antibodies, multispecific antibodies, human antibodies, humanized antibodies, camelized antibodies, chimeric antibodies, single domain antibodies, single chain antibodies, Fab fragments, F(ab') fragments, Fv fragments, single chain Fvs (scFv), disulfide-linked Fvs (dsFv), Fd, VH, VL, anti-idiotypic (anti-Id) antibodies (including, *e.g.,* anti-Id antibodies to antibodies of the invention), and epitope-biinding fragments of any of the above.

As used herein, "anti-CD4 antibody" means an antibody which binds specifically to human CD4 without significantly blocking binding of HIV gp120 to human CD4 in such a manner so as to be effective in decreasing, reducing, blocking or preventing HIV entry into $CD4^+$ cells.

The term "variable" in the context of variable domain of antibodies, refers to the fact that certain portions of the variable domains differ extensively in sequence among antibodies and are used in the binding and specificity of each particular antibody for its particular target. However, the variability is not evenly distributed through the variable domains of antibodies. It is concentrated in three segments called complementarity determining regions (CDRs; *i.e.,* CDR1, CDR2, and CDR3) also known as hypervariable regions both in the light chain and the heavy chain variable domains. The more highly conserved portions of variable domains are called the framework (FR). The variable domains of native heavy and light chains each comprise four FR regions, largely a adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the target binding site of antibodies (see Kabat, et al. Sequences of Proteins of Immunological Interest, National Institute of Health, Bethesda, Md. (1987)). As used herein, numbering of immunoglobulin amino acid residues is done according to the immunoglobulin amino acid residue numbering system of Kabat, *et al.,* unless otherwise indicated.

The term "antibody fragment" refers to a portion of a full-length antibody, generally the target binding or variable region. Examples of antibody fragments include F(ab), F(ab'), $F(ab')_2$ and Fv fragments. The phrase "functional fragment or analog" of an antibody is a compound having qualitative biological activity in common with a *full*-length antibody. For example, a functional fragment or analog of an anti-CD4 antibody is one which can bind to a CD4 receptor in such a manner so as to prevent or substantially reduce the ability of the receptor to bind to its ligands or initiate signaling. As used herein, "functional fragment" with respect to antibodies, refers to Fv, F(ab) and $F(ab')_2$ fragments. An "Fv" fragment consists of a dimer of one heavy and one light chain variable domain in a tight, non-covalent association ($V_H$ -$V_L$ dimer). It is in this configuration that the three CDRs of each variable domain interact to define a target binding site on the surface of the $V_H$ -$V_L$ dimer. Collectively, the six CDRs confer target binding specificity to the antibody. However, even a single variable domain (or half of an Fv comprising only three CDRs specific for a target) has the ability to recognize and bind target, although at a lower affinity than the entire binding site.

"Single-chain Fv" or "sFv" antibody fragments comprise the $V_H$ and $V_L$ domains of an antibody, wherein these domains are present in a single polypeptide chain. Generally, the Fv polypeptide further comprises a polypeptide linker between the $V_H$ and $V_L$ domains which enables the sFv to form the desired structure for target binding.

The F(ab) fragment contains the constant domain of the light chain and the first constant domain (CH1) of the heavy chain. F(ab') fragments differ from F(ab) fragments by the addition of a few residues at the carboxyl terminus of the heavy chain CH1 domain including one or more cysteines from the antibody hinge region. F(ab') fragments are produced by cleavage of the disulfide bond at the hinge cysteines of the $F(ab')_2$ pepsin digestion product. Additional chemical couplings of antibody fragments are known to those of ordinary skill in the art.

The term "monoclonal antibody" as used herein refers to an antibody obtained from a population of substantially homogeneous antibodies, *i.e.*, the individual antibodies comprising the population are identical except for possible naturally occurring mutations that may be present in minor amounts. Monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, which the remainder of the chain(s) is identical with or homologous to corresponding sequences in antibodies derived from another species or belonging to another antibody class or subclass, as well as fragments of such antibodies, so long as they exhibit the desired biological activity. Monoclonal antibodies are highly specific, being directed against a single target site. Furthermore, in contrast to conventional (polyclonal) antibody preparations which typically include different antibodies directed against different determinants (epitopes), each monoclonal antibody is directed against a single determinant on the target. In addition to their specificity, monoclonal antibodies are advantageous in that they may be synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies for use with the present invention may be isolated from phage antibody libraries using the well known techniques. The parent monoclonal antibodies may be made by the hybridoma method first described by Kohler, et al., Nature 256:495 (1975), or may be made by recombinant methods.

"Humanized" forms of non-human (e.g., murine) antibodies are chimeric immunoglobulins, immunoglobulin chains or fragments thereof (such as Fv, Fab, Fab', $F(ab')_2$ or other target-binding subsequences of antibodies) which contain minimal sequence derived from non-human immunoglobulin. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin template sequence. The humanized antibody may also comprise at least a portion of an immunoglobulin constant region (Fc), typically that of a human immunoglobulin template chosen.

"Antibody homolog" refers to any molecule which specifically binds CD4 as taught herein. Thus, an antibody homolog includes native or recombinant antibody, whether modified or not, portions of antibodies that retain the biological properties of interest, such as binding CD4, such as an $F_{ab}$ or $F_v$ molecule, a single chain antibody, a polypeptide carrying one or more CDR regions and so on. The amino acid sequence of the homolog need not be identical to that of the naturally occurring antibody but can be altered or modified to carry substitute amino acids, inserted amino acids, deleted amino acids, amino acids other than the twenty normally found in proteins and so on to obtain a polypeptide with enhanced or other beneficial properties.

A "variant Fc region" comprises an amino acid sequence which differs from that of another Fc region by virtue of at least one "amino acid modification" as herein defined. Preferably, the variant Fc region has at least one amino acid substitution compared to a native sequence Fc region or to the Fc region of a parent polypeptide, *e.g.,* from about one to about ten amino acid substitutions, and preferably from about one to about five amino acid substitutions in a native sequence Fc region or in the Fc region of the parent polypeptide. The variant Fc region herein will preferably possess at least about 80% homology with a native sequence Fc region and/or with an Fc region of a parent polypeptide, and most preferably at least about 90% homology therewith, more preferably at least about 95% homology therewith. Examples of "native sequence human Fc regions" are shown in FIG. 23 of WO 00/42072 and include a native sequence human IgG 1 Fc region (non-A and A allotypes); native sequence human IgG2 Fc region; native sequence human IgG3 Fc region; and native sequence human IgG4 Fc region as well as naturally occurring variants thereof. Native sequence murine Fc regions are shown in FIG. 22A of WO 00/42072. WO 00/42072 is incorporated by reference herein.

According to this invention, "altered" FcRn binding affinity is one which has either enhanced or diminished FcRn binding activity compared to a parent polypeptide or to a polypeptide comprising a native sequence Fc region. In one preferred embodiment, the antibody with altered FcRn binding affinity has increased binding to FcRn at pH 6.0 and/or decreased binding to FcRn at pH 7.0.

The terms "cell," "cell line," and "cell culture" include progeny. It is also understood that all progeny may not be precisely identical in DNA content, due to deliberate or inadvertent mutations. Variant progeny that have the same function or biological property, as screened for in the originally transformed cell, are included. The "host cells" used in the present invention generally are prokaryotic or eukaryotic hosts.

"Transformation" of a cellular organism, cell, or cell line with DNA means introducing DNA into the target cell so that the DNA is replicable, either as an extrachromosomal element or by chromosomal integration. "Transfection" of a cell or organism with DNA refers to the taking up of DNA, *e.g.,* an expression vector, by the cell or organism whether or not any coding sequences are in fact expressed. The terms "transfected host cell" and "transformed" refer to a cell in which DNA was introduced. The cell is termed "host cell" and it may be either prokaryotic or eukaryotic. Typical prokaryotic host cells include various strains of *E. coli.* Typical eukaryotic host cells are mammalian, such as Chinese hamster ovary or cells of human origin. The introduced DNA sequence may be from the same species as the host cell of a different species from the host cell, or it may be a hybrid DNA sequence, containing some foreign and some homologous DNA.

The term "vector" means a DNA construct containing a DNA sequence which is operably linked to a suitable control sequence capable of effecting the expression of the DNA in a suitable host. Such control sequences include a promoter to effect transcription, an optional operator sequence to control such transcription, a sequence encoding suitable mRNA ribosome binding sites, and sequences which control the termination of transcription and translation. The vector may be a plasmid, a phage particle, or simply a potential genomic insert. Once transformed into a suitable host, the vector may replicate and function independently of the host genome, or may in some instances, integrate into the genome itself. In the present specification, "plasmid" and "vector" are sometimes used interchangeably, as the plasmid is the most commonly used form of vector. However, the invention is intended to include such other forms of vectors which serve equivalent function as and which are, or become, known in the art.

The word "label" when used herein refers to a detectable compound or composition which can be conjugated directly or indirectly to a molecule or protein, *e.g.,* an antibody. The label may itself be detectable (*e.g.,* radioisotope labels or fluorescent labels) or, in the case of an enzymatic label, may catalyze chemical alteration of a substrate compound or composition which is detectable.

As used herein, "solid phase" means a non-liquid matrix to which an antibody can adhere. Example of solid phases encompassed herein include those formed partially or entirely of glass (*e.g.,* controlled pore glass), polysaccharides (*e.g.,* agarose), polyacrylamides, polystyrene, polyvinyl alcohol, and silicones. In certain embodiments, depending on the context, the solid phase can comprise the well of an assay plate; in others it is a purification column (*e.g.,* an affinity chromatography column).

As used herein, the terms "disorder" and "disease" are used interchangeably to refer to a pathological condition in a subject.

As used herein, the term "half life" refers to the time required to eliminate 50% of an antibody from the serum of an animal.

As used herein, the term "at risk" is used to describe an individual with an elevated possibility of acquiring HIV based on an increased exposure to HIV. The increased possibility of acquiring HIV can result from a number of factors including, but not limited to, the behavior, activities, occupation, family history, and/or environment of the "at risk" individual.

As used herein, the terms "expose," "exposed," and "exposing," when used in the context of exposing target cells to an antibody or a composition of the invention refer to contacting or subjecting said target cells with said antibody or composition *In vivo, i.e*., wherein said target cells are in a subject, wherein said subject is a human.

As used herein, the term "effective amount" refers to the amount of a therapy that is sufficient to result in the prevention of the development, recurrence, or onset of the disorder and one or more symptoms thereof, to enhance or improve the prophylactic effect(s) of another therapy, reduce the severity, the duration of disorder, ameliorate one or more symptoms of the disorder, prevent the advancement of the disorder, cause regression of the disorder, and/or enhance or improve the therapeutic effect(s) of another therapy.

## ANTIBODY GENERATION

Certain portions of the constant regions of antibody can be changed to provide antibody homologs, derivatives, fragments and the like with altered or improved properties over those observed in the parent antibody. For example, it is known in the art that certain IgG4 molecules have a tendency to rearrange and exchange Fab portions to form hybrids with other IgG4 molecules. Many IgG4 antibodies form intrachain disulfide bonds near the hinge region. It is thought that the intrachain bond can destabilize the intact, parent bivalent molecule forming monovalent molecules comprising a heavy chain with the associated light chain. Such molecules can re-associate, but on a random basis to form hybrid antibody molecules with unpredictable characteristics.

Modifications, substitutions or mutations to the amino acid sequence of IgG4 molecules, including in the hinge region, are disclosed that can stabilize IgG4 molecules and reduce the likelihood of forming bispecific molecules. Such modifications, substitutions or mutations can be beneficial if a therapeutic antibody is an IgG4 molecule as the enhanced stability will minimize the likelihood of having the molecule dissociate during production and manufacture, and while in vivo. A monovalent antibody may not have the same effectiveness as the bivalent parent molecule, although differences in effectiveness can be unpredictable. For example, when a native bivalent IgG4 is administered to a patient, the percentage of bivalent IgG4 decays to about 30% over a two-week period. However, amino acid substitutions, for example, at position 228 are disclosed that can enhance IgG4 stability to a surprising extent in vivo. Amino acid substitutions can be made particularly with recombinant antibodies wherein the nucleic acid coding sequence can be mutated to yield a replacement amino acid, for example, at position 228, a serine can be replaced with a proline as described herein.

A number of amino acids in the Fc region of antibodies can be candidates for modification, substitution or mutation. Candidate amino acids can include, for example, in IgG1 molecules, residues in the region from about position 233 to about position 237 (e.g., Glu-Leu-Leu-Gly-Gly); about position 252 to about position 256 (e.g., Met-Ile-Ser-Arg-Thr) and about position 318 (e.g., Glu) to about position 331 (e.g., Pro), including, for example, $Lys_{320}$, $Lys_{322}$, and $Pro_{329}$.

Further, antibodies to polypeptides such as CD4 can, in turn, be utilized to generate anti-idiotype antibodies that "mimic" such polypeptides using techniques well known to those skilled in the art (See, e.g., Greenspan, et al., FASEB J 7:437 (1989); Nissinoff, J Immunol 147:2429 (1991)). For example, antibodies which bind to and competitively inhibit polypeptide multimerization and/or binding of a polypeptide to a ligand can be used to generate anti-idiotypes that "mimic" the polypeptide multimerization and/or binding domain and, as a consequence, bind to and neutralize polypeptide and/or its ligand. Such neutralizing anti-idiotypes or Fab fragments of such anti-idiotypes can be used in therapeutic regimens to neutralize polypeptide ligand. For example, such anti-idiotypic antibodies can be used to bind a polypeptide such as CD4 and/or to bind its ligands/receptors, and thereby block its biological activity.

In addition, single-domain antibodies to CD4 can be made. Examples of this technology have been described in WO9425591 for antibodies derived from Camelidae heavy chain Ig, as well as in US20030130496 describing the isolation of single domain fully human antibodies from phage libraries.

One can also create a single peptide chain binding molecules in which the heavy and light chain Fv regions are connected. Single chain antibodies ("$scF_v$") and the method of their construction are described in U.S. Pat. No. 4,946,778. Alternatively, a $F_{ab}$ can be constructed and expressed by similar means. All of the wholly and partially human antibodies are less immunogenic than wholly murine mAbs, and the fragments and single chain antibodies are also less immunogenic.

The antibodies generated in accordance with the present invention demonstrate improved stability and increased half-life as compared to the unmodified antibody, *i.e*., the TNX-355 antibody. The antibodies generated in accordance with the invention are stably maintained as a bivalent antibody as compared to the unmodified antibody, *i.e*., TNX-355. The antibodies generated in accordance with the invention encompass a humanized bivalent antibody comprising a mutation in the hinge region, wherein the half-life of the antibody is at least 1.5 times longer than that of the unmodified antibody, *i.e.,* TNX-355.

In one embodiment, CDRL1, CDRL2, and CDRL3 of an antibody generated in accordance with the present invention have the sequences set forth in SEQ ID NO:11, SEQ ID NO:12, and SEQ ID NO:13, respectively and a Proline at residue 228.

In another embodiment, CDRH1, CDRH2, and CDRH3 of an antibody generated in accordance with the present invention have the sequences set forth in SEQ ID NO:14, SEQ ID NO:15, and SEQ ID NO:16, respectively and a Proline at residue 228.

In still another embodiment, an antibody generated in accordance with the present invention comprises CDRL1, CDRL2, and CDRL3 having the sequences set forth in SEQ ID NO:11, SEQ ID NO:12, and SEQ ID NO:13, respectively; CDRH1, CDRH2, and CDRH3 having the sequences set forth in SEQ ID NO:14, SEQ ID NO:15, and SEQ ID NO:16, respectively; and a Proline at residue 228.

In yet another embodiment, an antibody generated in accordance with the present invention comprises the variable light set forth in SEQ ID NO:1; the light chain constant region set forth in SEQ ID NO:2; and a proline at residue 228.

In an aspect of these embodiments, the fragment crystallizable (Fc) region of the antibody comprises mutations in nucleic acid sequence relative to the sequence of a native human IgG1 and/or native human IgG4 antibody.

Such mutations may be desirable in order to modify the antibody of the invention with respect to effector function, so as to enhance the effectiveness of the antibody in treating HIV. The antibody thus generated may have improved internalization capability and/or increased complement-mediated cell killing and antibody-dependent cellular cytotoxicity (ADCC). *See* Caron, et al., J. Exp Med. 176:1191-1195 (1992) and Shopes, B. J. Immunol. 148:2918-2922 (1992). Alternatively, an antibody can be engineered which has dual Fc regions and may thereby have enhanced complement lysis and ADCC capabilities. *See* Stevenson, et a/. Anti-Cancer Drug Design 3:219-230 (1989). These antibodies would retain substantially the same characteristics required for therapeutic utility as compared to their wild type counterpart.

To generate the antibody variant, one or more nucleic acid alterations (e.g. substitutions) can be introduced in the Fc region of the starting polypeptide that encodes the antibody. DNA encoding the amino acid sequence variant of the starting polypeptide can be prepared by a variety of methods known in the art. These methods include, but are not limited to, preparation by site-directed (or oligonucleotide-mediated) mutagenesis, PCR mutagenesis, and cassette mutagenesis of an earlier prepared DNA encoding the polypeptide.

The site-directed mutagenesis technique is well known in the art (*see, e.g.,* Carter, et al. Nucleic Acids Res. 13:4431-4443 (1985) and Kunkel, et al., Proc. Natl. Acad. Sci. USA 82:488 (1987)). Briefly, in carrying out site-directed mutagenesis of DNA, the starting DNA is altered by first hybridizing an oligonucleotide encoding the desired mutation to a single strand of such starting DNA. After hybridization, a DNA polymerase is used to synthesize an entire second strand, using the hybridized oligonucleotide as a primer, and using the single strand of the starting DNA as a template. Thus, the oligonucleotide encoding the desired mutation is incorporated in the resulting double-stranded DNA.

PCR mutagenesis is also suitable for making amino acid sequence variants of the starting polypeptide. *See* Higuchi, in PCR Protocols, pp.177-183 (Academic Press, 1990); and Vallette, et al., Nuc. Acids Res. 17:723-733 (1989). Briefly, when small amounts of template DNA are used as starting material in a PCR, primers that differ slightly in sequence from the corresponding region in a template DNA can be used to generate relatively large quantities of a specific DNA fragment that differs from the template sequence only at the positions where the primers differ from the template.

Another method for preparing variants, cassette mutagenesis, is based on the technique described by Wells, et al., Gene 34:315-323 (1985). The starting material is the plasmid (or other vector) comprising the starting polypeptide DNA to be mutated. The codon(s) in the starting DNA to be mutated are identified. There must be a unique restriction endonuclease site on each side of the identified mutation site(s). If no such restriction sites exist, they may be generated using the above-described oligonucleotide-mediated mutagenesis method to introduce them at appropriate locations in the starting polypeptide DNA. The plasmid DNA is cut at these sites to linearize it. A double-stranded oligonucleotide encoding the sequence of the DNA between the restriction sites but containing the desired mutation(s) is synthesized using standard procedures, wherein the two strands of the oligonucleotide are synthesized separately and then hybridized together using standard techniques. This double-stranded oligonucleotide is referred to as the cassette. This cassette is designed to have 5' and 3' ends that are compatible with the ends of the linearized plasmid, such that it can be directly ligated to the plasmid. This plasmid now contains the mutated DNA sequence.

The polypeptide variant(s) encoding the antibody with a mutated Fc region may be subjected to further modifications, oftentimes depending on the intended use of the polypeptide. Such modifications may involve further alteration of the amino acid sequence, fusion to heterologous polypeptide(s) and/or covalent modifications. With respect to further amino acid sequence alterations, any cysteine residue not involved in maintaining the proper conformation of the polypeptide variant also may be substituted, generally with serine, to improve the oxidative stability of the molecule and prevent aberrant cross linking. Polypeptide variants with altered Fc region amino acid sequences are described in U.S. Pat. No. 6,194,551B1, WO99/51642 and Idusogie, et al. J. Immunol. 164: 4178-4184 (2000), which are herein incorporated by reference in their entireties.

## IDENTIFICATION OF ANTI-CD4 ANTIBODIES

The present invention provides monoclonal antibodies that bind to CD4. Antibodies useful as starting materials for the preparation of antibodies according to the present invention include an antibody designated TNX-355 (based on the 5A8 antibody described in U.S. Pat. No. 5,871,732). The present invention includes antibodies that bind to the same epitope as that of TNX-355.

Candidate anti-CD4 antibodies can be tested by enzyme linked immunosorbent assay (ELISA), Western immunoblotting, or other immunochemical techniques.

Antibodies may be human antigen-binding antibody fragments and include, but are not limited to, $F_{ab}$, $F_{ab'}$ and $F(_{ab'})_2$, $F_d$, single-chain $F_v$s ($scF_v$), single-chain antibodies, disulfide-linked $F_v$s ($sdF_v$) and single-domain antibodies comprising either a $V_L$ or $V_H$ domain. Antigen-binding antibody fragments, including single-chain antibodies, may comprise the variable region(s) alone or in combination with the entirety or a portion of the following: hinge region, $C_{H1}$, $C_{H2}$, and $C_{H3}$ domains. Antigen-binding fragments comprising any combination of variable region(s) with a hinge region, $C_{H1}$, $C_{H2}$, and $C_{H3}$ domains.

Antibodies of the present invention may be described or specified in terms of the epitope(s) or portion(s) of CD4 which they recognize or specifically bind. The epitope(s) or polypeptide portion(s) may be specified as described herein, e.g., by N-terminal and C-terminal positions, by size in contiguous amino acid residues, conformational epitopes or listed in the Tables and Figures.

Antibodies of the present invention may also be described or specified in terms of their cross-reactivity. Antibodies that bind CD4 polypeptides, which have at least 95%, at least 90% (including 90%, 91%, 92%, 93%, 94%, 95%, 96%, 97%, 98%, 99% or 100%), at least 85%, at least 80%, at least 75%, at least 70%, at least 65%, at least 60%, at least 55%, and at least 50% identity (as calculated using methods known in the art and described herein) to CD4 are also included in the present invention. Anti-CD4 antibodies may also bind with a $K_D$ of less than about $10^{-7}$ M, less than about $10^{-6}$ M, or less than about $10^{-5}$ M to other proteins, such as anti-CD4 antibodies from species other than that against which the anti-CD4 antibody is directed.

Further included are antibodies which bind polypeptides encoded by polynucleotides which hybridize to a polynucleotide encoding CD4 under stringent hybridization conditions. Antibodies of the present invention may also be described or specified in terms of their binding affinity to a CD4 polypeptide. Preferred binding affinities include those with an equilibrium dissociation constant or $K_D$ from $10^{-8}$ to $10^{15}$ M, $10^8$ to $10^{12}$ M, $10^8$ to $10^{10}$ M, or $10^{10}$ to $10^{12}$ M. The invention also provides antibodies that competitively inhibit binding of an antibody to a CD4 epitope as determined by any method known in the art for determining competitive binding, for example, the immunoassays described herein. In preferred embodiments, the antibody competitively inhibits binding to the epitope by at least 95%, at least 90% (including 90%, 91%, 92%, 93%, 94%, 95%, 96%, 97%, 98%, 99% or 100%), at least 85%, at least 80%, at least 75%, at least 70%, at least 60%, or at least 50%.

**VECTORS AND HOST CELLS**

In another aspect, the present invention provides isolated nucleic acid sequences encoding anti-CD4 antibodies, including antibody sequences with one or more modifications, substitutions or mutations (e.g., antibody variants) as disclosed herein, vector constructs comprising nucleotide sequences encoding CD4-binding polypeptides, host cells comprising such a vector, and recombinant techniques for the production of the antibody.

For recombinant production of an antibody, including an antibody variant, the nucleic acid encoding it is isolated and inserted into a replicable vector for further cloning (amplification of the DNA) or for expression. DNA encoding the antibody, including an antibody variant, is readily isolated and sequenced using conventional procedures (*e.g.,* by using oligonucleotide probes that are capable of binding specifically to genes encoding the heavy and light chains of the antibody variant). Standard techniques for cloning and transformation may be used in the preparation of cell lines expressing the antibodies of the present invention.

VECTORS

Many vectors are available. The vector components generally include, but are not limited to, one or more of the following: a signal sequence, an origin of replication, one or more marker genes, an enhancer element, a promoter, and a transcription termination sequence. Recombinant expression vectors containing a nucleotide sequence encoding the antibodies of the present invention can be prepared using well known techniques. The expression vectors can include a nucleotide sequence operably linked to suitable transcriptional or translational regulatory nucleotide sequences such as those derived from mammalian, microbial, viral, or insect genes. Examples of regulatory sequences include transcriptional promoters, operators, enhancers, mRNA ribosomal binding sites, and/or other appropriate sequences which control transcription and translation initiation and termination. Nucleotide sequences are "operably linked" when the regulatory sequence functionally relates to the nucleotide sequence for the appropriate polypeptide. Thus, a promoter nucleotide sequence is operably linked to, *e.g.,* the antibody heavy chain sequence if the promoter nucleotide sequence controls the transcription of the appropriate nucleotide sequence.

In addition, sequences encoding appropriate signal peptides that are not naturally associated with antibody heavy and/or light chain sequences can be incorporated into expression vectors. For example, a nucleotide sequence for a signal peptide (secretory leader) may be fused in-frame to the polypeptide sequence so that the antibody is secreted to the periplasmic space or into the medium. A signal peptide that is functional in the intended host cells enhances extracellular secretion of the appropriate antibody. The signal peptide may be cleaved from the polypeptide upon secretion of antibody from the cell. Examples of such secretory signals are well known and include, e.g., those described in U.S. Pat. Nos. 5,698,435; 5,698,417; and 6,204,023.

The vector may be a plasmid vector, a single or double-stranded phage vector, or a single or double-stranded RNA or DNA viral vector. Such vectors may be introduced into cells as polynucleotides by well known techniques for introducing DNA and RNA into cells. The vectors, in the case of phage and viral vectors also may be introduced into cells as packaged or encapsulated virus by well known techniques for infection and transduction. Viral vectors may be replication competent or replication defective. In the latter case, viral propagation generally will occur only in complementing host cells. Cell-free translation systems may also be employed to produce the protein using RNAs derived from the present DNA constructs. Such vectors may include the nucleotide sequence encoding the constant region of the antibody molecule (see, e.g., PCT Publications WO 86/05807 and WO 89/01036; and U.S. Pat. No. 5,122,464) and the variable domain of the antibody may be cloned into such a vector for expression of the entire heavy or light chain.

HOST CELLS

The antibodies of the present invention can be expressed from any suitable host cell. Examples of host cells include prokaryotic, yeast, or higher eukaryotic cells and include but are not limited to microorganisms such as bacteria (e.g., *E. coli, B. subtilis)* transformed with recombinant bacteriophage DNA, plasmid DNA or cosmid DNA expression vectors containing antibody coding sequences; yeast (e.g., *Saccharomyces, Pichia)* transformed with recombinant yeast expression vectors containing antibody coding sequences; insect cell systems infected with recombinant virus expression vectors (e.g., Baculovirus) containing antibody coding sequences; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing antibody coding sequences; or mammalian cell systems (e.g.,

COS, CHO, BHK, 293, 3T3 cells) harboring recombinant expression constructs containing promoters derived from the genome of mammalian cells (e.g., metallothionein promoter) or from mammalian viruses (e.g., the adenovirus late promoter; the vaccinia virus 7.5K promoter).

Prokaryotes useful as host cells include gram negative or gram positive organisms such as *E. coli, B. subtilis, Enterobacter, Erwinia, Klebsiella, Proteus, Salmonella, Serratia,* and *Shigella,* as well as Bacilli, *Pseudomonas,* and *Streptomyces.* One preferred *E. coli* cloning host is *E. coli* 294 (ATCC 31,446), although other strains such as *E. coli* B, *E. coli* X1776 (ATCC 31,537), and *E. coli* W3110 (ATCC 27,325) are suitable. These examples are illustrative rather than limiting.

Expression vectors for use in prokaryotic host cells generally comprise one or more phenotypic selectable marker genes. A phenotypic selectable marker gene is, for example, a gene encoding a protein that confers antibiotic resistance or that supplies an autotrophic requirement. Examples of useful expression vectors for prokaryotic host cells include those derived from commercially available plasmids such as the pKK223-3 (Pharmacia Fine Chemicals, Uppsala, Sweden), pGEM1 (Promega Biotec, Madison, Wisconsin., USA), and the pET (Novagen, Madison, Wisconsin, USA) and pRSET (Invitrogen, Carlsbad, CA) series of vectors (Studier, J Mol Biol 219:37 (1991); Schoepfer, Gene 124:83 (1993)). Promoter sequences commonly used for recombinant prokaryotic host cell expression vectors include T7, (Rosenberg, et al., Gene 56:125 (1987)), β-lactamase (penicillinase), lactose promoter system (Chang, et al., Nature 275:615 (1978); Goeddel, et al., Nature 281:544 (1979)), tryptophan (trp) promoter system (Goeddel, et al., Nucl Acids Res 8:4057 (1980)), and tac promoter (Sambrook, et al., Molecular Cloning, A Laboratory Manual, 2nd ed., Cold Spring Harbor Laboratory (1990)).

Yeasts or filamentous fungi useful as host cells include those from the genus *Saccharomyces, Pichia, Actinomycetes, Kluyveromyces, Schizosaccharomyces, Candida, Trichoderma, Neurospora,* and filamentous fungi such as *Neurospora, Penicillium, Tolypocladium,* and *Aspergillus.* Yeast vectors will often contain an origin of replication sequence from a 2μ yeast plasmid, an autonomously replicating sequence (ARS), a promoter region, sequences for polyadenylation, sequences for transcription termination, and a selectable marker gene. Suitable promoter sequences for yeast vectors include, among others, promoters for metallothionein, 3-phosphoglycerate kinase (Hitzeman, et al., J Biol Chem 255:2073 (1980)) or other glycolytic enzymes (Holland, et al., Biochem 17:4900 (1978)) such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Other suitable vectors and promoters for use in yeast expression are further described in Fleer, et al., Gene 107:285 (1991). Other suitable promoters and vectors for yeast and yeast transformation protocols are well known in the art. Yeast transformation protocols are well known. One such protocol is described by Hinnen, et al., Proc Natl Acad Sci 75:1929 (1978). The Hinnen protocol selects for $Trp^+$ transformants in a selective medium.

Mammalian or insect host cell culture systems may also be employed to express recombinant antibodies. In principle, any higher eukaryotic cell culture is workable, whether from vertebrate or invertebrate culture. Examples of invertebrate cells include plant and insect cells (Luckow, et al., Bio/Technology 6:47 (1988); Miller, et al., Genetics Engineering, Setlow, et al., eds. Vol. 8, pp. 277-9, Plenam Publishing (1986); Mseda, et al., Nature 315:592 (1985)). For example, Baculovirus systems may be used for production of heterologous proteins. In an insect system, *Autographa californica* nuclear polyhedrosis virus (AcNPV) may be used as a vector to express foreign genes. The virus grows in Spodoptera frugiperda cells. The antibody coding sequence may be cloned individually into non-essential regions (for example the polyhedrin gene) of the virus and placed under control of an AcNPV promoter (for example the polyhedrin promoter). Other hosts that have been identified include Aedes, *Drosophila melanogaster,* and *Bombyx mori.* A variety of viral strains for transfection are publicly available, e.g., the L-1 variant of AcNPV and the Bm-5 strain of *Bombyx mori* NPV, and such viruses may be used as the virus herein, particularly for transfection of *Spodoptera frugiperda* cells. Moreover, plant cells cultures of cotton, corn, potato, soybean, petunia, tomato, and tobacco and also be utilized as hosts.

Vertebrate cells, and propagation of vertebrate cells, in culture (tissue culture) has become a routine procedure. See Tissue Culture, Kruse, et al., eds., Academic Press (1973). Examples of useful mammalian host cell lines are monkey kidney; human embryonic kidney line; baby hamster kidney cells; Chinese hamster ovary cells/-DHFR (CHO, Urlaub, et al., Proc Natl Acad Sci USA 77:4216 (1980)); mouse sertoli cells; human cervical carcinoma cells (HELA); canine kidney cells; human lung cells; human liver cells; mouse mammary tumor; and NS0 cells.

Host cells are transformed with vectors for antibody production and cultured in conventional nutrient media modified as appropriate for inducing promoters, transcriptional and translational control sequences, selecting transformants, or amplifying the genes encoding the desired sequences. Commonly used promoter sequences and enhancer sequences are derived from polyoma virus, Adenovirus 2, Simian virus 40 (SV40), and human cytomegalovirus (CMV). DNA sequences derived from the SV40 viral genome may be used to provide other genetic elements for expression of a structural gene sequence in a mammalian host cell, e.g., SV40 origin, early and late promoter, enhancer, splice, and polyadenylation sites. Viral early and late promoters are particularly useful because both are easily obtained from a viral genome as a fragment which may also contain a viral origin of replication. Exemplary expression vectors for use in mammalian host cells are commercially available.

Host cells are useful to produce the antibodies as described herein may be cultured in a variety of media. Commercially available media such as Ham's F10 (Sigma, St Louis, MO), Minimal Essential Medium (MEM, Sigma, St Louis, MO), RPMI-1640 (Sigma, St Louis, MO), and Dulbecco's Modified Eagle's Medium (DMEM, Sigma, St Louis, MO) are suitable for culturing host cells. In addition, any of the media described in Ham, et al., Meth Enzymol 58:44 (1979), Barnes, et al., Anal Biochem 102:255 (1980), and U.S. Pat. Nos. 4,767,704; 4,657,866; 4,560,655; 5,122,469; 5,712,163; or 6,048,728 may be used as culture media for the host cells. Any of these media may be supplemented as necessary with hormones and/or other growth factors (such as insulin, transferrin, or epidermal growth factor), salts (such as X-chlorides, where X is sodium, calcium, magnesium; and phosphates), buffers (such as HEPES), nucleotides (such as adenosine and thymidine), antibiotics (such as Gentamycin™ drug), trace elements (defined as inorganic compounds usually present at finalconcentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

**POLYNUCLEOTIDES ENCODING ANTIBODIES**

The present invention further provides polynucleotides or nucleic acids, e.g., DNA, comprising a nucleotide sequence encoding an antibody of the invention and fragments thereof. Exemplary polynucleotides include those encoding antibody chains comprising one or more of the amino acid sequences described herein. The invention also encompasses polynucleotides that hybridize under stringent or lower stringency hybridization conditions to polynucleotides that encode an antibody of the present invention.

The polynucleotides may be obtained, and the nucleotide sequence of the polynucleotides determined, by any method known in the art. For example, if the nucleotide sequence of the antibody is known, a polynucleotide encoding the antibody may be assembled from chemically synthesized oligonucleotides (e.g., as described in Kutmeier, et al., Bio/Techniques 17:242 (1994)), which, briefly, involves the synthesis of overlapping oligonucleotides containing portions of the sequence encoding the antibody, annealing and ligating of those oligonucleotides, and then amplification of the ligated oligonucleotides by PCR.

Alternatively, a polynucleotide encoding an antibody may be generated from nucleic acid from a suitable source. If a clone containing a nucleic acid encoding a particular antibody is not available, but the sequence of the antibody molecule is known, a nucleic acid encoding the immunoglobulin may be chemically synthesized or obtained from a suitable source (e.g., an antibody cDNA library, or a cDNA library generated from, or nucleic acid, preferably poly $A^+$ RNA, isolated from, any tissue or cells expressing the antibody, such as hybridoma cells selected to express an antibody of the invention) by PCR amplification using synthetic primers hybridizable to the 3' and 5' ends of the sequence or by cloning using an oligonucleotide probe specific for the particular gene sequence to identify, e.g., a cDNA clone from a cDNA library that encodes the antibody. Amplified nucleic acids generated by PCR may then be cloned into replicable cloning vectors using any method well known in the art.

Once the nucleotide sequence and corresponding amino acid sequence of the antibody is determined, the nucleotide sequence of the antibody may be manipulated using methods well known in the art for the manipulation of nucleotide sequences, e.g., recombinant DNA techniques, site directed mutagenesis, PCR, etc. (see, for example, the techniques described in Sambrook, et al., Molecular Cloning, A Laboratory Manual, 2nd ed., Cold Spring Harbor Laboratory (1990); Ausubel, et al., eds., Current Protocols in Molecular Biology, John Wiley & Sons (1998), which are both incorporated by reference herein in their entireties), to generate antibodies having a different amino acid sequence, for example to create amino acid substitutions, deletions, and/or insertions.

In some embodiments, the amino acid sequence of the heavy and/or light chain variable domains may be inspected to identify the sequences of the CDRs by well known methods, e.g., by comparison to known amino acid sequences of other heavy and light chain variable regions to determine the regions of sequence hypervariability. Using routine recombinant DNA techniques, one or more of the CDRs may be inserted within framework regions, e.g., into human framework regions to humanize a non-human antibody, as described supra. The framework regions may be naturally occurring or consensus framework regions, and preferably human framework regions (see, e.g., Chothia, et al., J Mol Biol 278: 457 (1998) for a listing of human framework regions). Preferably, the polynucleotide generated by the combination of the framework regions and CDRs encodes an antibody that specifically binds a polypeptide of the invention. Preferably, as discussed supra, one or more amino acid substitutions may be made within the framework regions, and, preferably, the amino acid substitutions improve binding of the antibody to its antigen. Additionally, such methods may be used to make amino acid substitutions or deletions of one or more variable region cysteine residues participating in an intrachain disulfide bond to generate antibody molecules lacking one or more intrachain disulfide bonds. Other alterations to the polynucleotide are within the skill of the art.

Alternatively, techniques described for the production of single chain antibodies (U.S. Pat. No. 4,946,778; Bird, Science 242:423 (1988); Huston, et al., Proc Natl Acad Sci USA 85:5879 (1988); and Ward, et al., Nature 334:544 (1989)) can be adapted to produce single chain antibodies. Single chain antibodies are formed by linking the heavy and light chain fragments of the $F_v$ region via an amino acid bridge, resulting in a single chain polypeptide. Techniques for the assembly of functional $F_v$ fragments in E. coli may also be used (Skerra, et al., Science 242:1038 (1988)).

## METHODS OF PRODUCING ANTI-CD4 ANTIBODIES

The antibodies of the invention can be produced by any method known in the art for the synthesis of antibodies, in particular, by chemical synthesis or preferably, by recombinant expression techniques.

Recombinant expression of an antibody of the invention, or fragment, derivative, or analog thereof, (e.g., a heavy or light chain of an antibody or a single chain antibody), involves construction of an expression vector containing a polynucleotide that encodes the antibody or a fragment of the antibody. Once a polynucleotide encoding an antibody molecule has been obtained, the vector for the production of the antibody may be produced by recombinant DNA technology. An expression vector can be constructed containing antibody coding sequences and appropriate transcriptional and translational control signals. These methods include, for example, in vitro recombinant DNA techniques, synthetic techniques, and in vivo genetic recombination.

The expression vector can be transferred to a host cell by conventional techniques and the transfected cells can then be cultured by conventional techniques to produce an antibody. In one aspect, vectors encoding both the heavy and light chains may be co-expressed in the host cell for expression of the entire immunoglobulin molecule, as detailed below.

A variety of host-expression vector systems may be utilized to express antibody molecules as described above. Such host-expression systems represent vehicles by which the coding sequences of interest may be produced and subsequently purified, but also represent cells which may, when transformed or transfected with the appropriate nucleotide coding sequences, express an antibody molecule in situ. Bacterial cells such as *E. coli,* and eukaryotic cells are commonly used for the expression of a recombinant antibody molecule, especially for the expression of whole recombinant antibody molecule. For example, mammalian cells such as CHO, in conjunction with a vector such as the major intermediate early gene promoter element from human cytomegalovirus, are an effective expression system for antibodies (Foecking, et al., Gene 45:101 (1986); Cockett, et al., Bio/Technology 8:2 (1990)).

In addition, a host cell strain may be chosen which modulates the expression of the inserted sequences, or modifies and processes the gene product in the specific fashion desired. Such modifications (e.g., glycosylation) and processing (e.g., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include, but are not limited to, CHO, COS, 293, 3T3, or myeloma cells.

For long-term, high-yield production of recombinant proteins, stable expression is preferred. For example, cell lines which stably express the antibody molecule may be engineered. Rather than using expression vectors which contain viral origins of replication, host cells can be transformed with DNA controlled by appropriate expression control elements (e.g., promoter, enhancer, sequences, transcription terminators, polyadenylation sites, etc.), and a selectable marker. Following the introduction of the foreign DNA, engineered cells may be allowed to grow for one to two days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the antibody molecule. Such engineered cell lines may be particularly useful in screening and evaluation of compounds that interact directly or indirectly with the antibody molecule.

A number of selection systems may be used, including but not limited to the herpes simplex virus thymidine kinase (Wigler, et al., Cell 11:223 (1977)), hypoxanthine-guanine phosphoribosyltransferase (Szybalska, et al., Proc Natl Acad Sci USA 48:202 (1992)), and adenine phosphoribosyltransferase (Lowy, et al., Cell 22:817 (1980)) genes can be employed in tk, hgprt or aprt-cells, respectively. Also, antimetabolite resistance can be used as the basis of selection for the following genes: dhfr, which confers resistance to methotrexate (Wigler, et al., Proc Natl Acad Sci USA 77:357 (1980); O'Hare, et al., Proc Natl Acad Sci USA 78:1527 (1981)); gpt, which confers resistance to mycophenolic acid (Mulligan, et al., Proc Natl Acad Sci USA 78:2072 (1981)); neo, which confers resistance to the aminoglycoside G-418 (Wu, et al., Biotherapy 3:87 (1991)); and hygro, which confers resistance to hygromycin (Santerre, et al., Gene 30:147 (1984)). Methods commonly known in the art of recombinant DNA technology may be routinely applied to select the desired recombinant clone, and such methods are described, for example, in Ausubel, et al., eds., Current Protocols in Molecular Biology, John Wiley & Sons (1993); Kriegler, Gene Transfer and Expression, A Laboratory Manual, Stockton Press (1990); and in Chapters 12 and 13, Dracopoli, et al., eds, Current Protocols in Human Genetics, John Wiley & Sons (1994); Colberre-Garapin, et al., J Mol Biol 150:1 (1981), which are incorporated by reference herein in their entireties.

The expression levels of an antibody molecule can be increased by vector amplification (for a review, see Bebbington, et al., "The use of vectors based on gene amplification for the expression of cloned genes in mammalian cells," DNA Cloning, Vol.3. Academic Press (1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse, et al., Mol Cell Biol 3:257 (1983)).

The host cell may be co-transfected with two expression vectors, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes, and is capable of expressing, both heavy and light chain polypeptides. In such situations, the light chain should be placed before the heavy chain to avoid an excess of toxic free heavy chain (Proudfoot, Nature 322:52 (1986); Kohler, Proc Natl Acad Sci USA 77:2197 (1980)). The coding sequences for the heavy and light chains may comprise cDNA or genomic DNA.

Once an antibody has been produced by an animal, chemically synthesized, or recombinantly expressed, it may be purified by any method known in the art for purification of an immunoglobulin molecule, for example, by chromatography (*e.g.,* ion exchange, affinity, particularly by affinity for the specific antigen after Protein A, and size-exclusion chromatography), centrifugation, differential solubility, or by any other standard technique for the purification of proteins. In addition, the antibodies or fragments thereof can be fused to heterologous polypeptide sequences described herein or otherwise known in the art, to facilitate purification.

Antibodies recombinantly can be fused or chemically conjugated (including both covalently and non-covalently conjugations) to a polypeptide. Fused or conjugated antibodies may be used for ease in purification. See e.g., PCT publication WO 93/21232; EP 439,095; Naramura, et al., Immunol Lett 39:91 (1994); U.S. Pat. No. 5,474,981; Gillies, et al., Proc Natl Acad Sci USA 89:1428 (1992); Fell, et al., J Immunol 146:2446 (1991), which are incorporated by reference in their entireties.

Moreover, the antibodies or fragments thereof can be fused to marker sequences, such as a peptide to facilitate purification. For example, the marker amino acid sequence can be a hexa-histidine peptide, such as the tag provided in a pQE vector (QIAGEN, Inc., Chatsworth, CA), among others, many of which are commercially available. As described in Gentz, et al., Proc Natl Acad Sci USA 86:821 (1989), for instance, hexa-histidine provides for convenient purification of the fusion protein. Other peptide tags useful for purification include, but are not limited to, the "HA" tag, which corresponds to an epitope derived from the influenza hemagglutinin protein (Wilson, et al., Cell 37:767 (1984)) and the "flag" tag.

ANTIBODY PURIFICATION

When using recombinant techniques, an antibody can be produced intracellularly, in the periplasmic space, or directly secreted into the medium. If an antibody is produced intracellularly, as a first step, the particulate debris, either host cells or lysed fragments, may be removed, for example, by centrifugation or,ultrafiltration. Carter, et al., Bio/Technology 10:163 (1992) describe a procedure for isolating antibodies which are secreted to the periplasmic space of E. coli. Briefly, cell paste is thawed in the presence of sodium acetate (pH 3.5), EDTA, and phenylmethylsulfonylfluoride (PMSF) over about 30 minutes. Cell debris can be removed by centrifugation. Where an antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

An antibody composition prepared from cells can be purified using, for example, hydroxylapatite chromatography, gel electrophoresis, dialysis, and affinity chromatography, with affinity chromatography being the preferred purification technique. The suitability of protein A as an affinity ligand depends on the species and isotype of any immunoglobulin Fc domain that is present in the antibody variant. Protein A can be used to purify antibodies that are based on human IgG1, IgG2 or IgG4 heavy chains (Lindmark, et al., J Immunol Meth 62:1 (1983)). Protein G is recommended for all mouse isotypes and for human IgG3 (Guss, et al., EMBO J 5:1567 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where an antibody comprises a $C_{H3}$ domain, the Bakerbond ABXTM resin (J. T. Baker; Phillipsburg, N.J.) can be useful for purification. Other techniques for protein purification such as fractionation on an ion-exchange column, ethanol precipitation, Reverse Phase HPLC, chromatography on silica, chromatography on heparin SEPHAROSE™ chromatography on an anion or cation exchange resin (such as a polyaspartic acid column), chromatofocusing, SDS-PAGE, and ammonium sulfate precipitation are also available depending on the antibody to be recovered.

Following any preliminary purification step(s), the mixture comprising an antibody of interest and contaminants may be subjected to low pH hydrophobic interaction chromatography using an elution buffer at a pH between about 2.5-4.5, preferably performed at low salt concentrations (e.g., from about 0-0.25 M salt).

To determine whether an antibody binds to human CD4, any conventional binding assay may be used (see, e.g., U.S. Pat. No. 5,871,732). Useful CD4 binding assays include Biacore, FACS analysis, ELISA assays, radioimmunoassays and the like, which detect binding of antibodies to human CD4. Full-length and soluble forms of human CD4 useful in such assays are described in PCT patent application PCT/US88/02940, which is herein incorporated by reference. Littman et al., Cell, 55, 541, 1988, describes the correct signal sequence cleavage site of pre-human CD4 and which was published after the filing date of PCT/US88/02940. The binding of an antibody to CD4, or to soluble fragments thereof, such as rsCD4, may conveniently be detected through the use of a secondary antibody specific for immunoglobulins of the species from which the antibody being tested was derived.

To determine whether an antibody does or does not significantly block binding of HIV gp120 to human CD4, any suitable competition assay may be used (see, e.g., U.S. Pat. No. 5,871,732). Useful assays include, for example, ELISA assays, radioimmunoassays and the like that quantify the ability of the antibody to cross block with HIV gp120 for binding to human CD4. Preferably, the ability of excess HIV gp120 to block binding of labeled human rsCD4 to immobilized antibody is measured.

Preferably, the ability of an antibody to bind to human CD4 is evaluated by testing its ability to bind to human $CD4^+$ cells (see, e.g., U.S. Pat. No. 5,871,732). Preferred $CD4^+$ cells for use in determining whether an antibody binds to human CD4 can be mammalian tissue culture cells transformed with DNA encoding full-length human CD4 and expressing the CD4 on their surface. Such cells include, for example, the CHO cells transformed with DNA encoding full-length CD4 which are described in R. A. Fisher et al., Nature, 331, 76-78 (1988) ("r-CD4-CHO cells"). Suitable r-CD4-CHO cells were deposited with the In Vitro International, Inc. culture collection in Linthicum, Md., under accession number IVI-10260 and transferred to the American Type Culture Collection, Rockville, Md., under accession number CRL 10884.

Binding of the antibody to the $CD4^+$ cell can be detected by staining the cells with a fluorescently labeled secondary antibody specific for immunoglobulins of the same species from which the antibody being tested is derived. A fluorescence activated cell sorter ("FACS") can be used to detect and quantify any binding. See generally H. M. Shapiro, Practical Flow Cytometry, Alan R. Liss, Inc., New York, N.Y. (1985).

The ability of an antibody to block binding of HIV gp120 to human CD4 may be determined by immunofluorescent staining. Parallel sets of CD4+ cells are prepared, one set stained only with the anti-CD4 antibody and the other set wherein the cells are preincubated with an excess of HIV gp120, and quantifying the degree to which the bound HIV gp120 blocks binding of the antibody to the cells. Binding of the antibody to the $CD4^+$ cells can be quantified by FACS analysis, using a fluorescently labeled secondary antibody specific for immunoglobulins of the same species from which the antibody being tested is derived.

The HIV gp120 used in assays may be provided by cells infected with HIV, by HIV itself, by host cells transformed with the gene for HIV gp120, or by isolated gp120. Preferably, a purified, soluble HIV gp120 isolated from a unicellular host transformed with a truncated HIV gp160 gene encoding HIV gp120 will be used. Purified, recombinant HIV gp120 is commercially available, for example, from Repligen (Cambridge, Mass.) and from Celltech (Berkshire, United Kingdom). Cells producing recombinant gp120 are described, for example, in Lasky et al., Science, 233, 209-12 (1986). Suitable soluble, recombinant HIV gp120 polypeptides are produced by *Spodoptera frugiperda* cells transformed with recombinant baculovirus carrying a DNA sequence encoding a CD4.

To determine whether an antibody blocks HIV-induced syncytia formation between $CD4^+$ cells, any known syncytia assay may be used (See, e.g., B.D. Walker et al. "Inhibition of Human Immunodeficiency Virus Syncytium Formation and Virus Replication by Castanospermine", PNAS 84:8120-8124 (1984)). One method utilizes HIV-infected $CD4^+$ tissue culture cells (e.g., H9) and uninfected C8166 cells, both of which may be obtained from the AIDS Reference Reagent Program, NIH, Bethesda, MD. In this method the chronically infected H9 cells are incubated in the presence of serially diluted amounts of the anti-CD4 antibody to be tested, followed by the addition of the uninfected C8166 cells. The samples are incubated and then scored for the number of syncytia formed. This number is compared with the maximum number of syncytia that would be formed in the absence of anti-CD4 or with an irrelevant antibody of the same isotype as the anti-CD4 antibody being tested.

An alternative method for comparing the syncytia blocking abilities of an antibody is the following (See U.S. Patent 5,871,732). Transformed tissue culture cells expressing recombinant HIV envelope glycoprotein (i.e., HIV gp120/gp41 after post-translational cleavage of HIV gp160) on their surface are incubated with human $CD4^+$ cells containing a detectable substance (e.g., Jurkat cells such as ATCC TIB152 labeled with $^{51}Cr$) in the presence of a known CD4 antibody, an antibody to be tested or no antibody. After incubation, the culture supematants are assayed for the detectable substance (e.g., measuring $^{51}Cr$). Cell lysis is an inevitable sequela to HIV-induced syncytia formation. Therefore, the amount of detectable substance released into a culture supernatant as a consequence of cell lysis is directly proportional to syncytia formation. In this way, the degrees to which a control CD4 antibody and a particular antibody block syncytia formation are easily quantified and compared.

A preferred transformed tissue culture cell for the above assay is a CHO cell expressing recombinant HIV envelope glycoprotein on its surface ("r-gp160-CHO cell"). Such r-gp160-CHO cells are exemplified by cultures which were deposited in the In Vitro International, Inc. culture collection, Linthicum, Md., under accession number IVI-10261 and transferred to the American Type Culture Collection, Rockville, Md., under accession number CRL 10885.

To determine whether an antibody is less immunosuppressive than a known CD4 antibody or an antibody of interest for which improvement is desired, in an in vitro tetanus toxoid-specific proliferation assay, the general protocol described in E. G. Engleman et al., J. Immunol., 127, 2124-29 (1981) is usable. In such a tetanus toxoid assay, the ability of an antibody and a control CD4 antibody to reduce tetanus toxoid-induced T cell proliferation is quantified. Typically, proliferation is measured by $^3$H-thymidine incorporation by the T cells.

To determine whether an antibody either does not bind to a polypeptide consisting of the human CD4 V1 region or whether it does bind to a polypeptide consisting of the human CD4 V1V2 region, for example, ELISA assays, radioimmunoassays (RIAs), FACS analysis or the like are useful. In such assays, the ability of an antibody to bind to either of those polypeptides may be detected through use of a secondary antibody specific for immunoglobulins of the species from which the antibody was derived.

Specific binding can be determined through inhibition studies with CD4 V 1 and CD4 V1V2 polypeptides (V1V2 is a polypeptide containing the V1 and V2 portions of the CD4 EC domain) as competitors for binding of the antibody to human CD4 displayed on the surface of a CD4$^+$ cell, using FACS analysis to quantify any inhibition.

Alternatively, determining whether an antibody binds or does not bind to a CD4 V1 polypeptide or CD4 V1V2 polypeptide is accomplished using an RIA competition assay in which the antibody is bound to the plate (directly or indirectly), and the CD4 V1 polypeptide or CD4 V1V2 polypeptide simultaneously competes with detectably labelled rsCD4 for binding to the antibody. The amount of bound rsCD4 is compared with the amount of rsCD4 bound in the absence of the CD4 V 1 or CD4 V1V2 polypeptide, allowing calculation of the percent inhibition of rsCD4 binding caused by the CD4 V 1 or CD4 V1V2 polypeptide.

Polypeptides consisting of the human CD4 V1 or CD4 V1V2 regions are CD4 $AA_3$-$AA_{115}$ or CD4 $AA_3$-$AA_{182}$, respectively, of the CD4 sequences set forth in PCT Ser. No. PCT/US88/02940 or in Maddon et al., Cell, supra, can be used in the practice of the screening assays of interest. Using the corrected numbering system of Littman et al., Cell, supra, these polypeptides correspond, respectively, to human CD4 $AA_1$-$AA_{113}$ and human CD4 $AA_1$-$AA_{180}$. A suitable CD4 V1 polypeptide also is described in Chao et al., J. Biol. Chem., 264,5812-17 (1989). A suitable CD4 V1V2 polypeptide also is described in Garlick et al., AIDS Res. Hum. Retroviruses, 6, 465-79 (1990).

To determine whether an antibody inhibits infection of CD4$^+$ cells by HIV, any indication of HIV infection could be monitored. Useful indicators of HIV infection include, for example, secretion of HIV core antigen p24 by cells chronically infected by HIV and HIV-induced syncytia formation. Inhibition of HIV infection can be determined by comparing HIV p24 levels in the presence and absence of the antibody of interest in HIV-infected CD4$^+$ cell cultures using an assay for p24 as described herein and as known in the art.

To determine whether an antibody causes no significant decrease in the number of circulating CD4$^+$ cells in vivo, the number of circulating CD4$^+$ cells isolated from a mammal within 24 hours after administration of the antibody to a mammal having normal immune function is quantified, and compared to the pre-administration number or the number in a control mammal to whom an isotype-matched antibody of irrelevant specificity has been administered instead of an antibody of this invention. Quantification of CD4$^+$ cells may be accomplished, for example, by staining the cells with fluorescently labeled anti-CD4 antibodies that do not cross block with the antibody for binding to CD4 followed by FACS analysis.

To determine the binding of antibodies of this invention against human FcRn, surface plasmon resonance using, for example, a BIAcore 3000 instrument can be used. Human FcRn is coupled to a sensor chip using an amine coupling kit. For example, a CM5 sensor chip can be activated with EDC/NHS for 7 min at 5 μl/min. 100 μg/ml of human FcRn can be injected for 30 sec to 2 min at a flow rate of 10 μl/min over the activated chip to give a final binding response unit (RU) of 100 to 200. After conjugation, the FcRn coupled chip can be blocked by an injection of 35 μl of 1M ethanolamine hydrochloride at 5μl/min.

The binding of the antibodies of this invention to human FcRn at pH 6.0 or pH 7.4 can be determined. The running buffer for the binding experiment is either PBS pH 6.0 or pH 7.4 containing 0.01% P20 and 0.02% sodium azide. Antibodies of this invention can be bufferexchanged into either pH 6.0 or pH 7.4 running buffer. In one preferred embodiment, the experiments are performed at 25°C. For the pH 6.0 run, antibodies, with concentrations ranging from 4 μM to 0.7 nM, are flowed over an FcRn coated chip at 30 μl/min for 4 min and then are allowed to dissociate from the chip for 5 min. For the pH 7.4 run, antibodies, with concentrations ranging from 12μM to 100 nM, are injected over the FcRn coated chip at 20μl/min for 1.5 min and then released for 2min. Antibodies are also flowed over an unconjugated spot on the sensor chip to allow subtraction of background non-specific binding from the binding to FcRn-coupled chip. Chip can be regenerated with 30 sec pulse of 0.1 M TRIS pH 8.3 in between injections. Steady state RU for each injection can be recorded at the end of each injection phase, and dissociation constants ($K_D$) are later calculated by plotting the steady state RU against injection concentration.

## PHARMACEUTICAL FORMULATION

Therapeutic formulations of an antibody may be prepared for storage as lyophilized formulations or aqueous solutions by mixing an antibody having the desired degree of purity with optional "pharmaceutically-acceptable" carriers, excipients or stabilizers typically employed in the art (all of which are termed "excipients"), i.e., buffering agents, stabilizing agents, preservatives, isotonifiers, non-ionic detergents, antioxidants, and other miscellaneous additives. See Remington's Pharmaceutical Sciences,16th edition, Osol, Ed. (1980). Such additives must be nontoxic to the recipients at the dosages and concentrations employed.

Buffering agents help to maintain the pH in the range which approximates physiological conditions. They are preferably present at concentration ranging from about 2 mM to about 50 mM. Suitable buffering agents include both organic and inorganic acids and salts thereof such as citrate buffers (e.g., monosodium citrate-disodium citrate mixture, citric acidtrisodium citrate mixture, citric acid-monosodium citrate mixture, etc.), succinate buffers (e.g., succinic acid-monosodium succinate mixture, succinic acid-sodium hydroxide mixture, succinic acid-disodium succinate mixture, etc.), tartrate buffers (e.g., tartaric acid-sodium tartrate mixture, tartaric acid-potassium tartrate mixture, tartaric acid-sodium hydroxide mixture, etc.), fumarate buffers (e.g., fumaric acid-monosodium fumarate mixture, etc.), fumarate buffers (e.g., fumaric acid-monosodium fumarate mixture, fumaric acid-disodium fumarate mixture, monosodium fumarate-disodium fumarate mixture, etc.), gluconate buffers (e.g., gluconic acid-sodium glyconate mixture, gluconic acid-sodium hydroxide mixture, gluconic acid-potassium glyuconate mixture, etc.), oxalate buffer (e.g., oxalic acid-sodium oxalate mixture, oxalic acid-sodium hydroxide mixture, oxalic acid-potassium oxalate mixture, etc.), lactate buffers (e.g., lactic acid-sodium lactate mixture, lactic acid-sodium hydroxide mixture, lactic acid-potassium lactate mixture, etc.) and acetate buffers (e.g., acetic acid-sodium acetate mixture, acetic acid-sodium hydroxide mixture, etc.). Additionally, there may be mentioned phosphate buffers, histidine buffers and trimethylamine salts such as Tris.

Preservatives may be added to retard microbial growth, and may be added in amounts ranging from 0.2%-1% (w/v). Suitable preservatives include phenol, benzyl alcohol, metacresol, methyl paraben, propyl paraben, octadecyldimethylbenzyl ammonium chloride, benzalconium halides (e.g., chloride, bromide, iodide), hexamethonium chloride, and alkyl parabens such as methyl or propyl paraben, catechol, resorcinol, cyclohexanol, and 3-pentanol.

"Stabilizers" may be added to ensure isotonicity of liquid compositions of antibodies and include polhydric sugar alcohols, preferably trihydric or higher sugar alcohols, such as glycerin, erythritol, arabitol, xylitol, sorbitol and mannitol.

Stabilizers refer to a broad category of excipients which can range in function from a bulking agent to an additive which solubilizes the therapeutic agent or helps to prevent denaturation or adherence to the container wall. Typical stabilizers can be polyhydric sugar alcohols (enumerated above); amino acids such as arginine, lysine, glycine, glutamine, asparagine, histidine, alanine, ornithine, L-leucine, 2-phenylalanine, glutamic acid, threonine, etc., organic sugars or sugar alcohols, such as lactose, trehalose, stachyose, mannitol, sorbitol, xylitol, ribitol, myoinisitol, galactitol, glycerol and the like, including cyclitols such as inositol; polyethylene glycol; amino acid polymers; sulfur containing reducing agents, such as urea, glutathione, thioctic acid, sodium thioglycolate, thioglycerol, alpha.-monothioglycerol and sodium thio sulfate; low molecular weight polypeptides (i.e. <10 residues); proteins such as human serum albumin, bovine serum albumin, gelatin or immunoglobulins; hydrophylic polymers, such as polyvinylpyrrolidone monosaccharides, such as xylose, mannose, fructose, glucose; disaccharides such as lactose, maltose, sucrose and trisaccacharides such as raffinose; and polysaccharides such as dextran. Stabilizers may be present in the range from 0.1 to 10,000 weights per part of weight active protein.

Non-ionic surfactants or detergents (also known as "wetting agents") may be added to help solubilize the therapeutic agent as well as to protect a therapeutic antibody against agitationinduced aggregation, which also permits the formulation to be exposed to shear surface stressed without causing denaturation of the protein. Suitable non-ionic surfactants include polysorbates (20, 80, etc.), polyoxamers (184, 188 etc.), Pluronic™ polyols, and polyoxyethylene sorbitan monoethers (TWEEN-20®, TWEEN-80®, etc.). Non-ionic surfactants may be present in a range of about 0.05 mg/ml to about 1.0 mg/ml, preferably about 0.07 mg/ml to about 0.2 mg/ml.

Additional miscellaneous excipients include bulking agents, (e.g., starch), chelating agents (e.g., EDTA), antioxidants (e.g., ascorbic acid, methionine, vitamin E), and cosolvents. The formulation may also contain more than one active compound as necessary for the particular indication being treated, preferably those with complementary activities that do not adversely affect each other. Such molecules are suitably present in combination in amounts that are effective for the purpose intended. The active ingredients may also be entrapped in microcapsule prepared, for example, by coascervation techniques or by interfacial polymerization, for example, hydroxymethylcellulose or gelatin-microcapsule and poly-(methylmethacylate) microcapsule, respectively, in colloidal drug delivery systems (for example, liposomes, albumin micropheres, microemulsions, nano-particles and nanocapsules) or in macroemulsions. Such techniques are disclosed in Remington's Pharmaceutical Sciences, 16th edition, Osal, Ed. (1980).

Formulations to be used for in vivo administration must be sterile. This is readily accomplished, for example, by filtration through sterile filtration membranes. Sustained-release preparations may be prepared. Suitable examples of sustained-release preparations include semipermeable matrices of solid hydrophobic polymers containing an antibody, which matrices are in the form of shaped articles, e.g., films, or microcapsules. Examples of sustained-release matrices include polyesters, hydrogels (for example, poly(2-hydroxyethyl-methacrylate), poly(vinylalcohol)), polylactides (U.S. Pat. No. 3,773,919), *copolymers of L*-glutamic acid and ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D- (-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37° C resulting in a loss of biological activity and possible changes in immunogenicity. Rational strategies can be devised for stabilization depending on the mechanism involved. For example, if the aggregation mechanism is discovered to be intermolecular S--S bond formation through thio-disulfide interchange, stabilization may be achieved by modifying sulfhydryl residues, lyophilizing from acidic solutions, controlling moisture content, using appropriate additives, and developing specific polymer matrix compositions.

The amount of therapeutic polypeptide, antibody, or fragment thereof which will be effective in the treatment of a particular disorder or condition will depend on the nature of the disorder or condition, and can be determined by standard clinical techniques. Where possible, it is desirable to determine the dose-response curve and the pharmaceutical compositions of antibodies first in vitro, and then in useful animal model systems prior to testing in humans.

In a preferred embodiment, an aqueous solution of therapeutic antibody or fragment thereof is administered by subcutaneous injection. Each dose may range from about 0.5 μg to about 50 μg per kilogram of body weight, or more preferably, from about 3 μg to about 30 μg per kilogram body weight.

The dosing schedule for subcutaneous administration may vary form once a month to daily depending on a number of clinical factors, including the type of disease, severity of disease, and the subject's sensitivity to the therapeutic agent.

## THERAPEUTIC USES OF ANTI-CD4 ANTIBODIES

It is contemplated that the antibodies of the present invention may be used to treat a mammal. In some embodiments, an antibody of interest is administered to a nonhuman mammal for the purposes of obtaining preclinical data, for example. Exemplary nonhuman mammals to be treated include nonhuman primates, dogs, cats, rodents and other mammals in which preclinical studies are performed. Such mammals may be established animal models for a disease to be treated with the antibody or may be used to study toxicity of the antibody of interest. In each of these embodiments, dose escalation studies may be performed on the mammal.

An antibody, with or without a therapeutic moiety conjugated to it, administered alone or in combination with other therapeutic factor(s) can be used as a therapeutic. The present invention includes antibody-based therapies which involve administering antibodies of the invention to an animal, a mammal, or a human, for treating a CD4-mediated disease, disorder, or condition. The animal or subject may be a mammal in need of particular treatment, such as a mammal having been diagnosed with a particular disorder, *e.g.,* one relating to CD4. Antibodies directed against CD4 are useful against AIDS or other HIV-related disorders. For example, by administering a therapeutically acceptable dose of an anti-CD4 antibody, or antibodies, or a cocktail of the present antibodies, or in combination with other antibodies of varying sources, disease symptoms may be ameliorated or prevented in the treated mammal, particularly humans.

Therapeutic compounds of the invention include, but are not limited to, antibodies of the invention (including fragments, analogs and derivatives thereof as described herein) and nucleic acids encoding antibodies of the invention as described below (including fragments, analogs and derivatives thereof). The antibodies of the invention can be used to treat, inhibit, or prevent diseases, disorders, or conditions associated with CD4, including, but not limited to, any one or more of the diseases, disorders, or conditions described herein. The treatment and/or prevention of diseases, disorders, or conditions associated with CD4 includes, but is not limited to, alleviating at least one symptom associated with those diseases, disorders, or conditions. Antibodies of the invention may be provided in pharmaceutically acceptable compositions as known in the art or as described herein.

The amount of an antibody which will be effective in the treatment, inhibition, and prevention of a disease or disorder associated with CD4 can be determined by standard clinical techniques. The dosage will depend on the type of disease to be treated, the severity and course of the disease, whether the antibody is administered for preventive or therapeutic purposes, previous therapy, the patient's clinical history and response to the antibody, and the discretion of the attending physician. An antibody can be administered in treatment regimes consistent with the disease, e.g., a single or a few doses over one to several days to ameliorate a disease state or periodic doses over an extended time to inhibit disease progression and prevent disease recurrence. In addition, *in vitro* assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. Effective doses may be extrapolated from dose-response curves derived from in vitro or animal model test systems.

For antibodies, the dosage administered to a patient is typically 0.1 mg/kg to 150 mg/kg of the patient's body weight. Preferably, the dosage administered to a patient is between 0.1 mg/kg and 20 mg/kg of the patient's body weight, more preferably 1 mg/kg to 10 mg/kg of the patient's body weight. Generally, human antibodies have a longer half-life within the human body than antibodies from other species due to the immune response to the foreign polypeptides. Thus, lower dosages of human antibodies and less frequent administration can be possible. Further, the dosage and frequency of administration of antibodies may be reduced by enhancing uptake and tissue penetration (e.g., into the brain) of the antibodies by modifications such as, for example, lipidation. For repeated administrations over several days or longer, depending on the condition, the treatment is sustained until a desired suppression of disease symptoms occurs. However, other dosage regimens may be useful. The progress of this therapy is easily monitored by conventional techniques and assays.

In accordance with the present invention, the antibody can be administered in treatment regimens consistent with the disease, e.g., a single or a few doses over one to several days or periodic doses over an extended time to ameliorate a disease state, inhibit disease progression and/or prevent disease recurrence. In particular, the antibody can be administered at a dose effective to reduce viral load from baseline by 50% to 90%, and preferably reduce viral load from base line by 65% to 95%. In accordance with the present invention, the reduction in viral load may be transient, preferably the reduction in viral load may be sustainable for at least a week, and most preferably sustainable for two to three weeks or longer.

The amount of an antibody or pharmaceutical composition of the invention used in the prophylactic and/or therapeutic regimens which will be effective in the prevention, treatment, and/or management of disorders can be determined by methods disclosed herein. The frequency and dosage will vary also according to factors specific for each patient depending on the specific antibody administered, the severity of the condition, the route of administration, as well as age, body, weight, response, and the past medical history of the patient. For example, the dosage of an antibody of the invention which will be effective in the treatment, prevention, and/or management of disease can be determined by administering the antibody to an animal model such as, e.g., the animal models disclosed herein or known in to those skilled in the art. In addition, in vitro assays may optionally be employed to help identify optimal dosage ranges.

In some embodiments, the prophylactic and/or therapeutic regimens comprise titrating the dosages administered to the patient so as to achieve a specified measure of therapeutic efficacy. Such measures include an improvement in CD4 activity or metabolism; a reduction in viral load; and virologic failure rates.

In certain embodiments, the dosage of the antibody, antibody of the invention in the prophylactic and/or therapeutic regimen is adjusted so as to achieve an improvement in CD4 activity or metabolism, a reduction in viral load or an improvement in virologic failure rates in a test specimen extracted from a patient after undergoing the therapeutic regimen, as compared with a reference sample. Here, the reference sample is a specimen extracted from the patient undergoing therapy, wherein the specimen is extracted from the patient at an earlier time point. In one embodiment, the reference sample is a specimen extracted from the same patient, prior to receiving the prophylactic or therapeutic regimen. In specific embodiments, the viral load in the test specimen is at least 2%, 5%, 10%, 15%, 20%, 30%, 40%, 50%, 60%, 70%, 80%, 90%, 95%, or 99% lower than in the reference sample

In other embodiments, the dosage of the antibody of the invention in the prophylactic and/or therapeutic regimen is adjusted so as to achieve a reduction in viral load in a test specimen extracted from a patient after undergoing the prophylactic and/or therapeutic regimen, as compared with a reference sample, wherein the reference sample specimen is extracted from a healthy patient.

In some embodiments, the dosage of the antibody of the invention in the prophylactic and/or therapeutic regimen is adjusted so as to achieve a viral load that falls within a predetermined reference range. In these embodiments, the viral load in a test specimen is compared with a predetermined reference range.

In one embodiment, the prophylactic and/or therapeutic regimens comprise administration of an antibody of the invention or pharmaceutical compositions thereof in multiple doses. When administered in multiple doses, the antibody or pharmaceutical compositions are administered with a frequency and in an amount sufficient to prevent, treat, and/or manage the condition. In one embodiment the prophylactic and/or therapeutic regimens comprise administering the antibody of the invention once a week, once every two weeks or every month.

In one embodiment, the frequency of administration ranges from once a day up to about once every eight weeks. In another embodiment, the frequency of administration ranges from about once a week up to about once every six weeks. In another embodiment, the frequency of administration ranges from about once every three weeks up to about once every four weeks. In certain embodiments, the antibody is administered over a period of one week to two years. In yet another embodiment, the antibody is administered over a period of two weeks or greater. In other embodiments, the antibody is administered over a period of two weeks to one year. In further embodiments, the antibody is administered over a period of two weeks to six months. In some embodiments, the antibody is administered over a period of two weeks to twelve weeks. In yet other embodiments, the antibody is administered over a period of two weeks to six weeks. In certain embodiments, the antibody is administered once a week, twice a week, three times a week, four times a week, five times a week, six times a week, or seven times a week. In preferred embodiments, the antibody is administered at least three times a week. In other preferred embodiments, the antibody is administered daily for five consecutive days, or daily for seven consecutive days. In other embodiments, the antibody is administered once a day, twice a day, three times a day, four times a day, or five times a day. In preferred embodiments, the antibody is administered three times a week over a period of two weeks.

The antibodies of the invention have been modified to increase stability and increase the half life *in vivo,* as a result the antibodies of the invention have therapeutic efficacy at a reduced dose. In some embodiments, each time the antibody is administered, it is administered at a dose of 10 mg/kg or less per week, or is administered at a dose of 15 mg/kg or less every other week. In some embodiments, the antibody is administered for one, two, three, four, five, six, seven, eight, nine, ten, eleven, twelve, thirteen, fourteen, or fifteen cycles.

In a specific embodiment, the antibody is administered at a dose of 0.001 mg/kg per week to 100 mg/kg per week. In another embodiment, the antibody is administered at a dose of 0.001 mg/kg per week to 10 mg/kg per week. In certain embodiments, the antibody is administered at a dose of 0.1 mg/kg per day or greater. In other embodiments, the antibody is administered at a dose in a range of between about 0.01 mg/kg per day to about 20 mg/kg per day. In specific embodiments, where the disease is acquired immune deficiency syndrome ("AIDS"), AIDS related complex, and human immunodeficiency virus infection, the dosage given is in a range of between greater than 0.01 mg/kg per day to about 60 mg/kg per day.

Generally, the dosage of an antibody of the invention administered to a subject is in the range of 0.1 mg/kg to 50 mg/kg, or 1 mg/kg to 50 mg/kg of the subject's body weight, more preferably in the range of 0.1 mg/kg to 25 mg/kg, or 1mg/kg to 25 mg/kg, most preferably in the range of 0.1 mg/kg to 10 mg/kg or 1 mg/kg to 10 mg/kg of the patient's body weight.

In a specific embodiment, the dosage of an antibody of the invention administered to a subject is 1mg/kg, 2 mg/kg, 3 mg/kg, 4 mg/kg, 5 mg/kg, 6 mg/kg, 7 mg/kg, 8 mg/kg, 9 mg/kg or 10 mg/kg per week.

In a specific embodiment, the prophylactic regimen comprises administering to a patient at a dosage ranging from about 0.1 to about 100,000 μg/kg of body weight of the subject receiving the antibody.

In a specific embodiment, the dosage of a antibody of the invention administered to a subject is in the range of 0.01 to 10 g/m2, and more typically, in the range of 0.1 g/m2 to 7.5 g/m2, of the subject's body weight. In one embodiment, the dosage administered to a subject is in the range of 0.5 g/m2 to 5 g/m2, or 1 g/m2 to 5 g/m2 of the subject's body's surface area.

In other embodiments, the prophylactic and/or therapeutic regimen comprises administering to a patient one or more doses of an effective amount of an antibody of the invention, wherein the dose of an effective amount achieves a plasma level of at least 0.1 μg/ml, at least 0.5 μg/ml, at least 1μg/ml, at least 2 μg/ml, at least 5 μg/ml, at least 6 μg/ml, at least 10 μg/ml, at least 15 μg/ml, at least 20 μg/ml, at least 25 μg/ml, at least 50 μg/ml, at least 100 μg/ml, at least 125 μg/ml, at least 150 μg/ml, at least 175 μg/ml, at least 200 μg/ml, at least 225 μg/ml, at least 250 μg/ml, at least 275 μg/ml, at least 300 μg/ml, at least 325 μg/ml, at least 350 μg/ml, at least 375 μg/ml, at least 400 μg/ml or at least 500 μg/ml of the antibody of the invention.

An antibody composition will be formulated, dosed and administered in a manner consistent with good medical practice. In a preferred embodiment, the antibody composition is formulated for subcutaneous administration. In another preferred embodiment, the antibody composition is formulated for intravenous administration. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "therapeutically effective amount" of the antibody variant to be administered will be governed by such considerations, and is the minimum amount necessary to prevent, ameliorate, or treat a disease or disorder. An antibody need not be, but is optionally formulated with one or more agents currently used to prevent or treat the disorder in question. The effective amount of such other agents depends on the amount of antibody present in the formulation, the type of disorder or treatment, and other factors discussed above. These are generally used in the same dosages and with administration routes as used hereinbefore or about from 1 to 99% of the heretofore employed dosages.

The antibodies of the invention may be administered alone or in combination with other types of agents or treatments, including any available treatment for HIV. For example, the antibodies of the present invention can be administered in combination with therapies that inhibit the CXCR4 or CCR5 co-receptor functions, or gp41 inhibitors. The antibodies of the present invention can also be used in combination with nucleoside inhibitors, fusion inhibitors, attachment/entry inhibitors, reverse transcriptase inhibitors, protease inhibitors, integrase inhibitors, or anti-HIV antibodies such as those disclosed by Tilley et al in U.S. Pat. No. 5,922,325 or Chang et al. in U.S. Pat. No. 6,309,880.

Administration of these therapeutic agents in combination typically is carried out over a defined time period (usually minutes, hours, days or weeks depending upon the combination selected). Combination therapy is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single capsule having a fixed ratio of each therapeutic agent or in multiple, single capsules for each of the therapeutic agents. Sequential or substantially simultaneous administration of each therapeutic agent can be effected by any appropriate route including, but not limited to, oral routes, intravenous routes, intramuscular routes, and direct absorption through mucous membrane tissues. The therapeutic agents can be administered by the same route or by different routes. For example, one component of a particular combination may be administered by intravenous injection while the other component(s) of the combination may be administered orally. The components may be administered in any therapeutically effective sequence.

In a preferred aspect, the antibody is substantially purified (e.g., substantially free from substances that limit its effect or produce undesired side-effects).

Various delivery systems are known and can be used to administer an antibody, including injection, e.g., encapsulation in liposomes, microparticles, microcapsules, recombinant cells capable of expressing the compound, receptor-mediated endocytosis (see, e.g., Wu, et al., J Biol Chem 262:4429 (1987)), construction of a nucleic acid as part of a retroviral or other vector, etc.

An anti-CD4 antibody can be administered to the mammal in any acceptable manner. Methods of introduction include but are not limited to parenteral, subcutaneous, intraperitoneal, intrapulmonary, intranasal, epidural, inhalation, and oral routes, and if desired for immunosuppressive treatment, intralesional administration. Parenteral infusions include intramuscular, intradermal, intravenous, intraarterial, or intraperitoneal administration. The antibodies or compositions may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local. In addition, it may be desirable to introduce the therapeutic antibodies or compositions of the antibodies into the central nervous system by any suitable route, including intraventricular and intrathecal injection; intraventricular injection may be facilitated by an intraventricular catheter, for example, attached to a reservoir, such as an Ommaya reservoir. In addition, the antibody is suitably administered by pulse infusion, particularly with declining doses of the antibody. Preferably the dosing is given by injections, most preferably intravenous or subcutaneous injections, depending in part on whether the administration is brief or chronic.

Pulmonary administration can also be employed, e.g., by use of an inhaler or nebulizer, and formulation with an aerosolizing agent. The antibody may also be administered into the lungs of a patient in the form of a dry powder composition (See e.g., U.S. Pat. No. 6,514,496).

In some embodiments, it may be desirable to administer the therapeutic antibodies locally to the area in need of treatment; this may be achieved by, for example, and not by way of limitation, local infusion, topical application, by injection, by means of a catheter, by means of a suppository, or by means of an implant, said implant being of a porous, non-porous, or gelatinous material, including membranes, such as sialastic membranes, or fibers. Preferably, when administering an antibody, care must be taken to use materials to which the protein does not absorb.

In another embodiment, the antibody can be delivered in a vesicle, in particular a liposome (see Langer, Science 249:1527 (1990); Treat, et al., in Liposomes in the Therapy of Infectious Disease and Cancer, Lopez-Berestein, et al., eds., 353-365 (1989); Lopez-Berestein, ibid., 317-27).

In yet another embodiment, the antibody can be delivered in a controlled release system. In one embodiment, a pump may be used (see Langer, Science 249:1527 (1990); Sefton, CRC Crit Ref Biomed Eng 14:201 (1987); Buchwald, et al., Surgery 88:507 (1980); Saudek, et al., N Engl J Med 321:574 (1989)). In another embodiment, polymeric materials can be used (see Medical Applications of Controlled Release, Langer, et al., eds., CRC Press (1974); Controlled Drug Bioavailability, Drug Product Design and Performance, Smolen, et al., eds., Wiley (1984); Ranger, et al., J Macromol Sci Rev Macromol Chem 23:61 (1983); see also Levy, et al., Science 228:190 (1985); During, et al., Ann Neurol 25:351 (1989); Howard, et al., J Neurosurg 71:105 (1989)). In yet another embodiment, a controlled release system can be placed in proximity of the therapeutic target.

The present invention also provides pharmaceutical compositions. Such compositions comprise a therapeutically effective amount of an antibody and a physiologically acceptable carrier. In a specific embodiment, the term "physiologically acceptable" means approved by a regulatory agency of the Federal or a state government or listed in the U.S. Pharmacopeia or other generally recognized pharmacopeia for use in animals, and more particularly in humans.

The term "carrier" refers to a diluent, adjuvant, excipient, or vehicle with which the therapeutic is administered. Such physiological carriers can be sterile liquids, such as water and oils, including those of petroleum, animal, vegetable, or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets; pills, capsules, powders, sustained-release formulations and the like. The composition can be formulated as a suppository, with traditional binders and carriers such as triglycerides.

Oral formulation can include standard carriers such as pharmaceutical grades of mannitol, lactose, starch, magnesium stearate, sodium saccharine, cellulose, magnesium carbonate, etc. Examples of suitable carriers are described in "Remington's Pharmaceutical Sciences" by E. W. Martin. Such compositions will contain an effective amount of the antibody, preferably in purified form, together with a suitable amount of carrier so as to provide the form for proper administration to the patient. As known in the art, the formulation will be constructed to suit the mode of administration.

In one embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

In addition, antibodies may be conjugated to various effector molecules such as heterologous polypeptides, drugs, radionucleotides, or toxins. See, e.g., PCT publications WO 92/08495; WO 91/14438; WO 89/12624; U.S. Pat. No. 5,314,995; and EP 396,387. Techniques for conjugating such therapeutic moiety to antibodies are well known, see, e.g., Amon, et al., in Monoclonal Antibodies and Cancer Therapy, Reisfeld, et al. (eds.), 243-56 Alan R. Liss (1985); Hellstrom, et al., in Controlled Drug Delivery, 2nd ed., Robinson, et al., eds., 623-53, Marcel Dekker (1987); Thorpe, in Monoclonal Antibodies '84: Biological And Clinical Applications, Pinchera, et al., eds., 475-506 (1985); Monoclonal Antibodies For Cancer Detection and Therapy, Baldwin, et al., eds., 303-16, Academic Press (1985); and Thorpe, et al., Immunol Rev 62:119 (1982). Alternatively, an antibody can be conjugated to a second antibody to form an antibody heteroconjugate, see, e.g., U.S. Pat. No. 4,676,980.

Antibody conjugates can be used for modifying a given biological response, the therapeutic agent or drug moiety is not to be construed as limited to classical chemical therapeutic agents. For example, the drug moiety may be a protein or polypeptide possessing a desired biological activity. Such proteins may include, for example, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("GCSF"), or other growth factors.

## ARTICLES OF MANUFACTURE

In another embodiment of the invention, an article of manufacture containing materials useful for the treatment of the disorders described above is provided. The article of manufacture comprises a container and a label. Suitable containers include, for example, bottles, vials, syringes, and test tubes. The containers may be formed from a variety of materials such as glass or plastic. The container holds a composition which is effective for preventing or treating the condition and may have a sterile access port (for example, the container may be an intravenous solution bag or a vial having a stopper pierceable by a hypodermic injection needle). The active agent in the composition is the antibody. The label on or associated with, the container indicates that the composition is used for treating the condition of choice. The article of manufacture may further comprise a second container comprising a pharmaceutically acceptable buffer, such as phosphate-buffered saline, Ringer's solution, and dextrose solution. It may further include other materials desirable from a commercial and user standpoint, including other buffers, diluents, filters, needles, syringes, and package inserts with instructions for use.

## EXAMPLES

The invention now will be exemplified for the benefit of the artisan by the following non-limiting examples that depict some of the embodiments by and in which the instant invention can be practiced.

EXAMPLE 1

This example describes the production of G4D and G4H anti-CD4 antibodies. The following table provides the sequences of the primers used to generate substitutions in the amino acid sequence of the IgG4 Heavy chain constant region of G4H and G4D.

| Primer | Sequence | SEQ ID NO |
|---|---|---|
| C4E1 | CTAGACTAGTGGATCCAGCTTTCTGGGGCAGGCC | 19 |
| C4ml-3 | CCTGGGCATGgTGGGCATGGGGGACCATATT | 20 |
| C4ml-5 | CCCCATGCCCAcCATGCCCAGGTAAGCCAACC | 21 |
| C4E2 | CGCCTCGAGGGATCCAGTGTGGGGACAGTGGGA | 22 |
| C4m2-3 | TGGTCCCCCCtcGAACTCAGGTGCTGAGGAAG | 23 |
| C4m2-5 | ACCTGAGTTCgaGGGGGGACCATCAGTCTTCC | 24 |

A. Generation of G4H

Three PCR reactions were done to generate IgG4H. PCR1 and PCR2 used IgG4 WT as template and PCR3 used the products of PCR1 + PCR2 as template. PCR1 reaction comprised the use of primer C4E1 (containing a BamHI site) and primer C4m1-3 (IgG4mutant1 hinge S229P 3'). PCR2 comprised the use of primer C4m1-5 (IgG4mutant1 hinge S229P 5') and primer C4E2 (containing a BamHI site). PCR3 comprised the use of primers C4E1 and C4E2. Then, the product of PCR3 was ligated into a commercially available vector following digestion with BamHI. Restriction digestion was used to check orientation and the final sequence was verified.

B. Generation of G4D

Similar to the procedure described for G4H above, three PCR reactions were done to generate G4D. PCR1 comprised the use of C4E1 and C4m2-3 (IgG4mutant2 CH2 L235E 3'). PCR2 comprised the use of primer C4m2-5 (IgG4mutant2 CH2 L235E 5') and primer C4E2. PCR3 comprised the use of primer C4E1 and C4E2. Then, the product of PCR3 was ligated into a commercially available vector following digestion with BamHI. Restriction digestion was used to check orientation and the final sequence was verified.

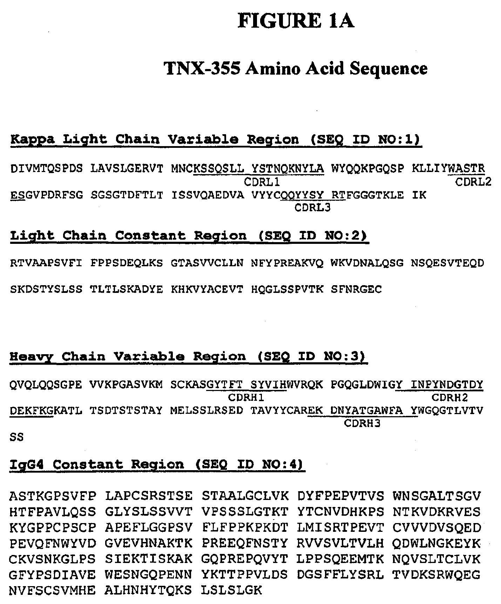

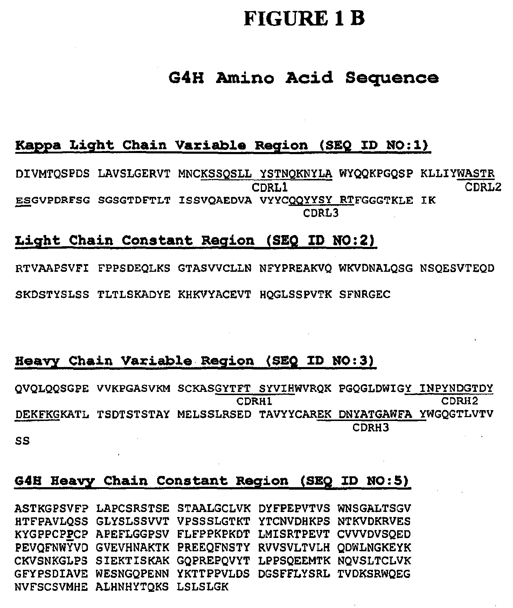

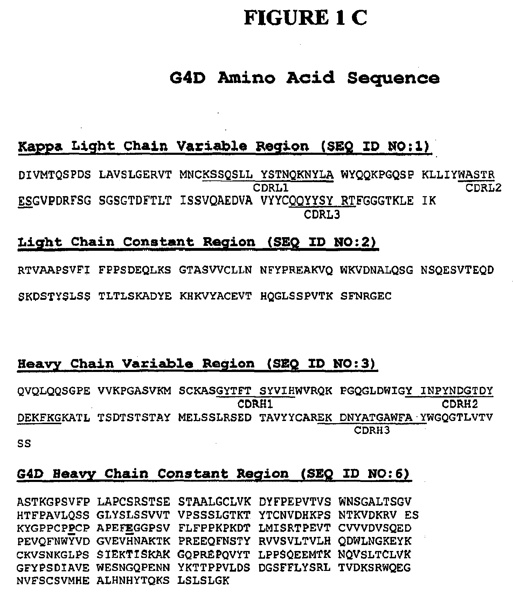

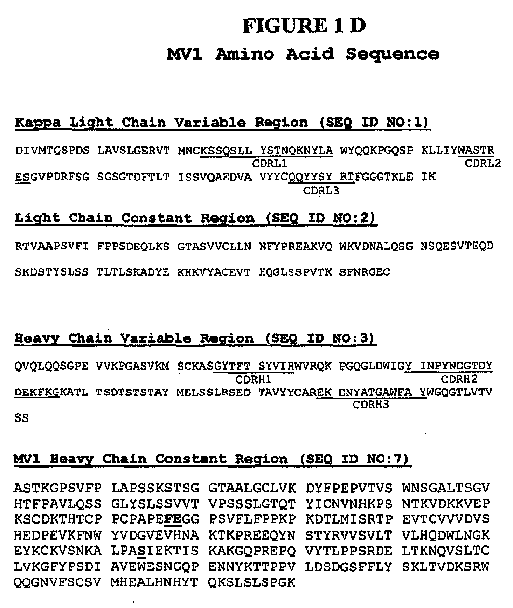

The amino acid sequences of the G4H and G4D heavy and light chains are set out in Figures 1B and 1C, respectively. The emboldened P in the constant region sequence of G4H stands for a proline which replaces a serine in the hinge region of the native TNX-355 Vh sequence at position 228. This hinge mutation prevents the strand separation characteristic of native IgG4. G4D, in addition to the Pro to Ser mutation, also contains a Glutamic acid substitution for Leucine at position 235, wherein the Fcγ receptor binding is altered thereby decreasing the effector function of IgG4.

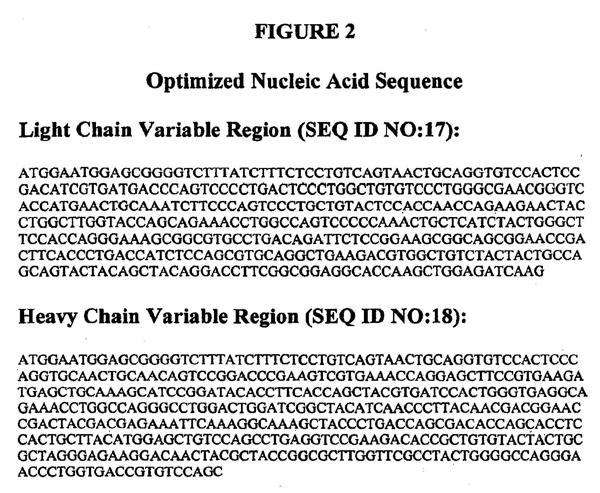

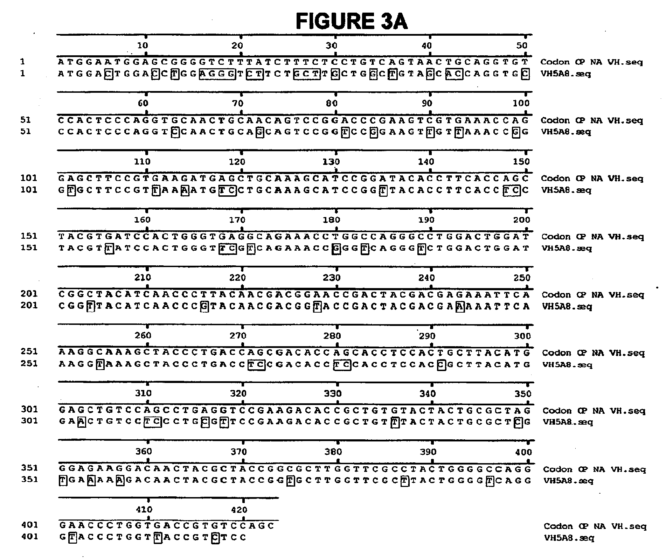

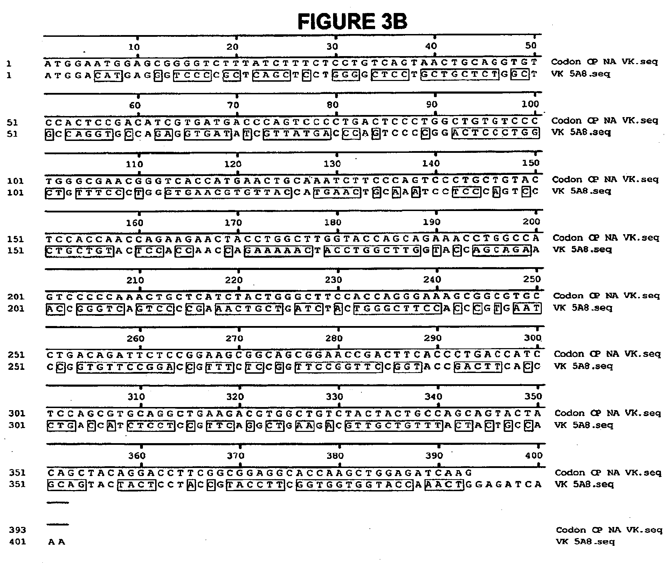

In addition, substitutions were introduced into the nucleic acid sequences of G4H and G4D in order to use more preferred codons for expression in mammalian cells, such as NS0 cells. The optimized nucleic acid sequences of the Heavy and Light chains are presented in Figure 2. Figure 3 depicts the changes in the nucleic acid sequence as compared to the parent TNX-355 nucleic acid sequence. No amino acids were changed by these substitutions.

EXAMPLE 2

An IgG1 antibody with binding affinity to CD4 was prepared according to the methods described above. The antibody designated MV1 has a leucine to glutamine change at position 234, a leucine to glycine change at position 235 and a proline to serine change at position 331 of the IgG1 constant region.

To generate IgG1 MV1 a 3 kb IgGγ1 constant region genomic DNA fragment was first amplified by PCR and cloned into a commercially available vector and then sequenced. Then, substitutions were introduced at L234F, L235E and P331S by using WT IgG1 as template. The following table provides the sequences of the primers used to generate substitutions in the amino acid sequence of the IgG1 heavy chain constant region of MV1.

| Primer | Sequence | SEQ ID NO |
|---|---|---|
| CE1 | GGGATCCACTAGTTCTAG | 25 |
| CE2 | TGTGAGGCGTGCACGTGT | 26 |
| CE3 | TCCACGCCGTCCACGTAC | 27 |
| CE4 | GGTCCCCCCTCGAATTCAGGTGCTGAGGAA | 28 |

(continued)

| Primer | Sequence | SEQ ID NO |
|---|---|---|
| CE5 | ACCTGAATTCGAGGGGGGACCGTCAGTCTTC | 29 |
| CE6 | GTACAGCGGTCACTCTCA | 30 |
| CE7 | GACGTGAGCCACGAAGAC | 31 |
| CE8 | TCCCAGCCAGCATCGAGAAAACCATCTCCA | 32 |
| CE9 | TTCTCGATGCTGGCTGGGAGGGCTTTGTT | 33 |

To generate the 3kb IgGγ1 constant region genomic DNA fragment, primers CE1 (5' primer, with SpeI site) and CE2 (3' primer, with FseI site) were used in a PCR reaction in the presence of a IgGγ1 containing vector template. This PCR product was cloned and the sequence verified.

A. Generation of the L234F and L235E Substitutions (Fragment 1)

Three PCR reactions were done to generate the L234F and L235E substitutions. PCR1 reaction comprised the use of primer CE1 and primer CE4 (3' primer, L234F, L235E) in the presence of the IgG1 DNA fragment described above. PCR2 reaction comprised the use of primer CE5 (5' primer, L234F, L235E) and CE3 (3' primer with DrdI) in the presence of the IgG 1 DNA fragment template described above. PCR3 reaction comprised the use of primers CE1 and CE3 in the presence of the PCR1 + PCR2 products to generate Fragment 1.

B. Generation of the P331S Substitution (Fragment 2)

Three PCR reactions were done to generate the P331S substitution. PCR1 reaction comprised the use of primer CE7 (5' primer, with DrdI site) and primer CE9 (3' primer P331S) in the presence of the IgG1 DNA fragment template. PCR2 reaction comprised the use of primer CE8 (5' primer P331S) and primer CE6 (3' primer with SfiI site) in the presence of the IgG1 DNA fragment template. PCR3 reaction comprised the use of primer CE7 and primer CE6 in the presence of the PCR1 + PCR2 products to generate Fragment 2.

C. Cloning fragment 1 and 2 to Generate MV1

Fragment 1 was digested with SpeI and DrdI. Fragment 2 was digested with DrdI and SfiI. The vector containing the 3kb IgG1 DNA fragment was also digested with SpeI and SfiI. Fragment 1 and Fragment 2 were ligated into the vector thereby replacing the segments in the original sequence with the Fragments containing the substituted amino acids. The sequence was then verified.

EXAMPLE 3

This example shows G4H antibodies in which serine 228 is changed to proline have similar inhibitory properties and serum stability as compared to the parent TNX-355.

HIV RNA was extracted from patient serum using QIAamp isolation kits (Qiagen, Hilden, Germany) and used to generate cDNA with random hexamer primers (SuperScript First Strand Synthesis System for RT-PCR, Invitrogen, Carlsbad, CA). HIV envelope protein open reading frame sequences were specifically amplified using a nested PCR procedure with outer PCR primers:

5'-GGCTTAGGCATCTCCTATGGCAGGAAGAA-3' (SEQ ID NO: 34) and
5'-CTGCCAATCAGGGAAGTAGCCTTGTGT-3' (SEQ ID NO: 35)

which initiate priming 239 bases upstream and 349 bases downstream of the envelope open reading frame, respectively. The inner primers used to amplify envelope open reading frame were:

5'-GATCGAATTCACGCGTAGCAGAAGACAGTGGCAATGA-3' (SEQ ID NO: 36) and
5'-GATCGTCGACTCTAGATTTTGACCACTTGCCACCCAT-3' (SEQ ID NO: 37)

These primers initiate priming at codon 2 of the envelope open reading frame and immediately downstream of the envelope termination codon, respectively. The resulting PCR products represent a population of envelope sequences from HIV viruses present in two samples, PT1 D1 (day 1) and PT1 W9 (week 9). The PCR products were then digested with appropriate restriction enzymes and cloned into a commercially available expression vector.

HIV reporter viruses for purposes of this experiment were constructed from patient derived HIV envelope protein population of vectors from (Day 1) or (Week 9). The reporter viruses were produced by co-transfecting the human embryonic kidney cell line HEK293FT (Invitrogen, Carlsbad, CA) with the envelope vectors described above and an envelope-deficient proviral DNA encoding a luciferase reporter gene. The cells were propagated in Dulbecco's Modified Eagle Medium (Invitrogen, Carlsbad, CA) supplemented with 10% fetal bovine serum (Invitrogen, Carlsbad, CA). Transfections were performed using Lipofectamine 2000 (Invitrogen, Carlsbad, CA). Culture supernatants were harvested 48 hours post-transfection and filtered through a 0.45 micron filter prior to infecting target cells. This transient transfection resulted in the generation of new viral particles comprising the luciferase reporter gene and an envelope protein derived from one of the envelope protein vectors described above.

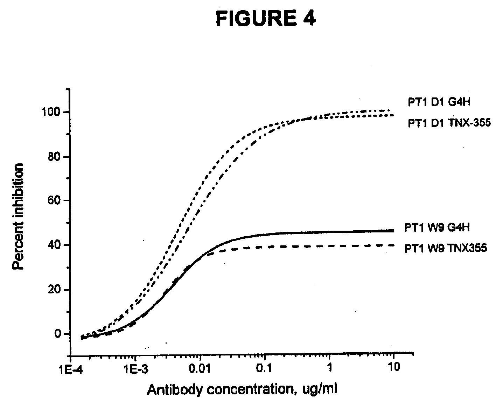

Viral infections were carried out in a human malignant glioma (U87) target cell expressing the human CD4 receptor and the human CCR5 chemokine receptor. Target cells were seeded into 96-well plates at a density of 3000 cells/well in Dulbecco's Modified Eagle Medium (Invitrogen, Carlsbad, CA) supplemented with 10% fetal bovine serum (Invitrogen, Carlsbad, CA) and cultured for 18-24. Target cells were then exposed to the virus generated above and mixed with varying concentrations of the G4H or TNX-355 anti-CD4 MAb in a final volume of 40-80 microliters. Infection was triggered by centrifugation of the 96-well plates at 1200*g for 1.5-2.0 hours at 25°C (*i.e.*, spinoculation). After spinoculation, culture medium was added to a final volume of 80 microliters per well and the infected cultures were incubated for 3 days at 37°C. After 3 days the level of luciferase expressed in target cells was measured using a luciferase reporter gene assay reagent (Promega, Madison, WI), as described by the manufacturer. Results are shown in Figure 4.

EXAMPLE 4

This example demonstrates that the serum half life of MV1, G4D and G4H is substantially increased as compared to the native TNX-355 in cynomolgus monkeys. Two groups of monkeys were administered TNX-355 MAb prepared from two different cell strains. G4H, G4D and MV1 were each administered to one group of monkeys as indicated in the Table below. Aqueous solutions of MAbs were prepared at a concentration of 5.2 mg/mL for Groups 1 and 2 and 3.4 mg/mL for Groups 3, 4, and 5. The dose volume was determined based on body weight for individual animals. Monkeys were restrained for dosing but were not sedated. Doses were administered as a bolus intravenous injection into either the saphenous or cephalic vein. The day of dosing was designated Study Day 1.

| Group Number | Test Article (10 mg/kg) | Dose (mL/kg) | Animal ID |
|---|---|---|---|
| 1 | TNX-355 (803-43.2 cell) | 2 | 19111,24056,24059,24126, |
| 2 | TNX-355 (H3 cell) | 2 | 23132; 24045, 24047, 24119, |
| 3 | G4H-IgG4 | 3 | 22938, 23123, 24058, 24111 |
| 4 | G4D-IgG4 | 3 | 22914, 22932, 24138, 24151 |
| 5 | MVI-IgG1 | 3 | 17800, 24041, 24049, 24135 |

Blood samples were collected from each monkey prior to dosing and post-dosing at 15 min, 1, 2, 4, 24, 48, 72, and 96 hr, intervals and on Days 7 and 10. Samples were drawn on several days beyond day 10, but the amount of antibody raised by the monkey's immune system interfered with the assay and therefore, the data from these samples could not be used. At each of the specific time points, approximately 1 ml of blood was collected via the peripheral vein from each animal and processed to serum according to standard techniques. All serum samples were stored upright to avoid contact with the rubber stopper; the samples were stored at or below -20°C.

A bivalent ELISA was used to quantitate monospecific bivalent MAb. In general, the present method involves incubating the sample with a capture agent and a detection agent, under appropriate conditions and for a sufficient period of time to allow binding, wherein detection of bound immune complex is directly proportional to the relative amount of bivalent antibodies present in the sample.

The assay used anti-idiotypic antibodies both to capture and detect the bivalent antibody. Bivalent antibodies were sandwiched between the capture agent bound to a solid support and detection agent, labeled with a detectable marker, in the liquid phase. Only bivalent antibodies were able to form a complex that can be detected. Monovalent antibodies present in the sample are able to bind the capture agent bound to a solid support or the detection agent present in the liquid phase. Monovalent antibodies and other materials that did not bind to the capture agent on the solid support were removed from the solid support during a wash step. Molecules bound to the capture agent, but not the detection agent, were not detected by the assay allowing a specific quantitation of bivalent molecules.

For the capture step, a solid support was coated with a capture agent. The capture agent used in the capture step may be directly or indirectly linked to the solid support. Plates were coated with 10 μg/ml anti-Id-anti-CD4 monoclonal antibody. The antibody was diluted to 10 μg/ml in phosphate buffered saline to make the coating solution. 200 μl of coating solution was added to each well of a Costar 96-well plate and incubated for 16-24 hrs in a humidified chamber. The plates were aspirated and excess solution was removed by slapping the plates on a paper towel. 200 μl of blocking solution (10 mM phosphate buffer, pH 7.4 containing 0.5% ProClin-300 (Supelco), 1% BSA, and 2.5% sucrose) was added immediately to each well and the plates were incubated overnight in a humidified chamber. The plates were then aspirated and excess solution removed before drying at room temperature. Dried plates were sealed in plastic bags and stored at 2-8°C.

HRP-conjugated anti-CD4 antibody was prepared using activated peroxidase according to the manufacturer's instructions (Zymed Laboratories). A conjugate stock at 100 μg/ml was made in 50% glycerol/PBS with 1% bovine serum albumin (BSA) and stored at - 20°C. A working dilution was prepared by further diluting the stock solution in the same buffer to make a 50X solution. The final concentration used in the assay was 143 ng/ml anti-CD4-HRP (at 1:700 dilution of the original stock in 50 mM Tris buffer, pH 7.4, containing 1% BSA, 0.05% TWEEN® 20, and 0.5% ProClin-300 (Zymed)).

Two microtiter plates were used for a single assay run. One plate was an uncoated low binding round bottom plate and the other was an anti-Id-anti-CD4 coated plate. Samples were added to the uncoated plate first and then were transferred to the anti-Id-anti-CD4 coated plate to eliminate any time-shift effect by reducing the time needed to add the samples to the wells. 10 μl reference, control or sample was added per well to an uncoated 96-well plate. Three wells were used per reference and sample. 60 μl of assay buffer (50 mM Tris buffer, pH 7.4, containing 1% BSA, 0.05% TWEEN® 20, and 0.5% ProClin-300) was pipetted into each well of the uncoated plate containing the reference or samples, using a multi-channel pipette. A dilution of anti-Id-anti-CD4-HRP conjugate (6 ml per plate) was made in the assay buffer described above and poured into a reservoir. The dilution factor was based on the conjugate lot at a 1:700 dilution or 143 ng/ml. 50 μl of the diluted conjugate was pipetted into each well of an anti-Id-anti-CD4 (10 μg/ml) coated plate using a multi-channel pipette. A 50 μl aliquot mixture of sample and assay buffer from each well of the uncoated plate was transferred to an anti-Id-anti-CD4 coated plate containing 50 μl of anti-Id-anti-CD4 conjugate per well using a multi-channel pipette.

Assay plates were incubated at room temperature on a shaker at 700 rpm for 90 ± 5 min. Assay plates were washed in 50 mM Tris buffer, pH 7.4 containing 0.9% NaCl, 0.05% TWEEN® 20 and 0.5% ProClin-300. Each well was washed 4 times. 100μl of TMB was pipetted into each well using a multi-channel pipette and each plate was incubated at room temperature on a shaker at 700 rpm for 10 min. 100 μl of stop solution (0.2 N $H_2SO_4$) was added to each well and the plate was placed briefly on the plate shaker. The OD for each well was read at 450 nm using 590 nm as the reference filter, using a program set up in REVALATION® software in Dynex plate reader, which fits a calibration curve using linear regression on log transformed concentration and log-transformed OD.

Serum concentrations of MAb for each monkey were determined using a standard competition ELISA to quantitate total MAb. In this assay, a secondary antibody, goat anti-mouse IgG (Fc specific), coated onto a solid phase (such as microtitration plate) was used as capture agent. MAb in the reference or test sample and horseradish peroxidase (HRP) labeled MAb competed for the limited binding sites on anti-idiotype antibody against anti-CD4 antibody, which is subsequently captured by the secondary antibody coated onto the plate. This complex in the well then can be detected by adding TMB (3, 3',5,5' tetramethylbenzidine) as the substrate for HRP.

A solid support was coated with goat anti-mouse IgG (Fc specific) which may be directly or indirectly linked to the solid support. HRP-labeled anti-CD4 antibody was prepared using activated peroxidase as described above. 20 ml reference, control or sample (diluted or undiluted) was added per well to the goat anti-mouse IgG Fc coated microtitration plate. Subsequently, 50 ml of the antibody-HRP to be tested was added (i.e., TNX-355-HRP, G4H-HRP, G4D-HRP or MV1-HRP) followed by the addition of 50 ml anti-idiotype antibody into each well. Plates were incubated for 90 minutes at room temperature on a shaker (~ 700 rpm). Assay plates were washed in 4 times using 50 mM Tris buffer, pH 7.4 containing 0.9% NaCl, 0.05% TWEEN® 20 and 0.5% ProClin-300. 100 ml of TMB substrate solution was added into each well, and incubated at room temperature for 10 minutes at 700 rpm. 100 μl of stop solution (0.2 N $H_2SO_4$) was added to each well and the plate was placed briefly on the plate shaker. The OD of each well was read at 450 nm using 590 nm as the reference filter. The data was analyzed using a program set up in REVALATION® software in Dynex plate reader, which fits a calibration curve using four parameter (4PL) on log transformed concentration and linear OD.

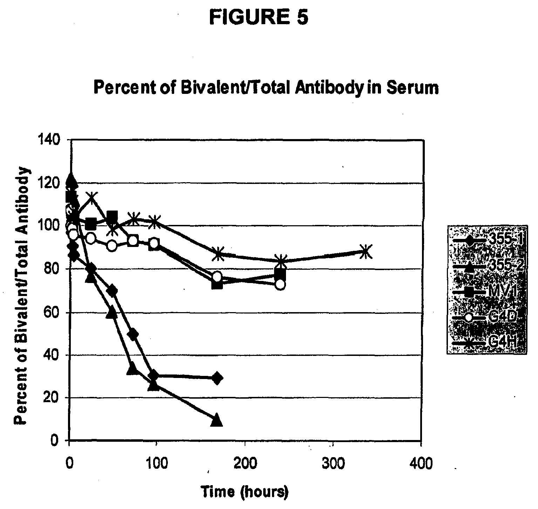

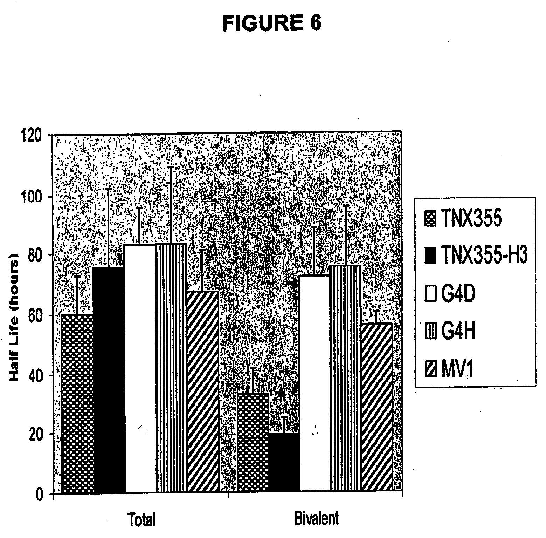

The percentage of bivalent MAb to total antibody is shown in Figure 5. The respective half lives of the bivalent form of TNX-355, MV1, G4D and G4H as compared to total MAb are presented in bar graph form in Figure 6.

EXAMPLE 5

This example demonstrates that expression of the G4H antibody can be improved by substituting nucleotides that encode more prevalent codons for mammalian cell expression into the G4H gene. The improvements are compared to the unmodified gene for TNX-355. The nucleotide substitutions are indicated in Figure 3.

NS0-eu cells were grown in mammalian cell culture medium (e.g., DMEM) to a density of $10^6$ cells/ml. The cells were maintained in exponential growth phase, and the medium was changed the day before transfection. The day of transfection, 40 x $10^6$ cells were washed and collected in 0.8 ml of DMEM + 2% FBS. Then, 10 μg of linearized nucleic acid, such as, light chain DNA, and 10 μg of, for example, linearized heavy chain DNA were added to the cell suspension (the total DNA volume should be less than 50 μl) and the culture incubated on ice for 15 min. The DNA and cell mixture was transferred to a chilled BioRad curvet (0.4 cm) and an electric pulse (750 V and 25 μF) was applied. The curvet was placed on ice immediately after the electric pulse and kept on ice for 10-15 min. The cells were collected in DMEM + 2% FBS at a density of 0.3-0.4 x $10^6$ cells/ml for 48 hrs. The recovered cells were plated in DMEM + 2% FBS + 0.1 μg/ml HXM solution (hypoxanthine, xanthine and mycophenolic acid) + 150 μg/ml of G418 at density of 0.1x$10^6$ cells/ml (200 μl/well). The cells were incubated under CO2 for 12-16 days or until colonies appear. The supernatants of the cell colonies or cells grown in suspension cultured was tested by ELISA (capture antibody was goat anti-human k and the detector antibody was a goat anti-human $F_c$ HRP-labeled antibody) and positive transfectomas were cloned in fresh medium. To further screen the positive transfectomas, either titration ELISA or the Biacore assay was conducted. Expanded transfectomas were maintained in shaker flasks.

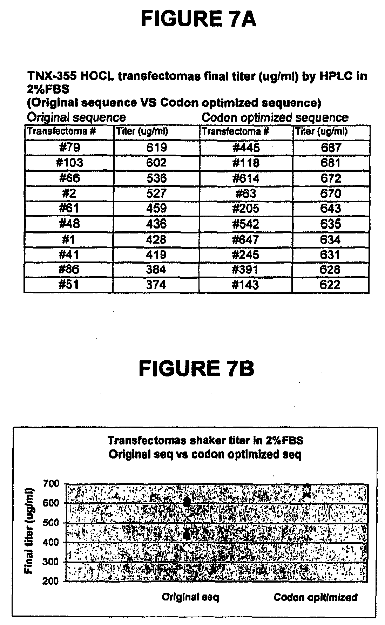

Titers from TNX-355 transfectomas from original sequence and from the codon optimized sequence are shown graphically in Figure 7B. This example demonstrates that expression of TNX-355 can be increased by optimizing codon selection to more preferred codons for mammalian cells. The titers for transfectomas expressing the optimized G4H sequence are compared to the titer for the original TNX-355 sequence in the chart of Figure 7A.

EXAMPLE 6

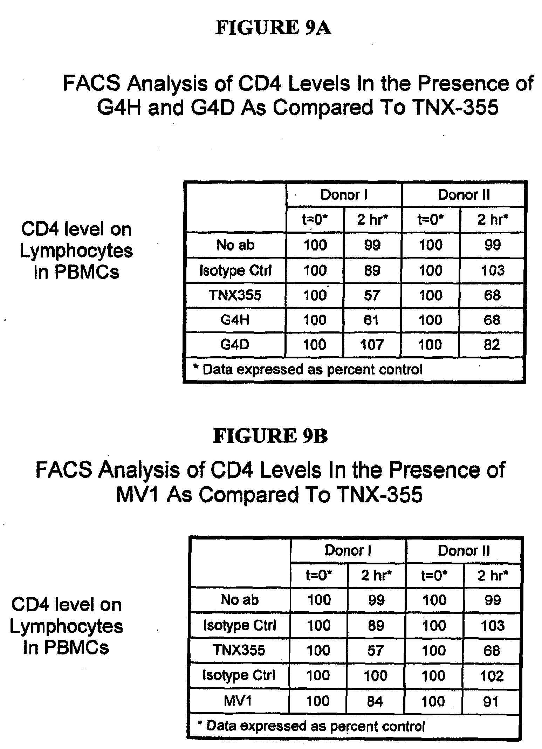

This example demonstrates that MV 1 and G4D have similar and substantially reduced internalization of the CD4 receptor, whereas G4H appears to be internalized similar to TNX-355 as illustrated by the data presented in Figures 9A and 9B. By decreasing the rate of internalization and turnover of the CD4 receptor occupied by the anti-CD4 antibody, the half-life of the antibody is improved.

Buffy coat cells were obtained from healthy volunteers (Gulf Coast Blood Center, Houston, TX). Human peripheral mononuclear cells (PBMCs) were isolated with a standard Ficol-Hypaque gradient method. PBMCs were cultured (0.5 x $10^6$ cells/well) in 96-well plate at 4°C. Each well contained 0.2 ml of RPMI 1640 supplemented with 10% FBS in the presence/absence of monoclonal antibodies (10 μg/ml) G4H (IgG4 carrying the S228P substitution), G4D (IgG4 carrying the S228P and L235E substitutions), MV1 (IgG1 carrying the L234E, L235G and P331S substitutions) and or isotype controls). After 30 minutes, the medium was replaced with fresh, cold RPMI1640 supplemented with 10% FBS and no antibodies. The cells were transferred to a 37°C humidified tissue culture chamber containing 5% $CO_2$. Monoclonal antibody treated cells were harvested immediately, 2 hr, or 24 hr after transferring the cells to 37°C. Cells were washed once with PBS and incubated in cold PBS containing 1 % BSA (PBSB) for 30 minutes. Cells were then stained with PE conjugated anti-human CD4 (BD Biosciences). After 30 minutes, cells were washed 3 times with PBSB and fixed in 1% paraformaldehyde solution overnight. The next day CD4 was analyzed with BD FACSCalibur™ system flow cytometer (BD Biosciences, San Jose, CA).

PMBC lymphocytes were assessed. The "no antibody" control and isotype control antibody did not cause CD4 internalization at time 0 and at two hours. In lymphocytes, G4D, the double mutant, did not cause CD4 internalization. The G4H mutant, however, did result in CD4 internalization. MV1 does not cause CD4 internalization in either cell type over the two tested time points. The data in Figure 9 is calculated as a percent control:

$$\text{Percent control} = \frac{\text{MFI @ X time (0, 2, 4 hr)}}{\text{MFI @ time 0}} * 100$$

It should be understood that various changes and modifications to the embodiments described herein will be apparent to those skilled in the art. Such changes and modifications can be made without departing from the spirit and scope of the present subject matter and without diminishing its intended advantages. It is therefore intended that such changes and modifications be covered by the appended claims.

The application discloses inter alia following items:

1. An antibody or antigen binding fragment thereof that specifically binds CD4, wherein the antibody comprises CDRL1, CDRL2, and CDRL3 with the sequences of SEQ ID NO:11, SEQ ID NO:12, and SEQ ID NO:13, respectively, wherein the antibody has an increased half life *in vivo* as compared to TNX-355.
2. An antibody or antigen binding fragment thereof that specifically binds CD4, wherein the antibody comprises CDRH1, CDRH2, and CDRH3 with the sequences of SEQ ID NO:14, SEQ ID NO:15, and SEQ ID NO:16, respectively, wherein the antibody has an increased half life *in vivo* as compared to TNX-355.

3. The antibody of item 1, wherein CDRH1, CDRH2, and CDRH3 of the antibody comprise the sequences of SEQ ID NO:14, SEQ ID NO:15, and SEQ ID NO:16, respectively.

4. An antibody or antigen binding fragment thereof that specifically binds CD4 comprising:

a. an amino acid sequence with at least 85% identity to SEQ ID NO:8;
b. an amino acid sequence with at least 85% identity to SEQ ID NO:9; or
c. an amino acid sequence with at least 85% identity to SEQ ID NO:10;

wherein the antibody has an increased half life *in vivo* as compared to TNX-355.

5. The antibody of item 1, wherein the antibody comprises the sequence of SEQ ID N0:8.

6. The antibody of item 1, wherein the antibody comprises a variant Fc region with increased affinity for neonatal Fc receptor ("FcRn") compared to TNX-355.

7. The antibody of item 1, 2, 3, 4, 5 or 6, wherein residue 228 is a residue other than Serine.

8. The antibody of item 7, wherein residue 228 is a Proline residue.

9. The antibody of item 1, 2, 3, 4, 5 or 6, wherein the antibody comprises a sequence with at least 85% identify to SEQ ID NO:8.

10. The antibody of item 1, 2,3,4,5, or 6, wherein residue 228 is a Proline, and wherein when assayed *in vitro* at least 80% of the total antibody is maintained as a bivalent antibody in serum for at least 200 hours.

11. The antibody of item 9, wherein the antibody comprises the sequence of SEQ ID NO:8.

12. The antibody of item 9, wherein the fragment crystallizable (Fc) region of said antibody comprises mutations in nucleic acid sequence relative to the sequence of a native human IgG1 and/or native human IgG4 antibody.

13. The antibody of item 1,2,3,4,5 or 6, wherein the light chain variable region of the antibody comprises a sequence with at least 85% identity to the sequence of SEQ ID NO:1.

14. The antibody of item 13, wherein the light chain variable region comprises the sequence of SEQ ID NO:1.

15. The antibody of item 1,2,3,4,5, or 6, wherein the light chain constant region of the antibody comprises a sequence with at least 85% identity to SEQ ID NO:2.

16. The antibody of item of item 15, wherein the light chain constant region comprises the sequence of SEQ ID NO:2.

17. The antibody of item 1,2,3,4, 5 or 6, wherein the variable light chain region of the antibody comprises the sequence of SEQ ID NO: 1 and wherein the light chain constant region of the antibody comprises the sequence of SEQ ID NO:2.

18. An antibody or antigen binding fragment thereof that specifically binds CD4, wherein the antibody heavy chain variable region comprises a sequence with at least 85% identity to SEQ ID NO:3, wherein the antibody has an increased half life *in vivo* as compared to TNX-355.

19. The antibody of item 18, wherein the antibody heavy chain variable region comprises SEQ ID NO:3.

20. The antibody of item 18, wherein residue 228 is a residue other than Serine.

21. The antibody of item 20, wherein residue 228 is a Proline residue.

22. The antibody of item 1, 2, 3, 4, 5,6, or 17, wherein the antibody is an IgG antibody.

23. The antibody of item 22, wherein the antibody is an IgG1, IgG2, IgG3 or IgG4.

24. The antibody of item 22, wherein the antibody is humanized.

25. The antibody of item 23, wherein the antibody is humanized.

26. A pharmaceutical composition comprising the antibody of any one of item 1, 2, 3, 4, 5, 6, or 17.

27. The pharmaceutical composition of item 26, wherein the composition is formulated for subcutaneous administration.

28. The pharmaceutical composition of item 27, wherein the composition comprises 1 to 10 mg/kg of said antibody.

29. The pharmaceutical composition of item 27, wherein the composition comprises 5 to 10 mg/kg of said antibody.

30. A polypeptide encoded by SEQ ID NO:17.

31. A polypeptide encoded by SEQ ID NO:18.

32. A nucleic acid encoding the antibody of item 1, 2, 3, 4, 5, 6 or 17.

33. A nucleic acid encoding SEQ ID NO:8, SEQ ID NO:9, or SEQ ID NO:10.

34. A nucleic acid comprising the sequence set forth in SEQ ID NO:17.

35. A nucleic acid comprising the sequence set forth in SEQ ID NO:18.

36. A vector comprising a nucleic acid of item 32.

37. A vector comprising a nucleic acid of any one of item ; 33 to 36.

38. A cell comprising the vector of item 36.

39. A cell comprising the vector of item 37.

40. A composition comprising the antibody of item 1,2,3,4, 5, 6, or 17.

41. The composition of item 40, wherein residue 228 of the antibody is a residue other than Serine.

42. The antibody of item 41, wherein residue 228 is a Proline residue.

43. The composition of item 40, wherein the antibody binds to domain 2 of CD4 and wherein when assayed *in vitro,* greater than 80% of the total antibody is maintained as a bivalent antibody for at least 200 hours.

44. The composition of item 40, wherein the antibody binds to domain 2 of CD4 and wherein the half-life of the antibody is at least 1.5 times longer than the unmodified antibody.

45. The composition of item 43 or 44, wherein the antibody comprises the amino acid sequence of SEQ ID NO:8.

46. The composition of item 40, wherein the composition is formulated for subcutaneous administration.

47. A method for preventing infection of target cells by human immunodeficiency virus type 1 ("HIV-1") comprising exposing said cells to a composition comprising the antibody of item 1, 2, 3,4, 5,6, or 17.

48. The method of item 47, wherein the residue 228 of the antibody is a residue other than Serine.

49. The method of item 48, wherein residue 228 of the antibody is a Proline.

50. The method of item 47, wherein the cells are in an individual suffering from human immunodeficiency syndrome (HIV).

51. The method of item 47, wherein the cells are in an individual at risk of acquiring HIV.

52. The method of item 50 or 51, wherein the composition is formulated for subcutaneous administration.

53. The method of item 52, wherein the cells are exposed to the composition following subcutaneous administration of said composition.

54. The method of item 53, wherein the composition is formulated to deliver a dose of 1 to 15 mg/kg of antibody.

55. The method of item 54 wherein the composition is administered either once a week or once every two weeks.

56. The method of item 55, wherein the composition is administered for at least nine cycles.

57. The method of item 47, wherein the composition is administered in combination with another anti-HIV therapeutic agent of a cocktail of anti-HIV therapeutic agent.

58. The method of item 57, wherein the anti-HIV therapeutic agent or cocktail of anti-HIV therapeutic agents comprise attachment inhibitors, fusion inhibitors, integrase inhibitors, reverse transcriptase inhibitors, and/or protease inhibitors.

## Claims

1. An antibody or antigen binding fragment thereof that specifically binds CD4, wherein the antibody comprises CDRL1, CDRL2, and CDRL3 with the sequences of SEQ ID NO:11, SEQ ID NO: 12, and SEQ ID NO: 13, respectively, and/or wherein the antibody comprises CDRH1, CDRH2, and CDRH3 with the sequences of SEQ ID NO: 14, SEQ ID NO: 15, and SEQ ID NO: 16, respectively, wherein the antibody has an increased half life *in vivo* as compared to TNX-355.

2. The antibody of claim 1, wherein the antibody comprises:

   a. an amino acid sequence with at least 85% identity to SEQ ID NO:8;
   b. an amino acid sequence with at least 85% identity to SEQ ID NO:9; or
   c. an amino acid sequence with at least 85% identity to SEQ ID NO:10.

3. The antibody of claim 1, wherein the antibody comprises a variant Fc region with increased affinity for neonatal Fc receptor ("FcRn") compared to TNX-355.

4. The antibody of claim 1, 2, or 3, wherein residue 228 (EU numbering system) is a Proline residue; and wherein when assayed *in vitro* at least 80% of the total antibody is maintained as a bivalent antibody in serum for at least 200 hours.

5. The antibody of claim 1, 2, or 3, wherein:

   a. the light chain variable region of the antibody comprises a sequence with at least 85% identity to the sequence of SEQ ID NO:1, preferably the sequence of SEQ ID NO:1;
   b. the light chain constant region of the antibody comprises a sequence with at least 85% identity to SEQ ID NO:2, preferably the sequence of SEQ ID NO:2;
   c. the variable light chain region of the antibody comprises the sequence of SEQ ID NO:1 and the light chain constant region of the antibody comprises the sequence of SEQ ID NO:2; or
   d. the antibody heavy chain variable region comprises a sequence with at least 85% identity to SEQ ID NO:3, preferably the sequence of SEQ ID NO:3.

6. The antibody of claim 1, 2 or 3, wherein residue 228 (EU numbering system) is a residue other than Serine, preferably, wherein residue 228 is a Proline residue.

7. The antibody of any one of claims 1 to 6, wherein the antibody is an IgG antibody, preferably IgG1, IgG2, IgG3 or IgG4; and/or wherein the antibody is humanized.

8. A pharmaceutical composition comprising the antibody of any one of claims 1 to 7.

9. The pharmaceutical composition of claim 8, wherein the composition formulated for subcutaneous administration comprises 1 to 10 mg/kg of said antibody, preferably 5 to 10 mg/kg of said antibody.

10. The pharmaceutical composition of claim 8 or 9, for use in preventing infection of target cells by human immunodeficiency virus type 1 ("HIV-1").

11. The pharmaceutical composition for use of claim 10, wherein the cells are in an individual suffering from Acquired Immune Deficiency Syndrome (AIDS) or in an individual at risk of acquiring an HIV infection.

12. The pharmaceutical composition for use of claim 10 or 11, wherein the pharmaceutical composition

a. is prepared to be administered either once a week or once every two weeks;
b. is prepared to be administered for at least nine cycles; and/or
c. is for administration in combination with another anti-HIV therapeutic agent or a cocktail of anti-HIV therapeutic agents, preferably wherein the anti-HIV therapeutic agent or cocktail of anti-HIV therapeutic agents comprise attachment inhibitors, fusion inhibitors, integrase inhibitors, reverse transcriptase inhibitors, and/or protease inhibitors.

**13.** A polypeptide encoded by SEQ ID NO:17 or 18.

**14.** A nucleic acid

a. encoding the antibody of any one of claims 1 to 7,
b. encoding SEQ ID NO:8, SEQ ID NO:9, or SEQ ID NO:10 or
c. comprising the sequence set forth in SEQ ID NO:17 or 18.

**15.** A vector comprising a nucleic acid of claim 14.

**16.** A cell comprising the vector of claim 15.

# FIGURE 1A

## TNX-355 Amino Acid Sequence

**Kappa Light Chain Variable Region (SEQ ID NO:1)**

```
DIVMTQSPDS LAVSLGERVT MNCKSSQSLL YSTNQKNYLA WYQQKPGQSP KLLIYWASTR
                         CDRL1                           CDRL2
ESGVPDRFSG SGSGTDFTLT ISSVQAEDVA VYYCQQYYSY RTFGGGTKLE IK
                                     CDRL3
```

**Light Chain Constant Region (SEQ ID NO:2)**

RTVAAPSVFI FPPSDEQLKS GTASVVCLLN NFYPREAKVQ WKVDNALQSG NSQESVTEQD

SKDSTYSLSS TLTLSKADYE KHKVYACEVT HQGLSSPVTK SFNRGEC

**Heavy Chain Variable Region (SEQ ID NO:3)**

```
QVQLQQSGPE VVKPGASVKM SCKASGYTFT SYVIHWVRQK PGQGLDWIGY INPYNDGTDY
                        CDRH1                         CDRH2
DEKFKGKATL TSDTSTSTAY MELSSLRSED TAVYYCAREK DNYATGAWFA YWGQGTLVTV
                                           CDRH3
SS
```

**IgG4 Constant Region (SEQ ID NO:4)**

ASTKGPSVFP LAPCSRSTSE STAALGCLVK DYFPEPVTVS WNSGALTSGV
HTFPAVLQSS GLYSLSSVVT VPSSSLGTKT YTCNVDHKPS NTKVDKRVES
KYGPPCPSCP APEFLGGPSV FLFPPKPKDT LMISRTPEVT CVVVDVSQED
PEVQFNWYVD GVEVHNAKTK PREEQFNSTY RVVSVLTVLH QDWLNGKEYK
CKVSNKGLPS SIEKTISKAK GQPREPQVYT LPPSQEEMTK NQVSLTCLVK
GFYPSDIAVE WESNGQPENN YKTTPPVLDS DGSFFLYSRL TVDKSRWQEG
NVFSCSVMHE ALHNHYTQKS LSLSLGK

# FIGURE 1 B

## G4H Amino Acid Sequence

### Kappa Light Chain Variable Region (SEQ ID NO:1)

```
DIVMTQSPDS LAVSLGERVT MNCKSSQSLL YSTNQKNYLA WYQQKPGQSP KLLIYWASTR
                               CDRL1                        CDRL2
ESGVPDRFSG SGSGTDFTLT ISSVQAEDVA VYYCQQYYSY RTFGGGTKLE IK
                                     CDRL3
```

### Light Chain Constant Region (SEQ ID NO:2)

RTVAAPSVFI FPPSDEQLKS GTASVVCLLN NFYPREAKVQ WKVDNALQSG NSQESVTEQD

SKDSTYSLSS TLTLSKADYE KHKVYACEVT HQGLSSPVTK SFNRGEC

### Heavy Chain Variable Region (SEQ ID NO:3)

```
QVQLQQSGPE VVKPGASVKM SCKASGYTFT SYVIHWVRQK PGQGLDWIGY INPYNDGTDY
                              CDRH1                        CDRH2
DEKFKGKATL TSDTSTSTAY MELSSLRSED TAVYYCAREK DNYATGAWFA YWGQGTLVTV
                                          CDRH3
SS
```

### G4H Heavy Chain Constant Region (SEQ ID NO:5)

ASTKGPSVFP LAPCSRSTSE STAALGCLVK DYFPEPVTVS WNSGALTSGV
HTFPAVLQSS GLYSLSSVVT VPSSSLGTKT YTCNVDHKPS NTKVDKRVES
KYGPPCPPCP APEFLGGPSV FLFPPKPKDT LMISRTPEVT CVVVDVSQED
PEVQFNWYVD GVEVHNAKTK PREEQFNSTY RVVSVLTVLH QDWLNGKEYK
CKVSNKGLPS SIEKTISKAK GQPREPQVYT LPPSQEEMTK NQVSLTCLVK
GFYPSDIAVE WESNGQPENN YKTTPPVLDS DGSFFLYSRL TVDKSRWQEG
NVFSCSVMHE ALHNHYTQKS LSLSLGK

# FIGURE 1 C

## G4D Amino Acid Sequence

**Kappa Light Chain Variable Region (SEQ ID NO:1)**

```
DIVMTQSPDS LAVSLGERVT MNCKSSQSLL YSTNQKNYLA WYQQKPGQSP KLLIYWASTR
                               CDRL1                        CDRL2
ESGVPDRFSG SGSGTDFTLT ISSVQAEDVA VYYCQQYYSY RTFGGGTKLE IK
                                    CDRL3
```

**Light Chain Constant Region (SEQ ID NO:2)**

RTVAAPSVFI FPPSDEQLKS GTASVVCLLN NFYPREAKVQ WKVDNALQSG NSQESVTEQD

SKDSTYSLSS TLTLSKADYE KHKVYACEVT HQGLSSPVTK SFNRGEC

**Heavy Chain Variable Region (SEQ ID NO:3)**

```
QVQLQQSGPE VVKPGASVKM SCKASGYTFT SYVIHWVRQK PGQGLDWIGY INPYNDGTDY
                              CDRH1                        CDRH2
DEKFKGKATL TSDTSTSTAY MELSSLRSED TAVYYCAREK DNYATGAWFA YWGQGTLVTV
                                                   CDRH3
SS
```

**G4D Heavy Chain Constant Region (SEQ ID NO:6)**

ASTKGPSVFP LAPCSRSTSE STAALGCLVK DYFPEPVTVS WNSGALTSGV
HTFPAVLQSS GLYSLSSVVT VPSSSLGTKT YTCNVDHKPS NTKVDKRV ES
KYGPPCPPCP APEFEGGPSV FLFPPKPKDT LMISRTPEVT CVVVDVSQED
PEVQFNWYVD GVEVHNAKTK PREEQFNSTY RVVSVLTVLH QDWLNGKEYK
CKVSNKGLPS SIEKTISKAK GQPREPQVYT LPPSQEEMTK NQVSLTCLVK
GFYPSDIAVE WESNGQPENN YKTTPPVLDS DGSFFLYSRL TVDKSRWQEG
NVFSCSVMHE ALHNHYTQKS LSLSLGK

# FIGURE 1 D

## MV1 Amino Acid Sequence

**Kappa Light Chain Variable Region (SEQ ID NO:1)**

DIVMTQSPDS LAVSLGERVT MNCKSSQSLL YSTNQKNYLA WYQQKPGQSP KLLIYWASTR
CDRL1 CDRL2
ESGVPDRFSG SGSGTDFTLT ISSVQAEDVA VYYCQQYYSY RTFGGGTKLE IK
CDRL3

**Light Chain Constant Region (SEQ ID NO:2)**

RTVAAPSVFI FPPSDEQLKS GTASVVCLLN NFYPREAKVQ WKVDNALQSG NSQESVTEQD

SKDSTYSLSS TLTLSKADYE KHKVYACEVT HQGLSSPVTK SFNRGEC

**Heavy Chain Variable Region (SEQ ID NO:3)**

QVQLQQSGPE VVKPGASVKM SCKASGYTFT SYVIHWVRQK PGQGLDWIGY INPYNDGTDY
CDRH1 CDRH2
DEKFKGKATL TSDTSTSTAY MELSSLRSED TAVYYCAREK DNYATGAWFA YWGQGTLVTV
CDRH3
SS

**MV1 Heavy Chain Constant Region (SEQ ID NO:7)**

ASTKGPSVFP LAPSSKSTSG GTAALGCLVK DYFPEPVTVS WNSGALTSGV
HTFPAVLQSS GLYSLSSVVT VPSSSLGTQT YICNVNHKPS NTKVDKKVEP
KSCDKTHTCP PCPAPE**FE**GG PSVFLFPPKP KDTLMISRTP EVTCVVVDVS
HEDPEVKFNW YVDGVEVHNA KTKPREEQYN STYRVVSVLT VLHQDWLNGK
EYKCKVSNKA LPA**S**IEKTIS KAKGQPREPQ VYTLPPSRDE LTKNQVSLTC
LVKGFYPSDI AVEWESNGQP ENNYKTTPPV LDSDGSFFLY SKLTVDKSRW
QQGNVFSCSV MHEALHNHYT QKSLSLSPGK

# FIGURE 2

## Optimized Nucleic Acid Sequence

### Light Chain Variable Region (SEQ ID NO:17):

ATGGAATGGAGCGGGGTCTTTATCTTTCTCCTGTCAGTAACTGCAGGTGTCCACTCC
GACATCGTGATGACCCAGTCCCCTGACTCCCTGGCTGTGTCCCTGGGCGAACGGGTC
ACCATGAACTGCAAATCTTCCCAGTCCCTGCTGTACTCCACCAACCAGAAGAACTAC
CTGGCTTGGTACCAGCAGAAACCTGGCCAGTCCCCCAAACTGCTCATCTACTGGGCT
TCCACCAGGGAAAGCGGCGTGCCTGACAGATTCTCCGGAAGCGGCAGCGGAACCGA
CTTCACCCTGACCATCTCCAGCGTGCAGGCTGAAGACGTGGCTGTCTACTACTGCCA
GCAGTACTACAGCTACAGGACCTTCGGCGGAGGCACCAAGCTGGAGATCAAG

### Heavy Chain Variable Region (SEQ ID NO:18):

ATGGAATGGAGCGGGGTCTTTATCTTTCTCCTGTCAGTAACTGCAGGTGTCCACTCCC
AGGTGCAACTGCAACAGTCCGGACCCGAAGTCGTGAAACCAGGAGCTTCCGTGAAGA
TGAGCTGCAAAGCATCCGGATACACCTTCACCAGCTACGTGATCCACTGGGTGAGGCA
GAAACCTGGCCAGGGCCTGGACTGGATCGGCTACATCAACCCTTACAACGACGGAAC
CGACTACGACGAGAAATTCAAAGGCAAAGCTACCCTGACCAGCGACACCAGCACCTC
CACTGCTTACATGGAGCTGTCCAGCCTGAGGTCCGAAGACACCGCTGTGTACTACTGC
GCTAGGGAGAAGGACAACTACGCTACCGGCGCTTGGTTCGCCTACTGGGGCCAGGGA
ACCCTGGTGACCGTGTCCAGC

# FIGURE 3A

```
         10        20        30        40        50
1   ATGGAATGGAGCGGGGTCTTTATCTTTCTCCTGTCAGTAACTGCAGGTGT  Codon OP NA VH.seq
1   ATGGACTGGACCTGGAGGGTCTTCTGCTTGCTGGCTGTAGCACCAGGTGC  VH5A8.seq

         60        70        80        90       100
51  CCACTCCCAGGTGCAACTGCAACAGTCCGGACCCGAAGTCGTGAAACCAG  Codon OP NA VH.seq
51  CCACTCCCAGGTCCAACTGCAGCAGTCCGGTCCGGAAGTTGTTAAACCGG  VH5A8.seq

        110       120       130       140       150
101 GAGCTTCCGTGAAGATGAGCTGCAAAGCATCCGGATACACCTTCACCAGC  Codon OP NA VH.seq
101 GTGCTTCCGTTAAAATGTCCTGCAAAGCATCCGGTTACACCTTCACCTCC  VH5A8.seq

        160       170       180       190       200
151 TACGTGATCCACTGGGTGAGGCAGAAACCTGGCCAGGGCCTGGACTGGAT  Codon OP NA VH.seq
151 TACGTTATCCACTGGGTTCGTCAGAAACCGGGTCAGGGTCTGGACTGGAT  VH5A8.seq

        210       220       230       240       250
201 CGGCTACATCAACCCTTACAACGACGGAACCGACTACGACGAGAAATTCA  Codon OP NA VH.seq
201 CGGTTACATCAACCCGTACAACGACGGTACCGACTACGACGAAAAATTCA  VH5A8.seq

        260       270       280       290       300
251 AAGGCAAAGCTACCCTGACCAGCGACACCAGCACCTCCACTGCTTACATG  Codon OP NA VH.seq
251 AAGGTAAAGCTACCCTGACCTCCGACACCTCCACCTCCACCGCTTACATG  VH5A8.seq

        310       320       330       340       350
301 GAGCTGTCCAGCCTGAGGTCCGAAGACACCGCTGTGTACTACTGCGCTAG  Codon OP NA VH.seq
301 GAACTGTCCTCCCTGCGTTCCGAAGACACCGCTGTTTACTACTGCGCTCG  VH5A8.seq

        360       370       380       390       400
351 GGAGAAGGACAACTACGCTACCGGCGCTTGGTTCGCCTACTGGGGCCAGG  Codon OP NA VH.seq
351 TGAAAAAGACAACTACGCTACCGGTGCTTGGTTCGCTTACTGGGGTCAGG  VH5A8.seq

        410       420
401 GAACCCTGGTGACCGTGTCCAGC                             Codon OP NA VH.seq
401 GTACCCTGGTTACCGTCTCC                                VH5A8.seq
```

# FIGURE 3B

```
                10         20         30         40         50
1     ATGGAATGGAGCGGGGTCTTTATCTTTCTCCTGTCAGTAACTGCAGGTGT   Codon OP NA VK.seq
1     ATGGACATGAGGGTCCCCGCTCAGCTCCTGGGGCTCCTGCTGCTCTGGCT   VK 5A8.seq

                60         70         80         90        100
51    CCACTCCGACATCGTGATGACCCAGTCCCCTGACTCCCTGGCTGTGTCCC   Codon OP NA VK.seq
51    GCCAGGTGCCAGAGGTGATATCGTTATGACCCAGTCCCCGGACTCCCTGG   VK 5A8.seq

               110        120        130        140        150
101   TGGGCGAACGGGTCACCATGAACTGCAAATCTTCCCAGTCCCTGCTGTAC   Codon OP NA VK.seq
101   CTGTTTCCCTGGGTGAACGTGTTACCATGAACTGCAAATCCTCCCAGTCC   VK 5A8.seq

               160        170        180        190        200
151   TCCACCAACCAGAAGAACTACCTGGCTTGGTACCAGCAGAAACCTGGCCA   Codon OP NA VK.seq
151   CTGCTGTACTCCACCAACCAGAAAAACTACCTGGCTTGGTACCAGCAGAA   VK 5A8.seq

               210        220        230        240        250
201   GTCCCCCAAACTGCTCATCTACTGGGCTTCCACCAGGGAAAGCGGCGTGC   Codon OP NA VK.seq
201   ACCGGGTCAGTCCCCGAAACTGCTGATCTACTGGGCTTCCACCCGTGAAT   VK 5A8.seq

               260        270        280        290        300
251   CTGACAGATTCTCCGGAAGCGGCAGCGGAACCGACTTCACCCTGACCATC   Codon OP NA VK.seq
251   CCGGTGTTCCGGACCGTTTCTCCGGTTCCGGTTCCGGTACCGACTTCACC   VK 5A8.seq

               310        320        330        340        350
301   TCCAGCGTGCAGGCTGAAGACGTGGCTGTCTACTACTGCCAGCAGTACTA   Codon OP NA VK.seq
301   CTGACCATCTCCTCCGTTCAGGCTGAAGACGTTGCTGTTTACTACTGCCA   VK 5A8.seq

               360        370        380        390        400
351   CAGCTACAGGACCTTCGGCGGAGGCACCAAGCTGGAGATCAAG          Codon OP NA VK.seq
351   GCAGTACTACTCCTACCGTACCTTCGGTGGTGGTACCAAACTGGAGATCA   VK 5A8.seq

393                                                        Codon OP NA VK.seq
401   AA                                                   VK 5A8.seq
```

# FIGURE 4

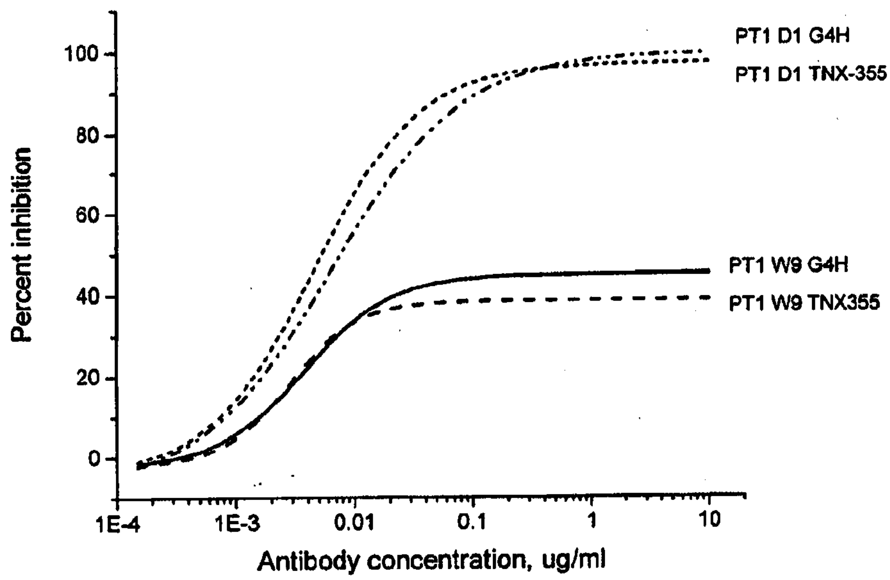
PT1 D1 G4H
PT1 D1 TNX-355
PT1 W9 G4H
PT1 W9 TNX355
Percent inhibition
100
80
60
40
20
0
1E-4
1E-3
0.01
0.1
1
10
Antibody concentration, ug/ml

# FIGURE 5

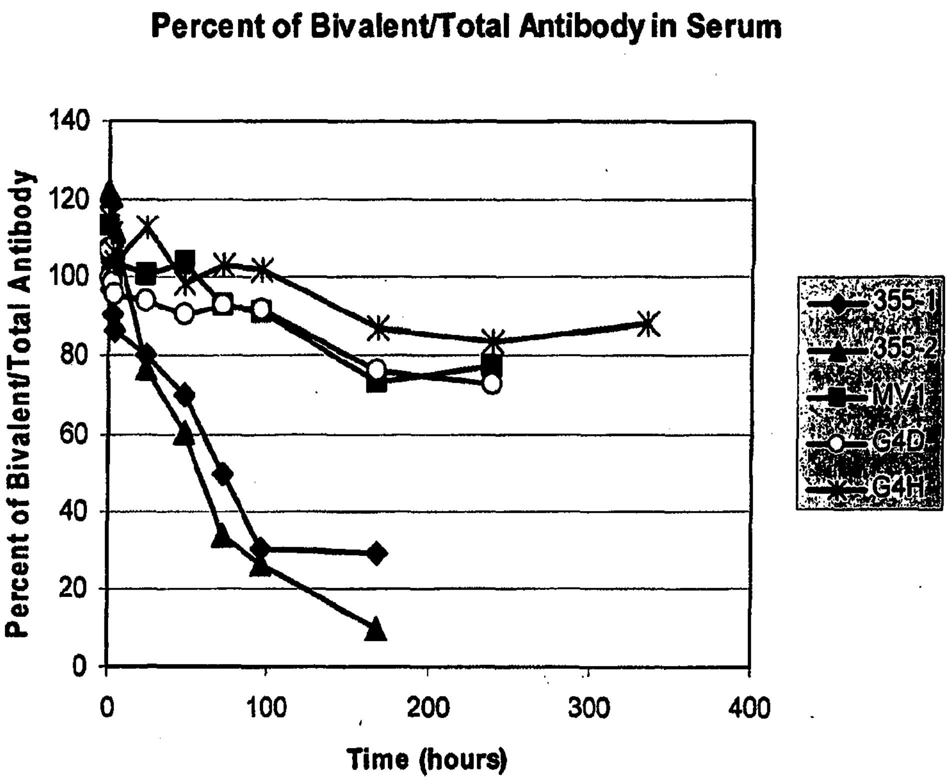
Percent of Bivalent/Total Antibody in Serum
Percent of Bivalent/Total Antibody
140
120
100
80
60
40
20
0
0
100
200
300
400
Time (hours)
355-1
355-2
MV1
G4D
G4H

# FIGURE 6

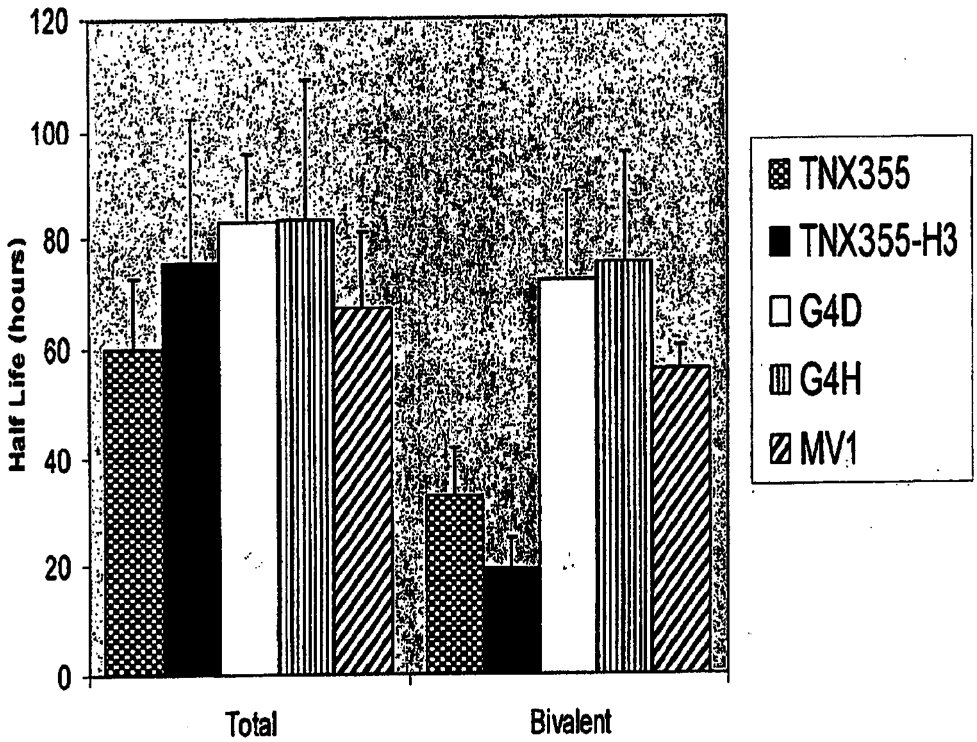
120
100
80
60
40
20
0
Half Life (hours)
Total
Bivalent
TNX355
TNX355-H3
G4D
G4H
MV1

# FIGURE 7A

**TNX-355 HOCL transfectomas final titer (ug/ml) by HPLC in 2%FBS**
**(Original sequence VS Codon optimized sequence)**

| *Original sequence* | | *Codon optimized sequence* | |
|---|---|---|---|
| Transfectoma # | Titer (ug/ml) | Transfectoma # | Titer (ug/ml) |
| #79 | 619 | #445 | 687 |
| #103 | 602 | #118 | 681 |
| #66 | 536 | #614 | 672 |
| #2 | 527 | #63 | 670 |
| #61 | 459 | #205 | 643 |
| #48 | 436 | #542 | 635 |
| #1 | 428 | #647 | 634 |
| #41 | 419 | #245 | 631 |
| #86 | 384 | #391 | 628 |
| #51 | 374 | #143 | 622 |

# FIGURE 7B

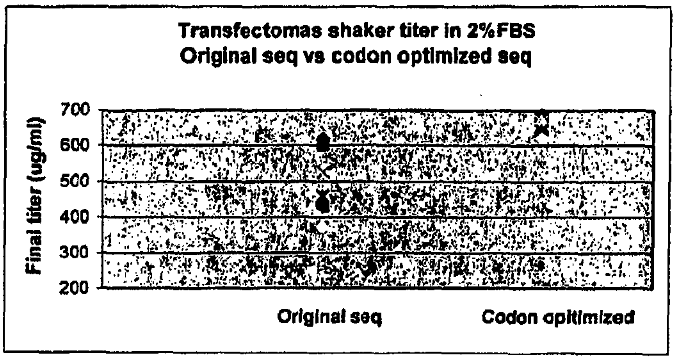

# FIGURE 8

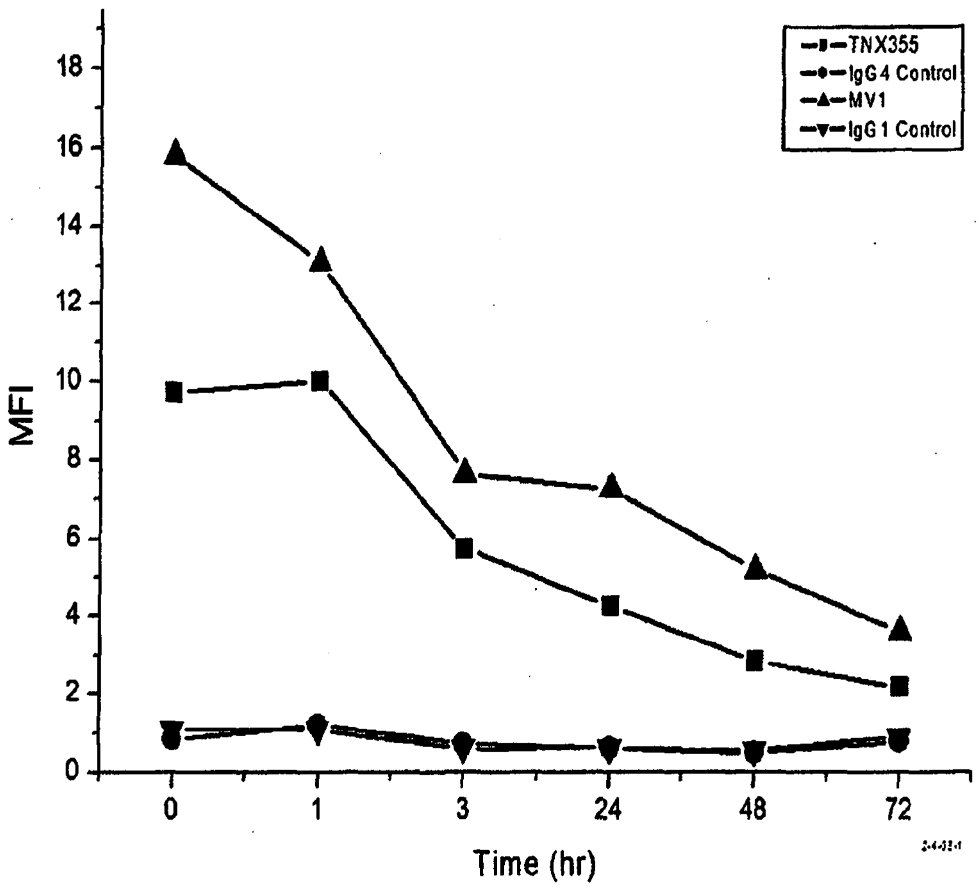
TNX355
IgG4 Control
MV1
IgG1 Control
18
16
14
12
10
8
6
4
2
0
MFI
0
1
3
24
48
72
Time (hr)

## FIGURE 9A

FACS Analysis of CD4 Levels In the Presence of G4H and G4D As Compared To TNX-355

CD4 level on Lymphocytes In PBMCs

| | Donor I | | Donor II | |
|---|---|---|---|---|
| | t=0* | 2 hr* | t=0* | 2 hr* |
| No ab | 100 | 99 | 100 | 99 |
| Isotype Ctrl | 100 | 89 | 100 | 103 |
| TNX355 | 100 | 57 | 100 | 68 |
| G4H | 100 | 61 | 100 | 68 |
| G4D | 100 | 107 | 100 | 82 |
| * Data expressed as percent control | | | | |

## FIGURE 9B

FACS Analysis of CD4 Levels In the Presence of MV1 As Compared To TNX-355

CD4 level on Lymphocytes In PBMCs

| | Donor I | | Donor II | |
|---|---|---|---|---|
| | t=0* | 2 hr* | t=0* | 2 hr* |
| No ab | 100 | 99 | 100 | 99 |
| Isotype Ctrl | 100 | 89 | 100 | 103 |
| TNX355 | 100 | 57 | 100 | 68 |
| Isotype Ctrl | 100 | 100 | 100 | 102 |
| MV1 | 100 | 84 | 100 | 91 |
| * Data expressed as percent control | | | | |

European Patent Office

# EUROPEAN SEARCH REPORT

Application Number
EP 16 16 4079

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZHANG XING-QUAN ET AL: "Synergistic in vitro antiretroviral activity of a humanized monoclonal anti-CD4 antibody (TNX-355) and enfuvirtide (T-20)", ANTIMICROBIAL AGENTS AND CHEMOTHERAPY, vol. 50, no. 6, June 2006 (2006-06), pages 2231-2233, XP002493875, ISSN: 0066-4804 * The whole document, in particular TNX-355 * ----- | 1-16 | INV. C07K16/00 C07K16/28 A61K39/00 |
| X | VERMEIRE ET AL: "Anti-HIV Agents Targeting the Interaction of gp120 with the Cellular CD4 Receptor", EXPERT OPINION ON INVESTIGATIONAL DRUGS, ASHLEY PUBLICATIONS LTD., LONDON, GB, vol. 14, no. 10, 1 January 2005 (2005-01-01), pages 1199-1212, XP002993525, ISSN: 1354-3784 * the whole document * ----- | 1-16 | TECHNICAL FIELDS SEARCHED (IPC) |
| X | VERMEIRE KURT ET AL: "Inhibitors of HIV infection via the cellular CD4 receptor.", CURRENT MEDICINAL CHEMISTRY 2006, vol. 13, no. 7, 2006, pages 731-743, XP009104962, ISSN: 0929-8673 * the whole document * ----- | 1-16 | C07K |
| Y | WO 97/09351 A (IDEC PHARMA CORP [US]) 13 March 1997 (1997-03-13) * the whole document * ----- | 1-16 | |
| Y | US 2006/099206 A1 (SINACORE MARTIN S [US] ET AL) 11 May 2006 (2006-05-11) * the whole document * ----- -/-- | 1-16 | |

2

The present search report has been drawn up for all claims

| Place of search | Date of completion of the search | Examiner |
|---|---|---|
| Munich | 15 July 2016 | Chapman, Rob |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 16 16 4079

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,P | EP 1 810 979 A (KIRIN BREWERY [JP]) 25 July 2007 (2007-07-25) * the whole document * & WO 2006/033386 A (KIRIN BREWERY [JP]; TAKAHASHI NOBUAKI [JP]; YOSHIDA HIDEAKI [JP]) 30 March 2006 (2006-03-30) ----- | 1-16 | |
| A | US 2005/276799 A1 (HINTON PAUL R [US] ET AL HINTON PAUL [US] ET AL) 15 December 2005 (2005-12-15) * the whole document * ----- | 6 | |
| X,P | WO 2007/094983 A (TANOX INC [US]; DUENSING THOMAS [US]; FUNG SEK CHUNG MICHAEL [US]; STA) 23 August 2007 (2007-08-23) * the whole document * ----- | 1-16 | |
| A,P | WO 2007/059782 A (GENMAB AS [DK]; PARREN PAUL [NL]; SCHUURMAN JANINE [NL]; VINK TOM [NL]) 31 May 2007 (2007-05-31) * the whole document * ----- | 1-16 | TECHNICAL FIELDS SEARCHED (IPC) |
| Y | NEWMAN R ET AL: "MODIFICATION OF THE FC REGION OF A PRIMATIZED IGG ANTIBODY TO HUMAN CD4 RETAINS ITS ABILITY TO MODULATE CD4 RECEPTORS BUT DOES NOT DEPLETE CD4+ T CELLS IN CHIMPANZEES", CLINICAL IMMUNOLOGY, ACADEMIC PRESS, US, vol. 98, no. 2, 1 February 2001 (2001-02-01), pages 164-174, XP001098835, ISSN: 1521-6616, DOI: 10.1006/CLIM.2000.4975 * the whole document * ----- -/-- | 1-16 | |

The present search report has been drawn up for all claims

2

| Place of search | Date of completion of the search | Examiner |
|---|---|---|
| Munich | 15 July 2016 | Chapman, Rob |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 16 16 4079

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | HEZAREH M ET AL: "Effector function activities of a panel of mutants of a broadly neutralizing antibody against human immunodeficiency virus type 1", JOURNAL OF VIROLOGY, THE AMERICAN SOCIETY FOR MICROBIOLOGY, US, vol. 75, no. 24, 1 December 2001 (2001-12-01), pages 12161-12168, XP002339184, ISSN: 0022-538X, DOI: 10.1128/JVI.75.24.12161-12168.2001 * the whole document * ----- | 1-16 | |
| Y | XU DANLIN ET AL: "In vitro characterization of five humanized OKT3 effector function variant antibodies", CELLULAR IMMUNOLOGY, ACADEMIC PRESS, SAN DIEGO, CA, US, vol. 200, no. 1, 25 February 2000 (2000-02-25), pages 16-26, XP002376698, ISSN: 0008-8749, DOI: 10.1006/CIMM.2000.1617 * the whole document * ----- | 1-16 | |

TECHNICAL FIELDS SEARCHED (IPC)

The present search report has been drawn up for all claims

2

| Place of search | Date of completion of the search | Examiner |
|---|---|---|
| Munich | 15 July 2016 | Chapman, Rob |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 16 4079

This annex lists the patent family members relating to the patent documents cited in the above-mentioned European search report. The members are as contained in the European Patent Office EDP file on
The European Patent Office is in no way liable for these particulars which are merely given for the purpose of information.

15-07-2016

| Patent document cited in search report | Publication date | Patent family member(s) | Publication date |
|---|---|---|---|
| WO 9709351 A | 13-03-1997 | AP 1193 A | 01-08-2003 |
| | | AT 348113 T | 15-01-2007 |
| | | AU 717674 B2 | 30-03-2000 |
| | | AU 6916296 A | 27-03-1997 |
| | | BG 64651 B1 | 31-10-2005 |
| | | BG 102343 A | 30-04-1999 |
| | | BR 9610404 A | 06-07-1999 |
| | | CA 2231182 A1 | 13-03-1997 |
| | | CN 1200737 A | 02-12-1998 |
| | | CZ 9800586 A3 | 12-08-1998 |
| | | DE 69636760 T2 | 18-10-2007 |
| | | EP 0854885 A1 | 29-07-1998 |
| | | HK 1016995 A1 | 22-05-2009 |
| | | HU 9802212 A2 | 28-01-1999 |
| | | JP 3619866 B2 | 16-02-2005 |
| | | JP H11514216 A | 07-12-1999 |
| | | NO 980915 A | 06-05-1998 |
| | | NZ 316835 A | 29-09-1999 |
| | | OA 10671 A | 25-11-2002 |
| | | RO 120198 B1 | 28-10-2005 |
| | | RU 2232773 C2 | 20-07-2004 |
| | | SK 27998 A3 | 04-11-1998 |
| | | US 6136310 A | 24-10-2000 |
| | | US 7452534 B1 | 18-11-2008 |
| | | US 2003077275 A1 | 24-04-2003 |
| | | US 2009221036 A1 | 03-09-2009 |
| | | US 2009226430 A1 | 10-09-2009 |
| | | WO 9709351 A1 | 13-03-1997 |
| US 2006099206 A1 | 11-05-2006 | AU 2005294131 A1 | 20-04-2006 |
| | | AU 2005294373 A1 | 20-04-2006 |
| | | BR PI0516264 A | 26-08-2008 |
| | | BR PI0516572 A | 16-09-2008 |
| | | CA 2582157 A1 | 20-04-2006 |
| | | CA 2582194 A1 | 20-04-2006 |
| | | CN 101027391 A | 29-08-2007 |
| | | CN 101287471 A | 15-10-2008 |
| | | EA 200700751 A1 | 30-06-2008 |
| | | EP 1797182 A2 | 20-06-2007 |
| | | EP 1828251 A2 | 05-09-2007 |
| | | JP 4980222 B2 | 18-07-2012 |
| | | JP 2008515430 A | 15-05-2008 |
| | | JP 2008515438 A | 15-05-2008 |
| | | KR 20070073885 A | 10-07-2007 |
| | | US 2006099206 A1 | 11-05-2006 |
| | | US 2009285806 A1 | 19-11-2009 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 16 16 4079

This annex lists the patent family members relating to the patent documents cited in the above-mentioned European search report.
The members are as contained in the European Patent Office EDP file on
The European Patent Office is in no way liable for these particulars which are merely given for the purpose of information.

15-07-2016

| Patent document cited in search report | Publication date | Patent family member(s) | Publication date |
|---|---|---|---|
| | | US 2011038863 A1 | 17-02-2011 |
| | | WO 2006041934 A2 | 20-04-2006 |
| | | WO 2006042158 A2 | 20-04-2006 |
| | | ZA 200703561 A | 30-09-2009 |
| EP 1810979 A | 25-07-2007 | CA 2580981 A1 | 30-03-2006 |
| | | EP 1810979 A1 | 25-07-2007 |
| | | ES 2387312 T3 | 20-09-2012 |
| | | JP 4958555 B2 | 20-06-2012 |
| | | US 2008063635 A1 | 13-03-2008 |
| | | US 2015141621 A1 | 21-05-2015 |
| | | WO 2006033386 A1 | 30-03-2006 |
| US 2005276799 A1 | 15-12-2005 | NONE | |
| WO 2007094983 A | 23-08-2007 | US 2009053220 A1 | 26-02-2009 |
| | | WO 2007094983 A2 | 23-08-2007 |
| WO 2007059782 A | 31-05-2007 | AU 2006317242 A1 | 31-05-2007 |
| | | BR PI0619056 A2 | 20-09-2011 |
| | | CA 2631184 A1 | 31-05-2007 |
| | | EA 200801460 A1 | 27-02-2009 |
| | | EP 1973576 A1 | 01-10-2008 |
| | | JP 5525729 B2 | 18-06-2014 |
| | | JP 2009517006 A | 30-04-2009 |
| | | JP 2014128266 A | 10-07-2014 |
| | | KR 20080090406 A | 08-10-2008 |
| | | KR 20150041197 A | 15-04-2015 |
| | | SG 10201600950T A | 30-03-2016 |
| | | US 2009226421 A1 | 10-09-2009 |
| | | WO 2007059782 A1 | 31-05-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 92651607 P
- US 6309880 B, Chang
- US 5817767 A, Allaway
- US 5922325 A, Tilley
- US 6241986 B, Zolla-Pazner
- US 6136310 A, Hanna
- US 5871732 A, Burkly
- WO 0042072 A
- WO 9425591 A
- US 20030130496 A
- US 4946778 A
- US 6194551 B1
- WO 9951642 A
- US 5698435 A
- US 5698417 A
- US 6204023 B
- WO 8605807 A
- WO 8901036 A
- US 5122464 A
- US 4767704 A
- US 4657866 A
- US 4560655 A
- US 5122469 A
- US 5712163 A
- US 6048728 A
- WO 9321232 A
- EP 439095 A
- US 5474981 A
- US 8802940 W
- US 3773919 A
- US 6514496 B
- WO 9208495 A
- WO 9114438 A
- WO 8912624 A
- US 5314995 A
- EP 396387 A
- US 4676980 A


### Non-patent literature cited in the description


- **L. RATNER.** *Nature,* 1985, vol. 313, 277-84
- **KABAT et al.** Sequences of Proteins of Immunological Interest. National Institutes of Health, 1991
- **KABAT et al.** Sequences of Proteins of Immunological Interest. National Institute of Health, 1987
- **KOHLER et al.** *Nature,* 1975, vol. 256, 495
- **GREENSPAN et al.** *FASEB J,* 1989, vol. 7, 437
- **NISSINOFF.** *J Immunol,* 1991, vol. 147, 2429
- **CARON et al.** *J. Exp Med.,* 1992, vol. 176, 1191-1195
- **SHOPES, B.** *J. Immunol.,* 1992, vol. 148, 2918-2922
- **STEVENSON.** *Anti-Cancer Drug Design,* 1989, vol. 3, 219-230
- **CARTER et al.** *Nucleic Acids Res.,* 1985, vol. 13, 4431-4443
- **KUNKEL et al.** *Proc. Natl. Acad. Sci. USA,* 1987, vol. 82, 488
- **HIGUCHI.** PCR Protocols. Academic Press, 1990, 177-183
- **VALLETTE et al.** *Nuc. Acids Res.,* 1989, vol. 17, 723-733
- **WELLS et al.** *Gene,* 1985, vol. 34, 315-323
- **IDUSOGIE et al.** *J. Immunol.,* 2000, vol. 164, 4178-4184
- **STUDIER.** *J Mol Biol,* 1991, vol. 219, 37
- **SCHOEPFER.** *Gene,* 1993, vol. 124, 83
- **ROSENBERG et al.** *Gene,* 1987, vol. 56, 125
- **CHANG et al.** *Nature,* 1978, vol. 275, 615
- **GOEDDEL et al.** *Nature,* 1979, vol. 281, 544
- **GOEDDEL et al.** *Nucl Acids Res,* 1980, vol. 8, 4057
- **SAMBROOK et al.** Molecular Cloning, A Laboratory Manual. Cold Spring Harbor Laboratory, 1990
- **HITZEMAN et al.** *J Biol Chem,* 1980, vol. 255, 2073
- **HOLLAND et al.** *Biochem,* 1978, vol. 17, 4900
- **FLEER et al.** *Gene,* 1991, vol. 107, 285
- **HINNEN et al.** *Proc Natl Acad Sci,* 1978, vol. 75, 1929
- **LUCKOW et al.** *Bio/Technology,* 1988, vol. 6, 47
- **MILLER et al.** Genetics Engineering. Plenam Publishing, 1986, vol. 8, 277-9
- **MSEDA et al.** *Nature,* 1985, vol. 315, 592
- Tissue Culture. Academic Press, 1973
- **URLAUB et al.** *Proc Natl Acad Sci USA,* 1980, vol. 77, 4216

- **HAM et al.** *Meth Enzymol,* 1979, vol. 58, 44
- **BARNES et al.** *Anal Biochem,* 1980, vol. 102, 255
- **KUTMEIER et al.** *Bio/Techniques,* 1994, vol. 17, 242
- Current Protocols in Molecular Biology. John Wiley & Sons, 1998
- **CHOTHIA et al.** *J Mol Biol,* 1998, vol. 278, 457
- **BIRD.** *Science,* 1988, vol. 242, 423
- **HUSTON et al.** *Proc Natl Acad Sci USA,* 1988, vol. 85, 5879
- **WARD et al.** *Nature,* 1989, vol. 334, 544
- **SKERRA et al.** *Science,* 1988, vol. 242, 1038
- **FOECKING et al.** *Gene,* 1986, vol. 45, 101
- **COCKETT et al.** *Bio/Technology,* 1990, vol. 8, 2
- **WIGLER et al.** *Cell,* 1977, vol. 11, 223
- **SZYBALSKA et al.** *Proc Natl Acad Sci USA,* 1992, vol. 48, 202
- **LOWY et al.** *Cell,* 1980, vol. 22, 817
- **WIGLER et al.** *Proc Natl Acad Sci USA,* 1980, vol. 77, 357
- **O'HARE et al.** *Proc Natl Acad Sci USA,* 1981, vol. 78, 1527
- **MULLIGAN et al.** *Proc Natl Acad Sci USA,* 1981, vol. 78, 2072
- **WU et al.** *Biotherapy,* 1991, vol. 3, 87
- **SANTERRE et al.** *Gene,* 1984, vol. 30, 147
- Current Protocols in Molecular Biology. John Wiley & Sons, 1993
- **KRIEGLER.** Gene Transfer and Expression, A Laboratory Manual. Stockton Press, 1990
- Current Protocols. Human Genetics. John Wiley & Sons, 1994
- **COLBERRE-GARAPIN et al.** *J Mol Biol,* 1981, vol. 150, 1
- The use of vectors based on gene amplification for the expression of cloned genes in mammalian cells. **BEBBINGTON et al.** DNA Cloning. Academic Press, 1987, vol. 3
- **CROUSE et al.** *Mol Cell Biol,* 1983, vol. 3, 257
- **PROUDFOOT.** *Nature,* 1986, vol. 322, 52
- **KOHLER.** *Proc Natl Acad Sci USA,* 1980, vol. 77, 2197
- **NARAMURA et al.** *Immunol Lett,* 1994, vol. 39, 91
- **GILLIES et al.** *Proc Natl Acad Sci USA,* 1992, vol. 89, 1428
- **FELL et al.** *J Immunol,* 1991, vol. 146, 2446
- **GENTZ et al.** *Proc Natl Acad Sci USA,* 1989, vol. 86, 821
- **WILSON et al.** *Cell,* 1984, vol. 37, 767
- **CARTER et al.** *Bio/Technology,* 1992, vol. 10, 163
- **LINDMARK et al.** *J Immunol Meth,* 1983, vol. 62, 1
- **GUSS et al.** *EMBO J,* 1986, vol. 5, 1567
- **LITTMAN et al.** *Cell,* 1988, vol. 55, 541
- **R. A. FISHER et al.** *Nature,* 1988, vol. 331, 76-78
- **H. M. SHAPIRO.** Practical Flow Cytometry. Alan R. Liss, Inc, 1985
- **LASKY et al.** *Science,* 1986, vol. 233, 209-12
- **B.D. WALKER et al.** Inhibition of Human Immunodeficiency Virus Syncytium Formation and Virus Replication by Castanospermine. *PNAS,* 1984, vol. 84, 8120-8124
- **E. G. ENGLEMAN et al.** *J. Immunol.,* 1981, vol. 127, 2124-29
- **MADDON et al.** *Cell*
- **LITTMAN et al.** *Cell*
- **CHAO et al.** *J. Biol. Chem.,* 1989, vol. 264, 5812-17
- **GARLICK et al.** *AIDS Res. Hum. Retroviruses,* 1990, vol. 6, 465-79
- Remington's Pharmaceutical Sciences. 1980
- **WU et al.** *J Biol Chem,* 1987, vol. 262, 4429
- **LANGER.** *Science,* 1990, vol. 249, 1527
- **TREAT et al.** Liposomes in the Therapy of Infectious Disease and Cancer. 1989, 353-365
- LIPOSOMES IN THE THERAPY OF INFECTIOUS DISEASE AND CANCER. 317-27
- **SEFTON.** *CRC Crit Ref Biomed Eng,* 1987, vol. 14, 201
- **BUCHWALD et al.** *Surgery,* 1980, vol. 88, 507
- **SAUDEK et al.** *N Engl J Med,* 1989, vol. 321, 574
- Medical Applications of Controlled Release. CRC Press, 1974
- Controlled Drug Bioavailability. Drug Product Design and Performance. Wiley, 1984
- **RANGER et al.** *J Macromol Sci Rev Macromol Chem,* 1983, vol. 23, 61
- **LEVY et al.** *Science,* 1985, vol. 228, 190
- **DURING et al.** *Ann Neurol,* 1989, vol. 25, 351
- **HOWARD et al.** *J Neurosurg,* 1989, vol. 71, 105
- **AMON et al.** Monoclonal Antibodies and Cancer Therapy. Alan R. Liss, 1985, 243-56
- **HELLSTROM et al.** Controlled Drug Delivery. Marcel Dekker, 1987, 623-53
- Monoclonal Antibodies. **THORPE et al.** Biological And Clinical Applications. 1985, vol. 84, 475-506
- Monoclonal Antibodies For Cancer Detection and Therapy. Academic Press, 1985, 303-16
- **THORPE et al.** *Immunol Rev,* 1982, vol. 62, 119